# EUROPEAN PATENT SPECIFICATION

(11) **EP 3 958 906 B1**
(45) Date of publication and mention of the grant of the patent: **24.09.2025**
(21) Application number: 20795863.8
(22) Date of filing: 24.04.2020
(51) Int. Cl.: A61K 47/42, A61K 9/00, A61P 25/00, C07K 14/47, C12N 15/10, C12N 5/071, C12N 15/87

(54) **METHOD AND VEHICLE FOR DELIVERING AGENTS ACROSS THE BLOOD-BRAIN BARRIER**
VERFAHREN UND HILFSSTOFF ZUR ABGABE VON WIRKSTOFFEN DURCH DIE BLUT-HIRN-SCHRANKE
PROCÉDÉ ET VÉHICULE POUR DISTRIBUER DES AGENTS À TRAVERS LA BARRIÈRE HÉMATO-ENCÉPHALIQUE

(30) Priority: 26.04.2019 US 201962839001 P
(43) Date of publication of application: 02.03.2022
(73) Proprietor: Braun, Janice E.A., Calgary, AB T3E 5T5 (CA)
(72) Inventor: Braun, Janice E.A., Calgary, AB T3E 5T5 (CA)
(74) Representative: Appleyard Lees IP LLP
(86) International application number: PCT/CA2020/050538
(87) International publication number: WO 2020/215158

(56) References cited:
- WO-A1-2017/147719
- WO-A1-2018/085587
- THÉRY CLOTILDE ET AL: "ISEV2018 abstract book", JOURNAL OF EXTRACELLULAR VESICLES, vol. 7, no. sup1, 20 April 2018 (2018-04-20), pages 1461450, XP093008732, DOI: 10.1080/20013078.2018.1461450
- PINK DESMOND ET AL: "Cysteine String Protein Controls Two Routes of Export for Misfolded Huntingtin", FRONTIERS IN NEUROSCIENCE, vol. 15, 5 January 2022 (2022-01-05), XP093007917, DOI: 10.3389/fnins.2021.762439
- DAVID RUFINO-RAMOS, PATRÍCIA R. ALBUQUERQUE, VITOR CARMONA, RITA PERFEITO, RUI JORGE NOBRE, LUIS PEREIRA DE ALMEIDA: "Extracellular vesicles: Novel promising delivery systems for therapy of brain diseases", JOURNAL OF CONTROLLED RELEASE, vol. 262, 28 September 2017 (2017-09-28), pages 247 - 258, XP055755652, ISSN: 0168-3659, DOI: 10.1016/j.jconrel.2017.07.001
- JINGTI DENG, KOUTRAS CAROLINA, DONNELIER JULIEN, ALSHEHRI MANA, FOTOUHI MARYAM, GIRARD MARTINE, CASHA STEVE, MCPHERSON PETER S., R: "Neurons export extracellular vesicles enriched in cystein string protein and misfolded protein cargo", SCIENTIFIC REPORTS, vol. 7, no. 1, 19 April 2017 (2017-04-19), pages 956, XP055755654, DOI: 10.1038/s41598-017-01115-6
- CHRISTINA ZAROUCHLIOTI, DAVID A. PARFITT, WENWEN LI, LAUREN M. GITTINGS, MICHAEL E. CHEETHAM: "DNAJproteins in neurodegeneration: essential and protective factors", PHILOSOPHICAL TRANSACTIONS. ROYAL SOCIETY OF LONDON. B: BIOLOGICAL SCIENCES., vol. 373, no. 1738, 19 January 2018 (2018-01-19), XP055755656, ISSN: 0962-8436, DOI: 10.1098/rstb.2016.0534
- PATEL, MAYUR M.; PATEL, BHOOMIKA M. : "Crossing the blood-brain barrier: recent advances in drug delivery to the brain", CNS DRUGS, vol. 31, no. 22, 31 January 2017 (2017-01-31), AUCKLAND, NZ , pages 109 - 133, XP009531419, ISSN: 1172-7047, DOI: 10.1007/s40263-016-0405-9
- JOHNSON, JADAH N.; AHRENDT, EVA; BRAUN, JANICE E. A.: "CSPa: the neuroprotective J protein", BIOCHEMISTRY AND CELL BIOLOGY, vol. 88, no. 2, 31 March 2010 (2010-03-31), CA , pages 157 - 165, XP009531418, ISSN: 0829-8211, DOI: 10.1139/O09-124

## Description

### CROSS REFERENCE TO PRIOR APPLICATIONS

This application claims priority under the Paris Convention to U.S. Provisional Patent Application 62/839,001 filed April 26, 2019.

### FIELD OF THE DISCLOSURE

The present disclosure relates generally to a composition and vehicle for use in drug delivery. In particular, the present disclosure provides compositions for use in delivering an agent across the blood-brain barrier and an agent delivery vehicle for same.

### BACKGROUND OF THE DISCLOSURE

Central nervous system (CNS) diseases and disorders are an enormous burden on patients, families and health care systems around the world. Looking at neurodegenerative diseases alone, the number of deaths attributed to Parkinson's disease more than doubled in the period between 1990 and 2010 (The Lancet Neurology Editorial Board. Lancet Neurol 12, 119, (2013)). During the same time period, estimates of the number of deaths caused by Alzheimer's disease (AD) and other dementias more than tripled (The Lancet Neurology Editorial Board. Lancet Neurol 12, 119, (2013)).

CNS diseases and disorders have been notoriously difficult to treat. To date, therapeutic agents for treating neurodegenerative diseases have not succeeded in clinical trials and there are no effective cures. Development of therapies to treat other types of CNS diseases and disorders, such as psychiatric diseases and disorders, has also faced challenges. Although therapeutic agents for treating psychiatric diseases and disorders are available, many existing treatment options have shown limited effectiveness and significant negative side effects.

In addition to the human health burden, various CNS diseases and disorders in domesticated animals impose significant economic costs to farming and agriculture, as well as veterinary care costs to owners of sport animals and companion animals / pets.

A major obstacle to the successful development of new therapeutic agents for treating CNS diseases and disorders, including neurodegenerative and psychiatric diseases and disorders, has been the inability to deliver potential therapeutic agents across the blood-brain barrier. Thus, there is a need to develop better methods and tools for delivering agents across the blood-brain barrier and to the CNS.

### SUMMARY OF THE DISCLOSURE

The invention is defined with reference to the appended claims.

Disclosed herein is a method of delivering an agent across the blood-brain barrier; a method of delivering an agent to the CNS; a method of treating a CNS disease or disorder; an agent delivery vehicle for same; and an extracellular vesicle for same.

In one disclosure, a method of delivering an agent across the blood-brain barrier in a subject is provided. The method comprises administering an effective amount of extracellular vesicles to the subject, wherein the extracellular vesicles comprise the agent and a DnaJ domain-containing protein chaperone or a fragment thereof.

There is disclosed a method of delivering an agent across the blood-brain barrier provided herein, the DnaJ domain-containing protein chaperone comprises one or more of a cysteine string protein alpha (CSPα) polypeptide, a DnaJ Heat Shock Protein Family Hsp40 Member B2 (DnaJB2) polypeptide, a DnaJ Heat Shock Protein Family Hsp40 Member B6 (DnaJB6) polypeptide, a DnaJ Heat Shock Protein Family Hsp40 Member C7 (DnaJC7) polypeptide, and a DnaJ Heat Shock Protein Family Hsp40 Member C10 (DnaJC10) polypeptide, or a fragment or fragments thereof.

A method of delivering an agent across the blood-brain barrier, wherein the DnaJ domain-containing protein chaperone is a CSPα polypeptide or a fragment thereof is also disclosed.

Further disclosed is a method of delivering an agent across the blood-brain barrier, wherein the extracellular vesicles comprise exosomes, microvesicles, exophers, large extracellular vesicles, or combinations thereof.

Also disclosed is a method of delivering an agent across the blood-brain barrier,wherein the administration is to a site other than the CNS.

Also disclosed is a method of delivering an agent across the blood-brain barrier, wherein the administration comprises intravenous (IV), intraperitoneal (IP), intramuscular, subcutaneous, transdermal, oral, buccal, intraocular, nasal, or rectal route of administration, or a combination thereof.

Further disclosed is a method of delivering an agent across the blood-brain barrier, wherein the agent is chemically synthesized or naturally derived.

Also disclosed is a method of delivering an agent across the blood-brain barrier, wherein the agent is a polypeptide, a nucleic acid, a small molecule drug, or a combination thereof.

Also disclosed is a method of delivering a polypeptide across the blood-brain barrier, wherein the polypeptide is a structural protein, an enzyme, or an antibody or antigen-reactive fragment thereof.

In another disclosure, there is a method of delivering a nucleic acid across the blood-brain, wherein the nucleic acid is deoxyribonucleic acid (DNA), ribonucleic acid (RNA), or a derivative thereof.

A disclosure of a method of delivering an agent across the blood-brain barrier is provided herein, wherein the subject is a mammal.

In another disclosure of the method of delivering an agent across the blood-brain barrier provided herein, the subject is a human.

In a related disclosure, a method of delivering an agent to the CNS of a subject is provided. The method comprises administering an effective amount of extracellular vesicles to the subject, wherein the extracellular vesicles comprise the agent and a DnaJ domain-containing protein chaperone or a fragment thereof.

In another disclosure of the method of delivering an agent to the CNS provided herein, the DnaJ domain-containing protein chaperone comprises one or more of a CSPα polypeptide, a DnaJB2 polypeptide, a DnaJB6 polypeptide, a DnaJC7 polypeptide, and a DnaJC10 polypeptide, or a fragment or fragments thereof.

In a further disclosure there is provided a method of delivering an agent to the CNS provided herein, the DnaJ domain-containing protein chaperone is a CSPα polypeptide or a fragment thereof.

In another disclosure there is provided a method of delivering an agent to the CNS provided herein, the extracellular vesicles comprise exosomes, microvesicles, exophers, large extracellular vesicles, or combinations thereof.

In another disclosure there is provided a method of delivering an agent to the CNS provided herein, the administering is to a site other than the CNS.

In a further disclosure there is provided a method of delivering an agent to the CNS provided herein, the administering comprises intravenous, intraperitoneal, intramuscular, subcutaneous, transdermal, oral, buccal, intraocular, nasal, or rectal route of administration, or a combination thereof.

In another disclosure there is provided a method of delivering an agent to the CNS provided herein, the agent is chemically synthesized or naturally derived.

In a further disclosure there is provided a method of delivering an agent to the CNS provided herein, the agent is a polypeptide, a nucleic acid, a small molecule drug, or a combination thereof.

In another disclosure there is provided a method of delivering a polypeptide to the CNS provided herein, the polypeptide is a structural protein, an enzyme, or an antibody or antigen-reactive fragment thereof.

In a further disclosure there is provided a method of delivering a nucleic acid to the CNS provided herein, the nucleic acid is DNA, RNA, or a derivative thereof.

In another disclosure there is provided a method of delivering an agent to the CNS provided herein, the subject is a mammal.

In a further disclosure there is provided a method of delivering an agent to the CNS provided herein, the subject is a human.

In another disclosure, a method of treating a CNS disease or disorder in a subject is provided. The method comprises administering an effective amount of extracellular vesicles to the subject, wherein the extracellular vesicles comprise:
a DnaJ domain-containing protein chaperone or a fragment thereof, and
an agent useful for treatment of the CNS disease or disorder.

In another disclosure of the method of treating a CNS disease or disorder provided herein, the CNS disease or disorder is a neurodegenerative, psychiatric, neurodevelopmental, or motor disease or disorder.

In a further disclosure of the method of treating a CNS disease or disorder provided herein, the CNS disease or disorder is Alzheimer's disease, Huntington's disease, Parkinson's disease, amyotrophic lateral sclerosis (ALS), dementia, multiple sclerosis, epilepsy, a demyelinating disorder; a myelopathy, a chaperonopathy, an autoimmune disorder, a prion disease, addiction, depression, an anxiety disorder, obsessive-compulsive disorder, attention deficit hyperactivity disorder, Tourette's syndrome, bipolar affective disorder, schizophrenia, Asperger syndrome, autism, CNS infection, CNS trauma, or stroke.

In another disclosure of the method of treating a CNS disease or disorder provided herein, the DnaJ domain-containing protein chaperone comprises one or more of a CSPα polypeptide, a DnaJB2 polypeptide, a DnaJB6 polypeptide, a DnaJC7 polypeptide, and a DnaJC10 polypeptide, or a fragment or fragments thereof.

In a further disclosure of the method of treating a CNS disease or disorder provided herein, the DnaJ domain-containing protein chaperone is a CSPα polypeptide or a fragment thereof.

In another disclosure of the method of treating a CNS disease or disorder provided herein, the extracellular vesicles comprise exosomes, microvesicles, exophers, large extracellular vesicles, or combinations thereof.

In another disclosure of the method of treating a CNS disease or disorder provided herein, the administering is to a site other than the CNS.

In a further disclosure of the method of treating a CNS disease or disorder provided herein, the administering comprises intravenous, intraperitoneal, intramuscular, subcutaneous, transdermal, oral, buccal, intraocular, nasal, or rectal route of administration, or a combination thereof.

In a further disclosure of the method of treating a CNS disease or disorder provided herein, the agent is chemically synthesized or naturally derived.

In another disclosure of the method of treating a CNS disease or disorder provided herein, the agent is a polypeptide, a nucleic acid, a small molecule drug, or a combination thereof.

In a further disclosure of the method of treating a CNS disease or disorder provided herein, the polypeptide is a structural protein, an enzyme, or an antibody or antigen-reactive fragment thereof.

In a further disclosure of the method of treating a CNS disease or disorder provided herein, the nucleic acid is DNA, RNA, or a derivative thereof.

In a further disclosure of the method of treating a CNS disease or disorder provided herein, the subject is a mammal.

In another disclosure of the method of treating a CNS disease or disorder provided herein, the subject is a human.

In another disclosure, an agent delivery vehicle is provided. The agent delivery vehicle comprises an effective amount of extracellular vesicles capable of crossing the blood-brain barrier, wherein the extracellular vesicles comprise:
a DnaJ domain-containing protein chaperone or a fragment thereof, and
an agent useful for treatment of the CNS disease or disorder.

In another disclosure of the agent delivery vehicle provided herein, the DnaJ domain-containing protein chaperone comprises one or more of a CSPα polypeptide, a DnaJB2 polypeptide, a DnaJB6 polypeptide, a DnaJC7 polypeptide, and a DnaJC10 polypeptide, or a fragment or fragments thereof.

In a further disclosure of the agent delivery vehicle provided herein, the DnaJ domain-containing protein chaperone is a CSPα polypeptide or a fragment thereof.

In another disclosure of the agent delivery vehicle provided herein, the extracellular vesicles comprise exosomes, microvesicles, exophers, large extracellular vesicles, or combinations thereof.

In a further disclosure of the agent delivery vehicle provided herein, the agent is chemically synthesized or naturally derived.

In another disclosure of the agent delivery vehicle provided herein, the agent is a polypeptide, a nucleic acid, a small molecule drug, or a combination thereof.

In a further disclosure of the agent delivery vehicle provided herein, the polypeptide is a structural protein, an enzyme, or an antibody or antigen-reactive fragment thereof.

In another disclosure of the agent delivery vehicle provided herein, the nucleic acid is DNA, RNA, or a derivative thereof.

In a further disclosure of the agent delivery vehicle provided herein, the CNS disease or disorder is a neurodegenerative, psychiatric, neurodevelopmental, or motor disease or disorder.

In another disclosure of the agent delivery vehicle provided herein, the CNS disease or disorder is Alzheimer's disease, Huntington's disease, Parkinson's disease, amyotrophic lateral sclerosis, dementia, multiple sclerosis, epilepsy, a demyelinating disorder; a myelopathy, a chaperonopathy, an autoimmune disorder, a prion disease, addiction, depression, an anxiety disorder, obsessive-compulsive disorder, attention deficit hyperactivity disorder, Tourette's syndrome, bipolar affective disorder, schizophrenia, Asperger syndrome, autism, CNS infection, CNS trauma, or stroke.

In a further disclosure, an extracellular vesicle is provided. The extracellular vesicle comprises a DnaJ domain-containing protein chaperone or a fragment thereof and an agent useful for treatment of a CNS disease or disorder.

In another disclosure of the extracellular vesicle provided herein, the DnaJ domain-containing protein chaperone comprises one or more of a CSPα polypeptide, a DnaJB2 polypeptide, a DnaJB6 polypeptide, a DnaJC7 polypeptide, and a DnaJC10 polypeptide, or a fragment or fragments thereof.

In a further disclosure of the extracellular vesicle provided herein, the DnaJ domain-containing protein chaperone is a CSPα polypeptide or a fragment thereof.

In another dislcosure of the extracellular vesicle provided herein, the extracellular vesicle is an exosome, a microvesicle, an exopher, or a large extracellular vesicle.

In a further disclosure of the extracellular vesicle provided herein, the agent is chemically synthesized or naturally derived.

In another disclosure of the extracellular vesicle provided herein, the agent is a polypeptide, a nucleic acid, a small molecule drug, or a combination thereof.

In another disclosure of the extracellular vesicle provided herein, the polypeptide is a structural protein, an enzyme, or an antibody or antigen-reactive fragment thereof.

In a further disclosure of the extracellular vesicle provided herein, the nucleic acid is DNA, RNA, or a derivative thereof.

In another disclosure of the extracellular vesicle provided herein, the CNS disease or disorder is a neurodegenerative, psychiatric, neurodevelopmental, or motor disease or disorder.

In a further disclosure of the extracellular vesicle provided herein, the CNS disease or disorder is Alzheimer's disease, Huntington's disease, Parkinson's disease, amyotrophic lateral sclerosis, dementia, multiple sclerosis, epilepsy, a demyelinating disorder; a myelopathy, a chaperonopathy, an autoimmune disorder, a prion disease, addiction, depression, an anxiety disorder, obsessive-compulsive disorder, attention deficit hyperactivity disorder, Tourette's syndrome, bipolar affective disorder, schizophrenia, Asperger syndrome, autism, CNS infection, CNS trauma, or stroke.

In another disclosure, a composition is provided. The composition comprises the extracellular vesicle comprising a DnaJ domain-containing protein chaperone or a fragment thereof and an agent useful for treatment of a CNS disease or disorder; and a pharmaceutically acceptable carrier, diluent, or excipient.

### BRIEF DESCRIPTION OF THE DRAWINGS

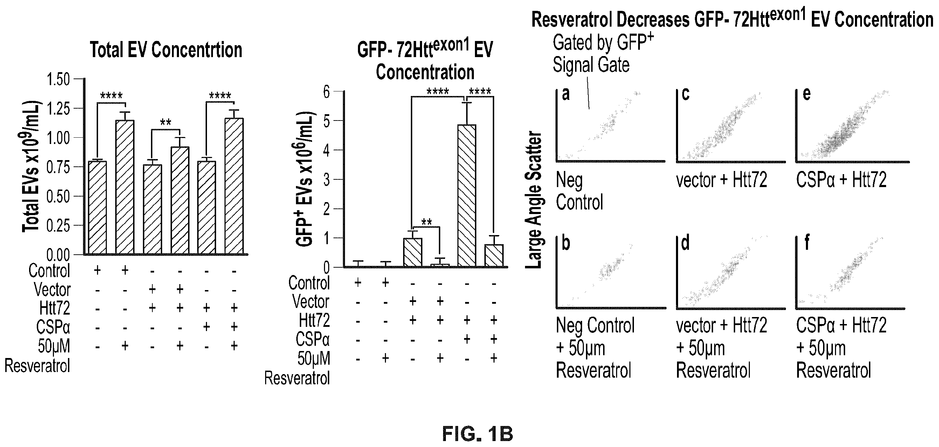

Fig. 1B is a set of bar graphs (two left-most panels) and nanoscale flow cytometry scatter plots (right panel) showing the concentration of total and GFP-72Q huntingtin^{exon1}-containing EVs in the presence or absence of 50 µM resveratrol. The experiment was replicated 10-12 times. (**P<0.01, ****P<0.0001)

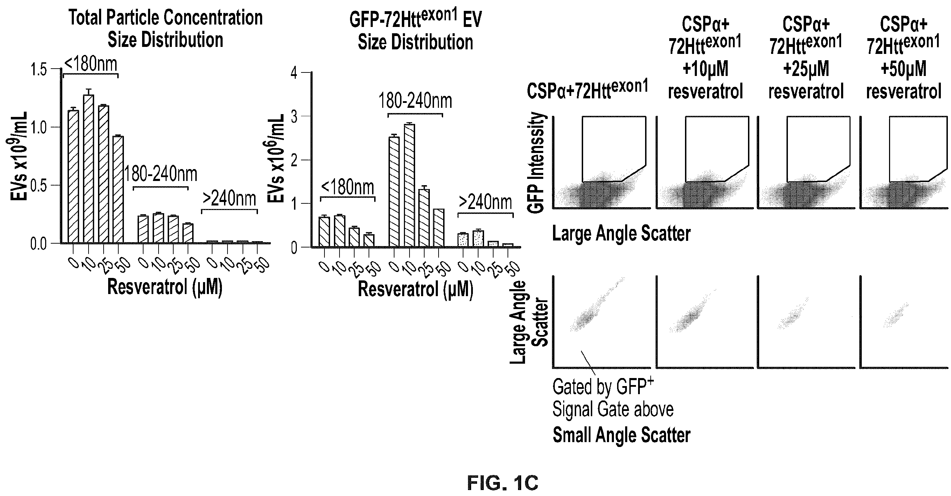

Fig. 1C is a set of bar graphs (two left-most panels) and flow cytometry scatter plots (right panel) showing the resveratrol concentration response on total and GFP-72Q huntingtin^{exon1} containing EVs levels.

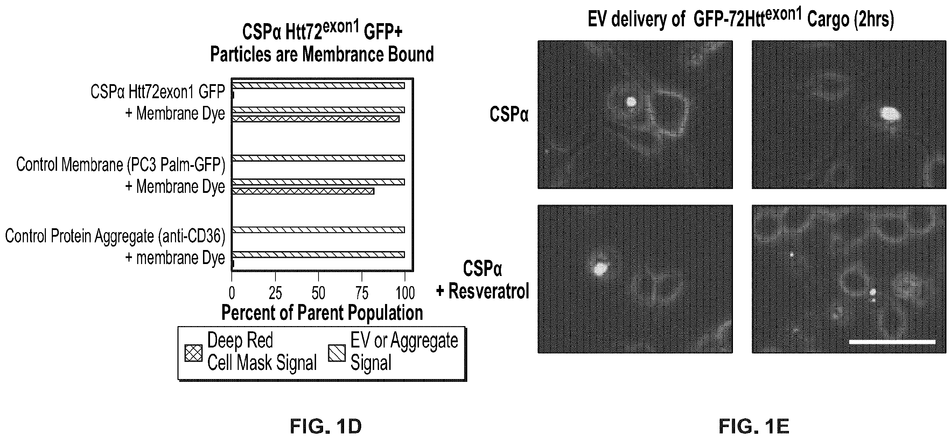

Fig. 1D is a bar graph showing that GFP-72Q huntingtin^{exon1} containing EVs co-stain with CellMask^{™} Deep Red plasma membrane stain.
Fig. 1E is a set of representative images of GFP fluorescence of recipient naive CAD cells exposed to unfractionated media collected from cells expressing GFP-72Q huntingtin^{exon1} and CSPα in the absence (top panels) and presence (bottom panels) of 50 µM resveratrol. Live cells were imaged 2 hours following media application. The experiment was replicated 10-12 times.

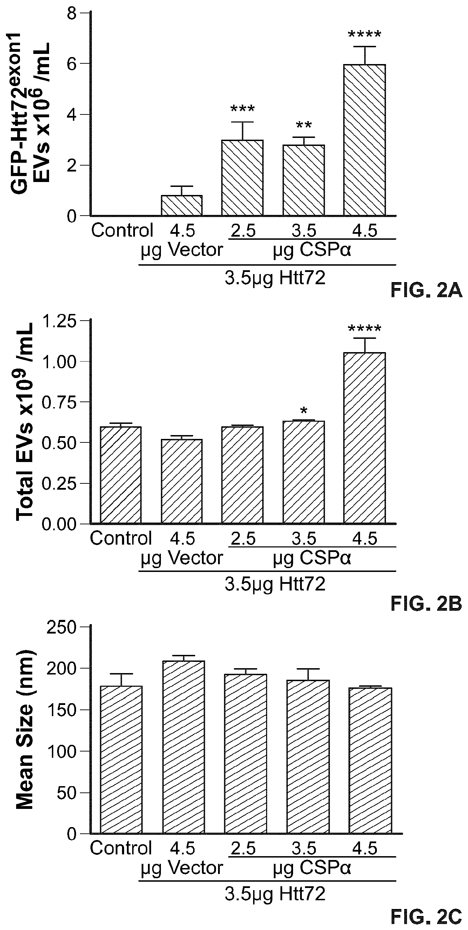

Fig. 2A is a bar graph of the concentrations of GFP-72Q huntingtin^{exon1}-containing EVs collected from CAD neurons expressing a control vector or various levels of CSPα, as measured using nanoscale flow cytometry. (**P<0.01, ***P<0.001, ****P<0.0001)
Fig. 2B is a bar graph of the concentrations of total EVs collected from CAD neurons expressing a control vector or various levels of CSPα, as measured using nanoscale flow cytometry. (*P<0.05, ****P<0.0001)
Fig. 2C is a bar graph of the mean size of total EVs collected from CAD neurons expressing a control vector or various levels of CSPα, as measured using nanoscale flow cytometry.

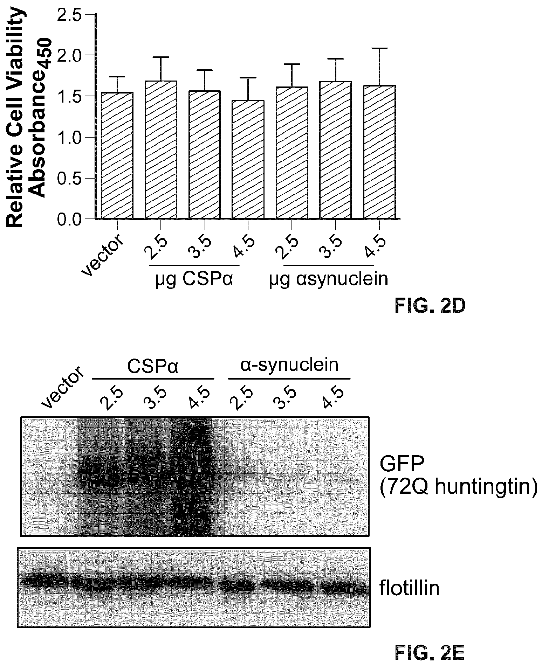

Fig. 2D is a bar graph of the relative viability of CAD cells expressing a control vector or GFP-tagged 72Q huntingtin^{exon1} and various levels of CSPα or α-synuclein following media collection.
Fig. 2E is a set of images of a western blot analysis of EVs collected from CAD cells expressing a control vector or GFP-tagged 72Q huntingtin^{exon1} and various levels of CSPα or α-synuclein. Blots were probed for GFP and flotillin (loading control). The images are representative of 4 independent western blot experiments.

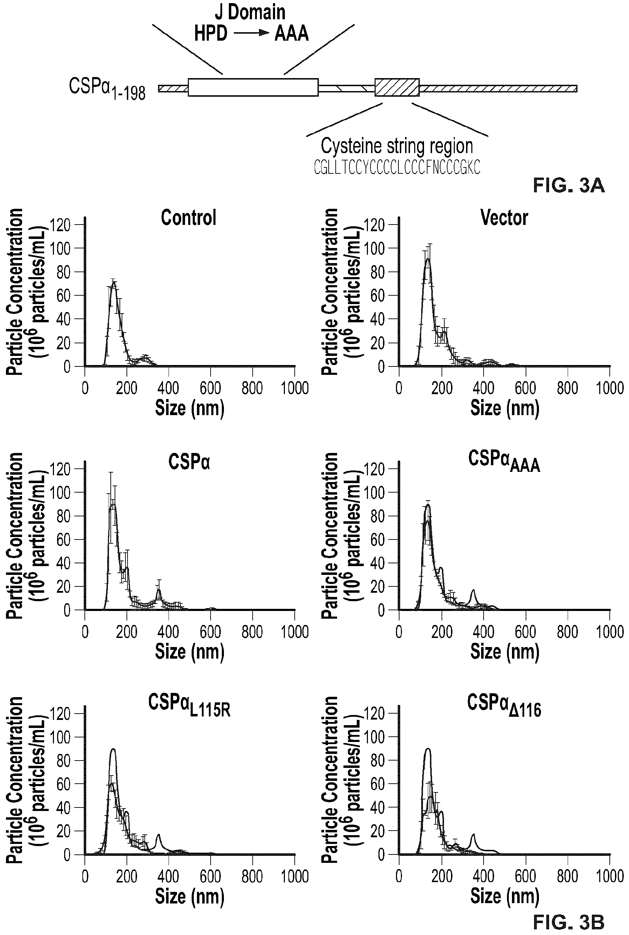

Fig. 3A is an illustration of the domain structure of CSPα, highlighting the sites of the CSPα_{HPD-AAA}, CSPα_{L115R} and CSPα_{Δ116} mutations.
Fig. 3B is a set of graphs of the frequency of total EVs of various sizes exported from CAD cells expressing a vector control, CSPα, CSPα_{HPD-AAA}, CSPα_{L115R}, or CSPα_{Δ116} (but not GFP-72Q huntingtin^{exon1}), as measured by nanoparticle tracking analysis (NTA). Mean histogram data for CSPα is overlaid for comparison.

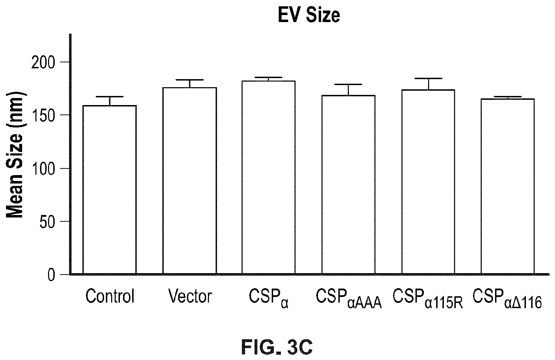

Fig. 3C is a bar graph of the mean size of total EVs collected from CAD cells expressing a vector control, CSPα, CSPα_{HPD-AAA}, CSPα_{L115R}, or CSPα_{Δ116}.

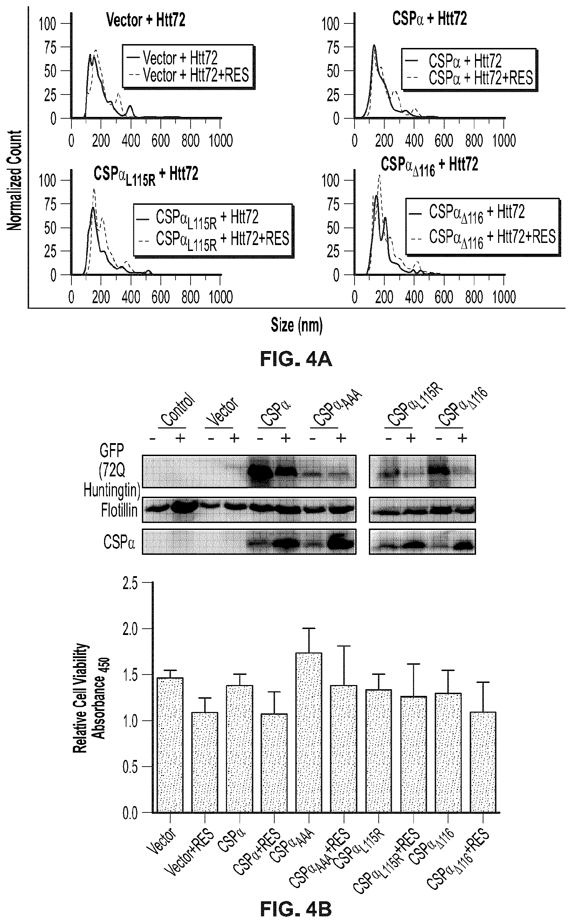

Fig. 4A is a set of graphs of the frequency of total EVs of various sizes exported from CAD cells expressing GFP-72Q huntingtin^{exon1} and a vector control, CSPα, CSPα_{L115R}, or CSPα_{Δ116}, as measured by NTA. The CAD cells were grown in the presence or absence of 50 µM resveratrol.
Fig. 4B is a set of images of a western blot analysis of EVs collected from CAD cells expressing GFP-72Q huntingtin^{exon1} and the indicated CSPα mutants in the presence (+) or absence (-) of 50 µM resveratrol (top panel). Blots were probed for GFP, flotillin (loading control) and CSPα. A bar graph of the relative viability of the CAD cells following media collection is also shown (bottom panel).

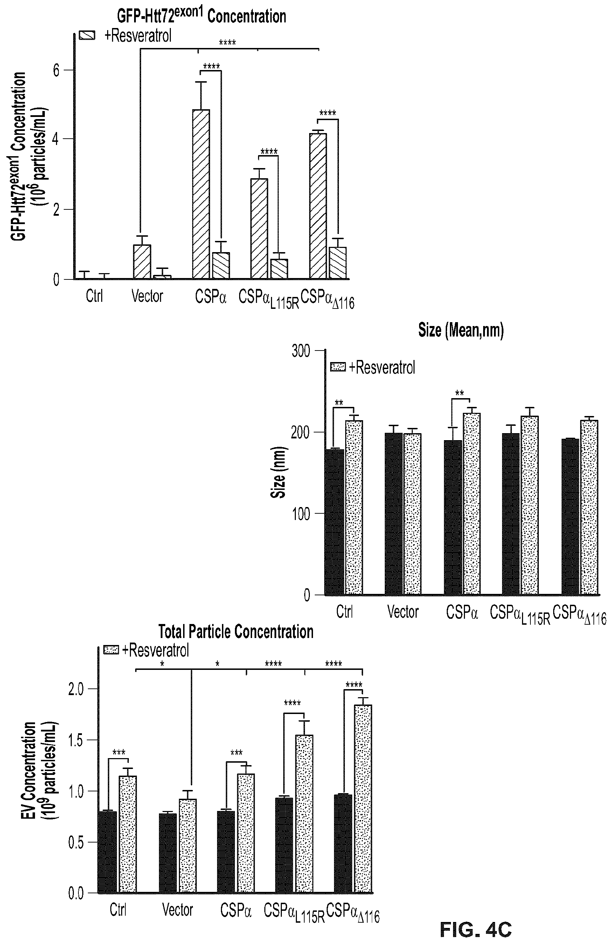

Fig. 4C is a set of bar graphs of the concentration of GFP-72Q huntingtin^{exon1}-containing EVs (top panel); the mean size of total EVs (middle panel); and the concentration of total EVs (bottom panel) collected from CAD cells expressing GFP-72Q huntingtin^{exon1} and the indicated CSPα mutants in the presence or absence of 50 µM resveratrol, as determined by nanoscale flow cytometry. (*P<0.05, **P<0.01, ***P<0.001, ****P<0.0001)

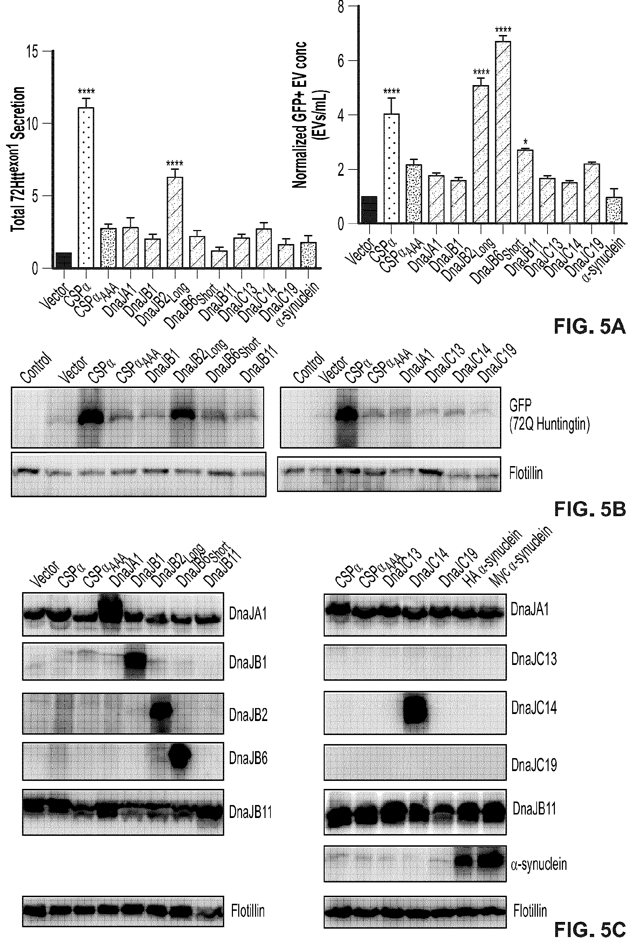

Fig. 5A is a set of bar graphs of a quantification by western blot analysis (left panel) and by nanoscale flow cytometry (right panel) of secreted GFP-tagged 72Q huntingtin^{exon1} from cells expressing GFP-tagged 72Q huntingtin^{exon1} and a vector control, myc-tagged CSPα, CSPα_{HPD-AAA}, DnaJA1, DnaJB1, DnaJB2_{long}, DnaJB6ₛₕₒᵣₜ, DnaJB11, DnaJC13, DnaJC14, DnaJC19 or α-synuclein. (*P<0.05, ****P<0.0001)
Fig. 5B is a set of images of representative western blots of EVs collected from cells expressing GFP-tagged 72Q huntingtin^{exon1} and the indicated proteins. Blots were probed for GFP and flotillin (loading control).
Fig. 5C is a set of images of western blots of EVs collected from CAD cells expressing the indicated proteins. Blots were probed for the proteins indicated on the right.

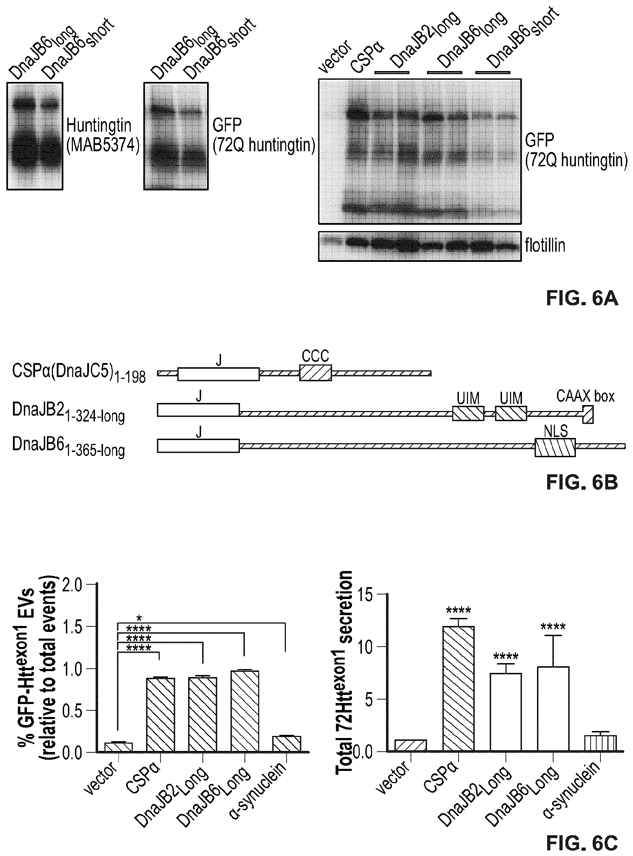

Fig. 6A is a set of images of a western blot analysis of EVs from neurons expressing the indicated DnaJB6 isoforms, probed with anti-huntingtin and anti-GFP antibodies (two left-most panels). Western blot analysis of EVs collected from CAD cells transfected with 3.5 µg GFP-tagged 72Q huntingtin^{exon1} and 3.5 µg vector control; 3.5 µg CSPα; 3.5 or 4.5 µg DnaJB2_{long}; 3.5 or 4.5 µg of DnaJB6_{long}; or 3.5 or 4.5 µg of DnaJB6ₛₕₒᵣₜ, probed for GFP and flotillin (right panel).
Fig. 6B is an illustration of the domain structure of CSPα, DnaJB2_{long} and DnaJB6_{long} highlighting the common DnaJ domains (J) and unique cysteine string region (CCC), ubiquitin interacting motifs (UIM), CAAX box and nuclear localization signal (NLS).
Fig. 6C is a set of bar graphs of the percentage of GFP-positive EVs in the total EV pool, as determined by nanoscale flow cytometry (left panel); and the quantification of secreted GFP-tagged 72Q huntingtin^{exon1}, as determined by western blot analysis (right panel). (*P<0.05, ****P<0.0001)

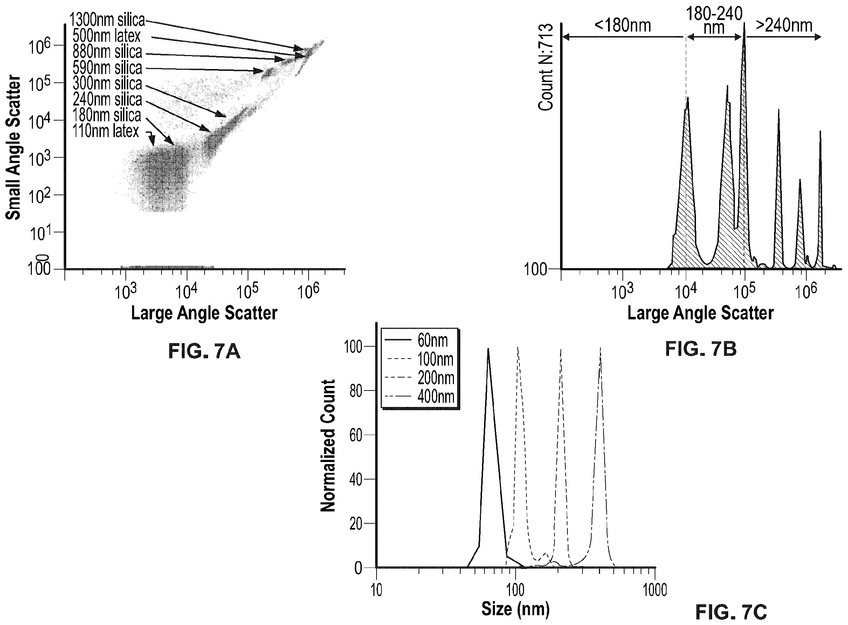

Fig. 7A is a scatter plot of the nanoscale flow cytometry size standards, including both fluorescent latex and non-fluorescent silica standards, ranging in size from 110 nm - 1,300 nm.
Fig. 7B is a graph showing the frequencies of the nanoscale flow cytometry size standards depicted in Fig. 7A. Dashed vertical lines indicate the division of size ranges of detected EVs described herein to carry GFP-tagged 72Q huntingtin^{exon1} cargo. Fig. 7C is a graph of the NTA size standards (mean), including a 60 nm, a 200 nm and a 400 nm polystyrene NIST size standard (Thermo Scientific 3000 Series) and a 100 nm silica size standard.

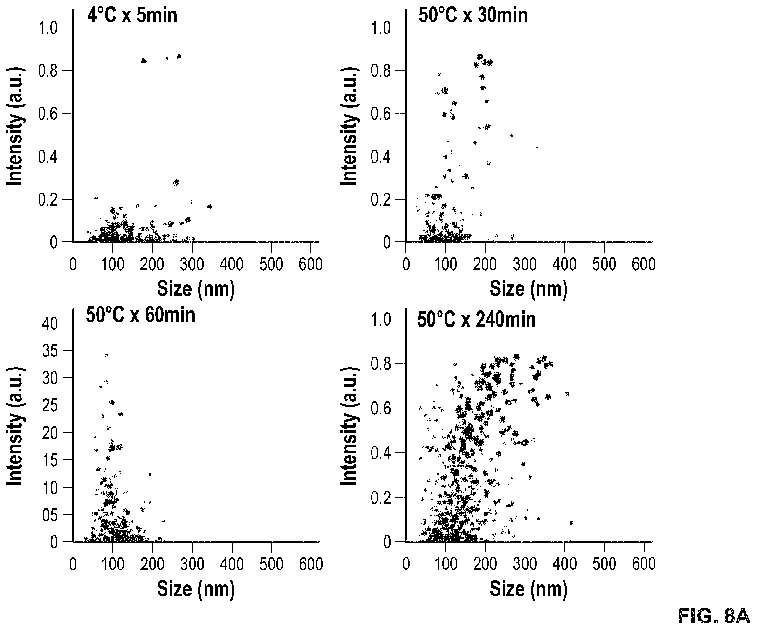

Fig. 8A is a set of scatter plots of an NTA analysis of anti-CD36-FITC antibody aggregation at 50°C.

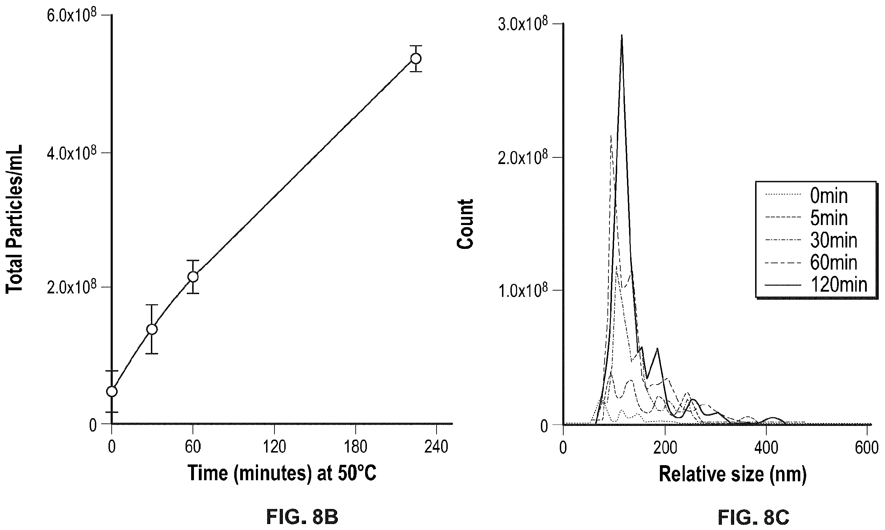

Fig. 8B is a line graph of the time dependent aggregation of anti-CD36-PE antibody at 50°C.
Fig. 8C is a histogram showing the NTA profiles of anti-CD36-FITC (or PE) antibody aggregation at 50°C.

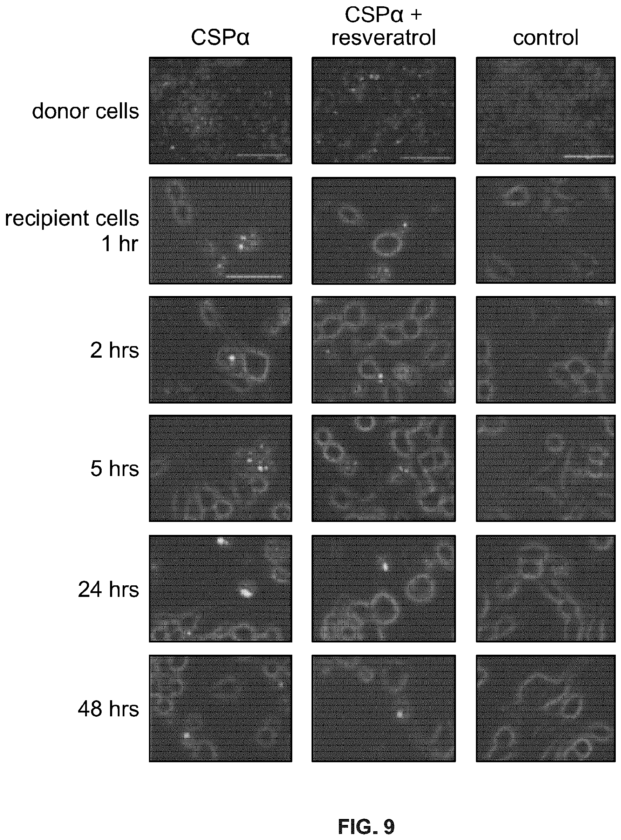

Fig. 9 is a set of fluorescence microscopy images of GFP-72Q huntingtin^{exon1} aggregates in donor CAD cells at the time of media collection (top panel), and in recipient CAD cells at the indicated times following media application (lower panels). White scale bar = 200 µm; orange scale bar = 50 µm.

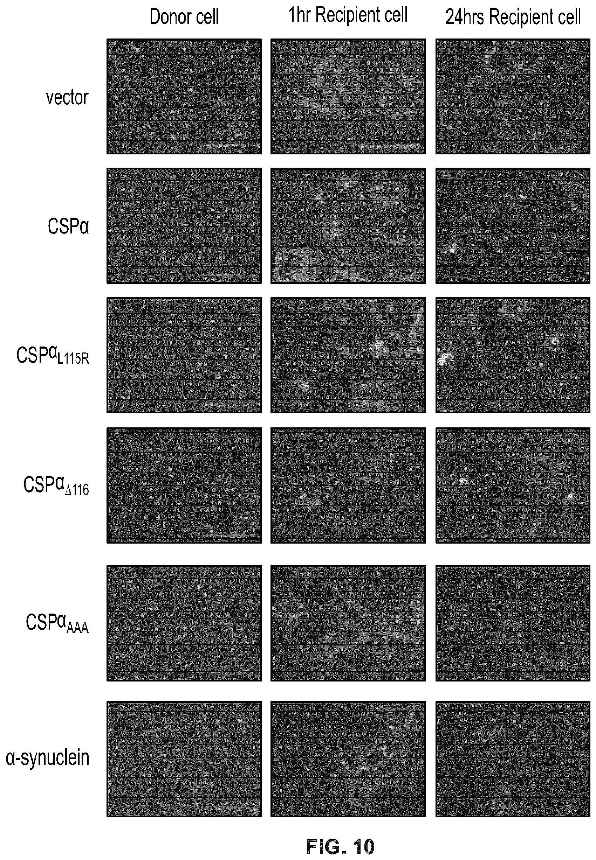

Fig. 10 is a set of representative fluorescence microscopy images of GFP-72Q huntingtin^{exon1} aggregates in donor cells transfected with DNA encoding 3.5µg CSPα, CSPα_{L115R}, CSPα_{Δ11e}, CSPα_{HPD-AAA}, or 4.5ug of α-synuclein. Recipient CAD cells are shown at 1 hour and 24 hours after application of conditioned media. White scale bar = 200 µm; orange scale bar = 50 µm.

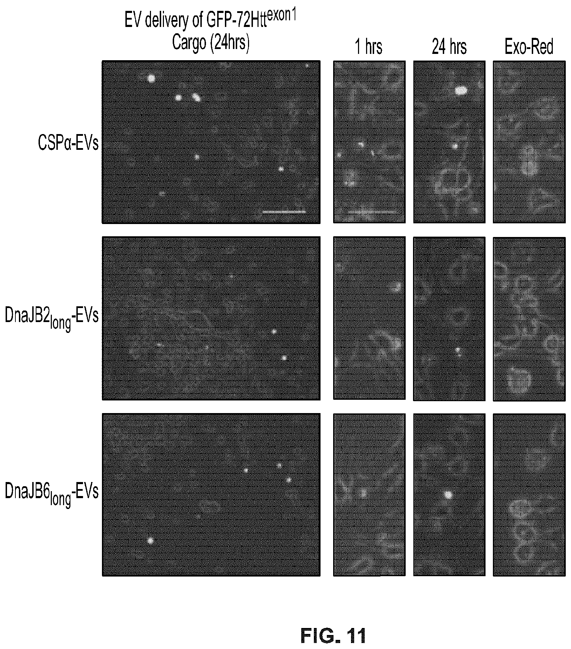

Fig. 11 is a set of representative fluorescence microscopy images of GFP-72Q huntingtin^{exon1} aggregates in recipient CAD cells at 1 hour and 24 hours after application of conditioned media from cells transfected with DNA encoding 3.5 µg CSPα, 4.5 µg DnaJB2_{long}, or 4.5 µg DnaJB6_{long}. The RNA cargo carried within EVs was labelled with Exo-Red and EVs were applied to target cells for two hours (right-most panels). White scale bar = 200 µm; orange scale bar = 50 µm.

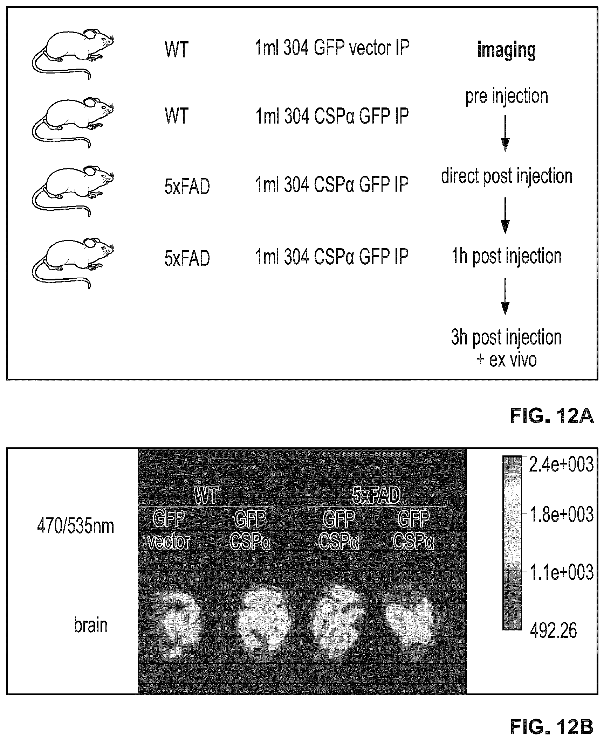

Fig. 12A is a schematic of an experiment, where EVs produced from cells expressing CSPα and green fluorescent protein (GFP)-tagged 72Q huntingtin^{exon1} or from cells expressing a vector control and GFP-tagged 72Q huntingtin^{exon1} were administered intraperitoneally to 10-12-month old wild-type (WT) or 5xFAD mice.
Fig. 12B is an *ex vivo* image overlay (black and white and fluorescent) of the brain harvested from each of the mice depicted in Fig.12A at 3 hours following the intraperitoneal administration of EVs. The scale in arbitrary photon units is depicted on the right.

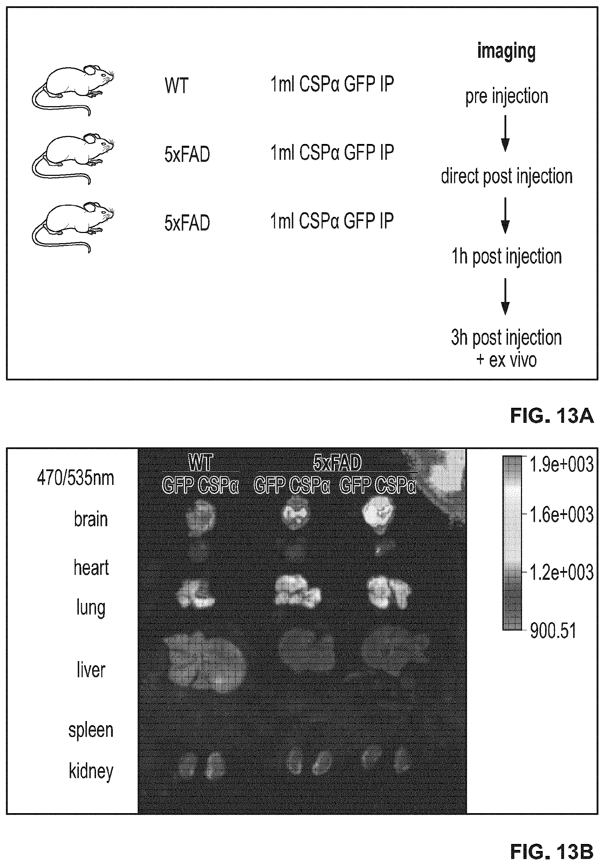

Fig. 13A is a schematic of an experiment, where EVs produced from cells expressing CSPα and GFP-tagged 72Q huntingtin^{exon1} were administered intraperitoneally to 10-12-month old WT or 5xFAD mice.
Fig. 13B is an *ex vivo* image overlay (black and white and fluorescent) of the brain, heart, lung, liver, spleen and kidney harvested from each of the mice depicted in Fig. 13A at 3 hours following the intraperitoneal administration of EVs. The scale in arbitrary photon units is depicted on the right.

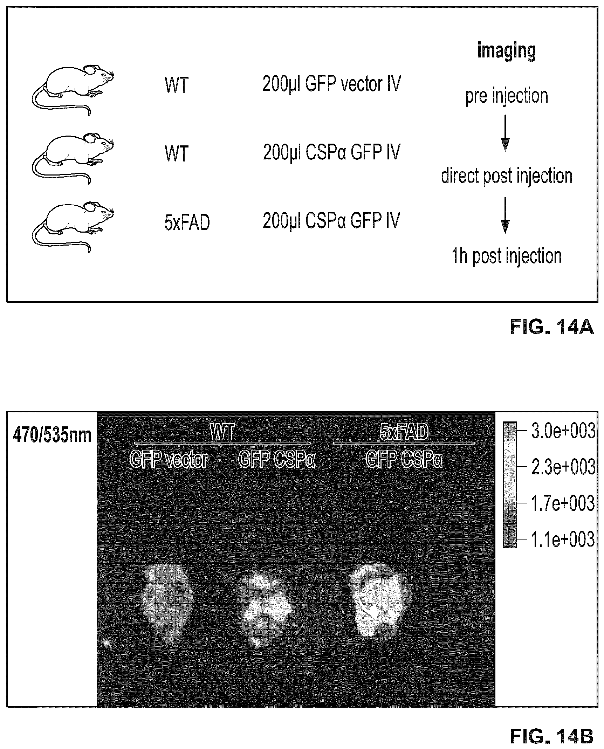

Fig. 14A is a schematic of an experiment, where EVs produced from cells expressing CSPα and GFP-tagged 72Q huntingtin^{exon1} or from cells expressing a vector control and GFP-tagged 72Q huntingtin^{exon1} were administered intravenously to 10-12-month old WT or 5xFAD mice.
Fig. 14B is an *ex vivo* image overlay (black and white and fluorescent) of the brain harvested from each of the mice depicted in Fig. 14A at 1 hour following the intravenous administration of EVs. The scale in arbitrary photon units is depicted on the right.

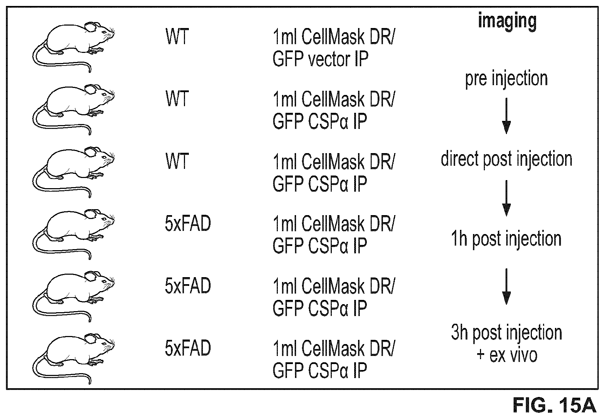

Fig. 15A is a schematic of an experiment, where EVs produced from cells expressing CSPα and GFP-tagged 72Q huntingtin^{exon1} or from cells expressing a vector control and GFP-tagged 72Q huntingtin^{exon1} were labelled with Deep Red membrane stain administered intraperitoneally to 10-12-month old WT or 5xFAD mice.

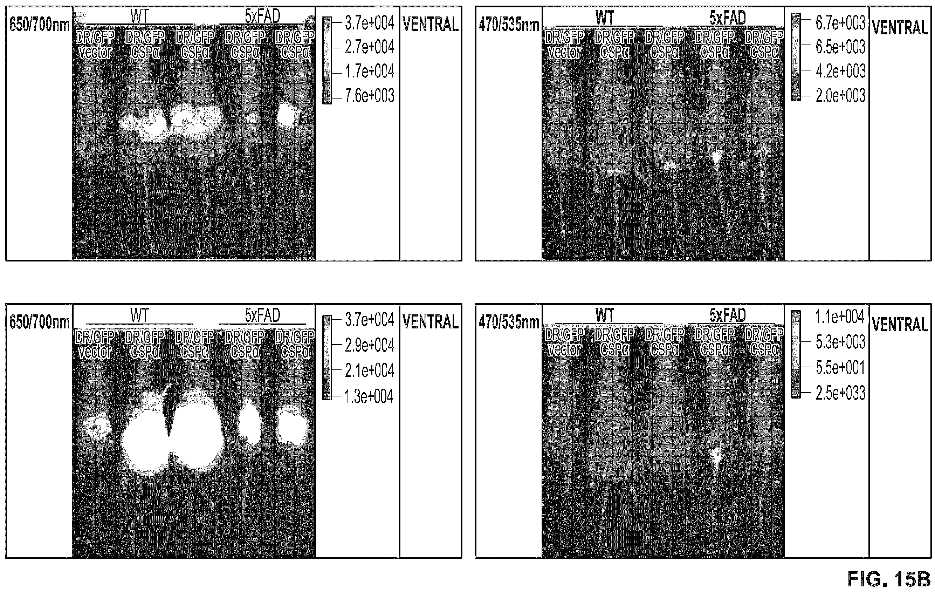

Fig. 15B is a set of *in vivo* whole body image overlays (X-ray and fluorescent) of the mice depicted in Fig. 15A. The imaging was performed from the ventral view immediately following the intraperitoneal administration of EVs (top panels), and at 3 hours following the intraperitoneal administration of EVs (bottom panels). Deep Red was imaged using a 650-700 nm emission filter (left panels). GFP was imaged using a 470-535 nm emission filter (right panels). The scale in arbitrary photon units is depicted on the right.

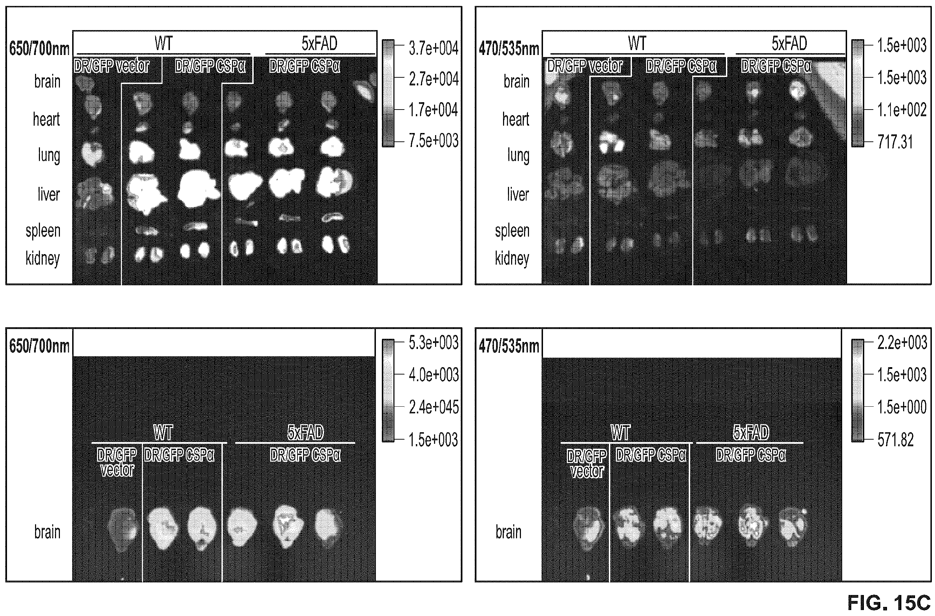

Fig. 15C is a set of ex *vivo* image overlays (black and white and fluorescent) of the brain, heart, lung, liver, spleen and kidney harvested from each of the mice depicted in Figs. 15A and 15B at 3 hours following the intraperitoneal administration of EVs. Deep Red was imaged using a 650-700 nm emission filter (left panels). GFP was imaged using a 470-535 nm emission filter (right panels). The scale in arbitrary photon units is depicted on the right.

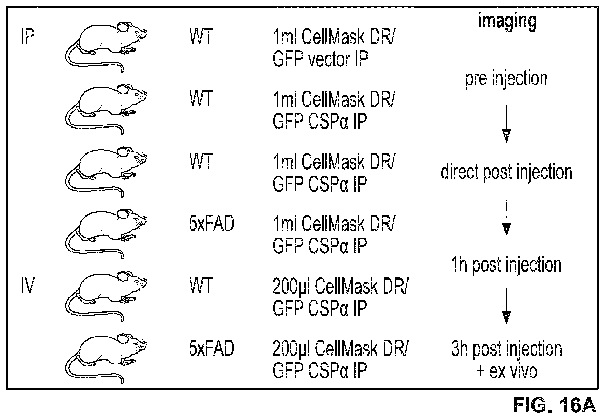

Fig. 16A is a schematic of an experiment, where EVs produced from cells expressing CSPα and GFP-tagged 72Q huntingtin^{exon1} or from cells expressing a vector control and GFP-tagged 72Q huntingtin^{exon1} were labelled with Deep Red membrane stain and administered intraperitoneally (IP) or intravenously (IV) to 10-12-month old WT or 5xFAD mice.

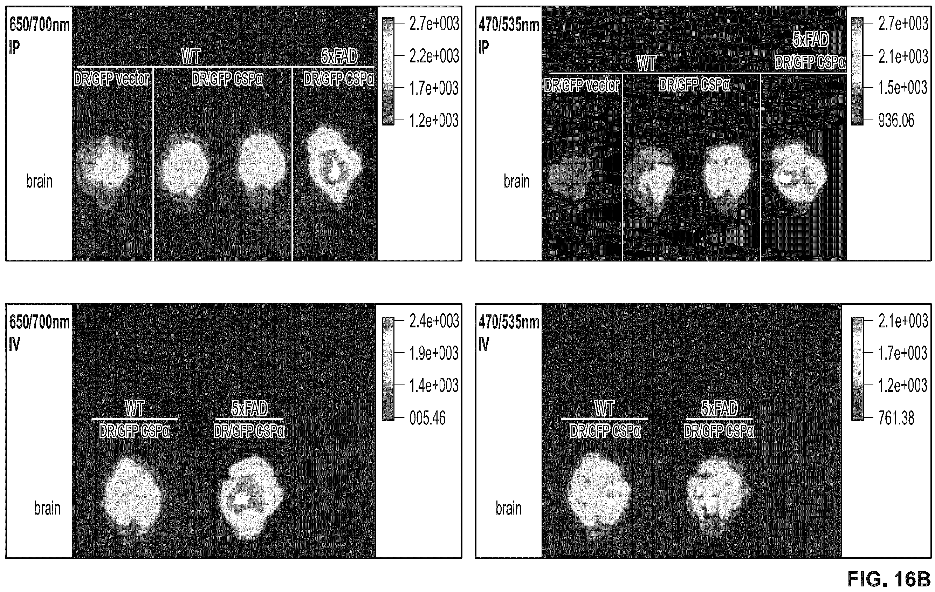

Fig. 16B is a set of *ex vivo* image overlays (black and white and fluorescent) of the brain harvested from each of the mice depicted in Fig. 16A at 3 hours following the intraperitoneal (IP) or intravenous (IV) administration of EVs. Deep Red was imaged using a 650-700 nm emission filter. GFP was imaged using a 470-535 nm emission filter. The scale in arbitrary photon units is depicted on the right.

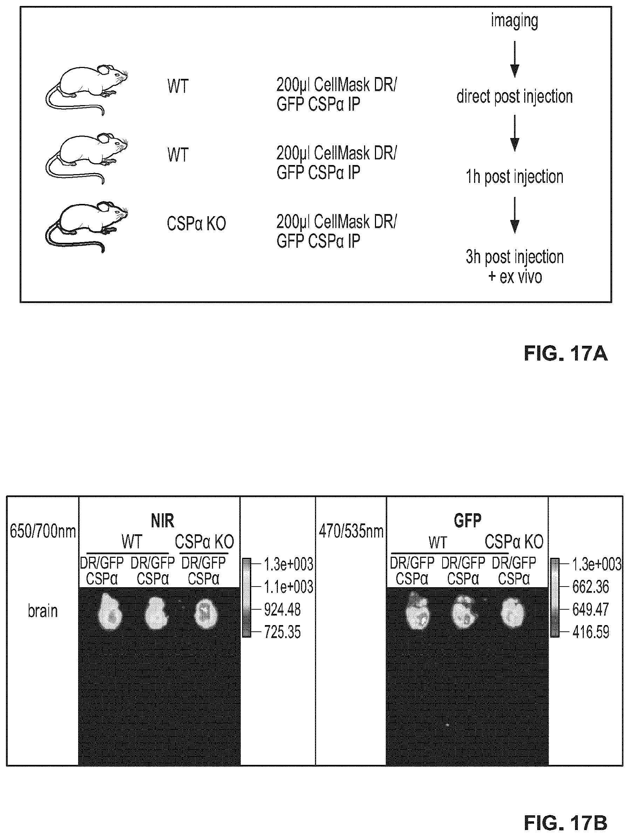

Fig. 17A is a schematic of an experiment, where EVs produced from cells expressing CSPα and GFP-tagged 72Q huntingtin^{exon1} were labelled with Deep Red membrane stain administered intraperitoneally to 22-27-day old WT or cysteine string protein alpha knock-out (CSPα KO) mice.
Fig. 17B is a set of *ex vivo* image overlays (black and white and fluorescent) of the brain harvested from each of the mice depicted in Fig. 17A at 3 hours following the intraperitoneal administration of EVs. Deep Red was imaged using a 650-700 nm emission filter. GFP was imaged using a 470-535 nm emission filter. The scale in arbitrary photon units is depicted on the right.

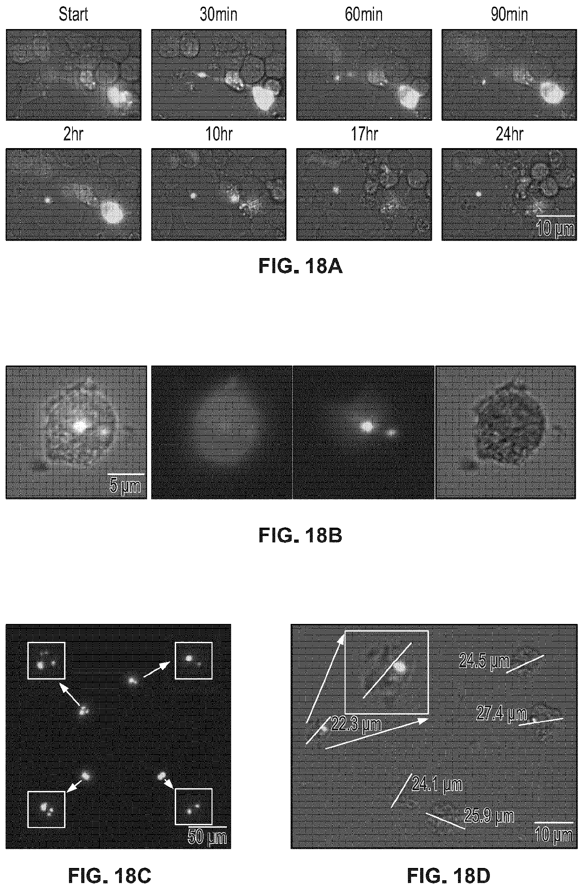

Fig. 18A is a set of microscopy images (bright-field and fluorescent) of the export of EVs containing GFP-tagged 72Q huntingtin^{exon1} aggregates from CAD cells transiently expressing CSPα and GFP-tagged 72Q huntingtin^{exon1} over a period of 24 hours.
Fig. 18B is a set of microscopy images (bright-field and fluorescent) of an EV containing GFP-72Q huntingtin^{exon1} aggregates. Red fluorescence indicates Deep Red-stained vesicle membrane. Green fluorescence indicates GFP-tagged 72Q huntingtin^{e×on1}.
Fig. 18C is a high resolution fluorescence deconvolution microscopy image of GFP-72Q huntingtin^{exon1} aggregates located in EVs.
Fig. 18D is a microscopy image (bright-field and fluorescence) showing the sizes of large EVs (i.e., exophers or large extracellular vesicles) exported from CAD cells transiently expressing CSPα and GFP-tagged 72Q huntingtin^{exon1}.

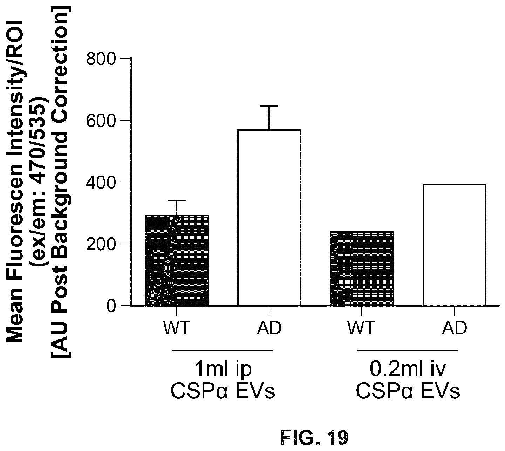

Fig. 19 is a graph of GFP fluorescence intensity detected in the brains of WT (black bars) and 5xFAD (white bars) mice used in the *ex vivo* imaging experiment shown in Fig. 16B. GFP was imaged using a 470-535 nm emission filter. (AU, arbitrary photon units) (N = 2-6; mean and standard error of the mean (SEM))

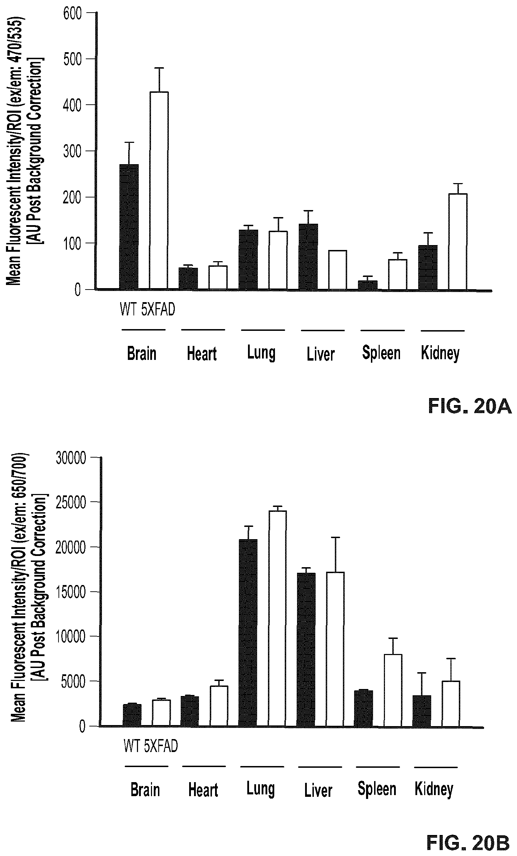

Fig. 20A is a graph of GFP fluorescence intensity detected in the brain, heart, lung, liver, spleen and kidney of WT (black bars) and 5xFAD (white bars) mice used in the *ex vivo* imaging experiment shown in Fig. 15C. GFP was imaged using a 470-535 nm emission filter. (AU, arbitrary photon units) (N = 2-6; mean and standard deviation (SD))
Fig. 20B is a graph of Deep Red fluorescence intensity detected in the brain, heart, lung, liver, spleen and kidney of WT (black bars) and 5xFAD (white bars) mice used in the ex *vivo* imaging experiment shown in Fig. 15C. Deep Red was imaged using a 650-700 nm emission filter. (AU, arbitrary photon units) (N = 2-6; mean and SD)

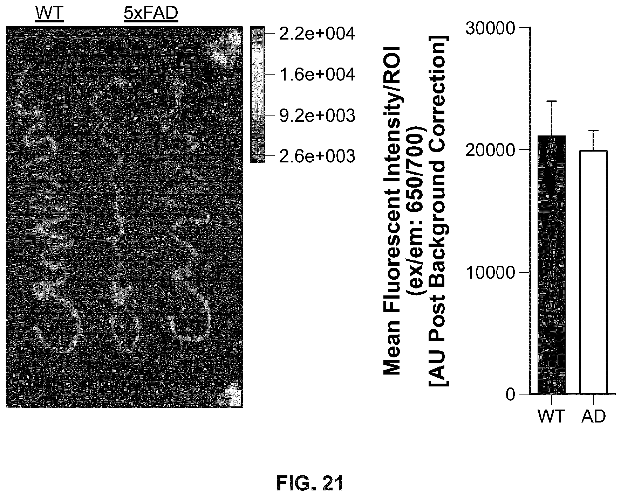

Fig. 21 is an *ex vivo* microscopy image (bright-field and fluorescence) of the gastrointestinal (GI) tract of approximately 11-month old WT and 5xFAD mice, 3 hours after IP injection (1 ml) of EVs stained with Deep Red membrane stain. A bar graph of the Deep Red fluorescence intensity detected in the gastrointestinal tract is also shown. Deep Red was imaged using a 650-700 nm emission filter. (AU, arbitrary photon units) (N = 4-5; mean and SD)

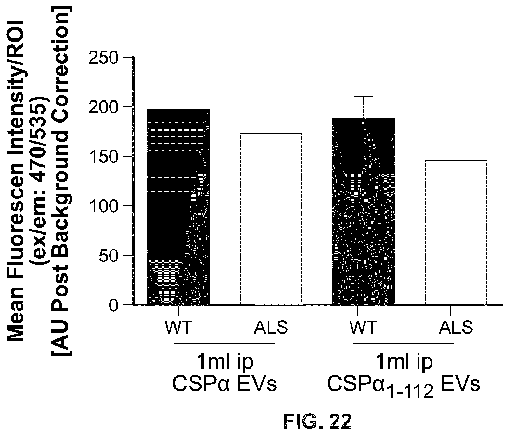

Fig. 22 is a graph of GFP fluorescence intensity detected *ex vivo* in the brains of approximately 6-month old mouse models of amyotrophic lateral sclerosis (ALS, mouse strain SOD-1^{G93A}, white bars) and age matched WT (black bars) mice, 3 hours after IP injection (1 ml) of EVs produced from cells expressing CSPα and GFP-tagged 72Q huntingtin^{exon1}, or from cells expressing a truncated form of CSPα (CSPα₁₋₁₁₂) and GFP-tagged 72Q huntingtin^{exon1}. GFP was imaged using a 470-535 nm emission filter. (AU, arbitrary photon units) (N = 1-3; mean and SEM)

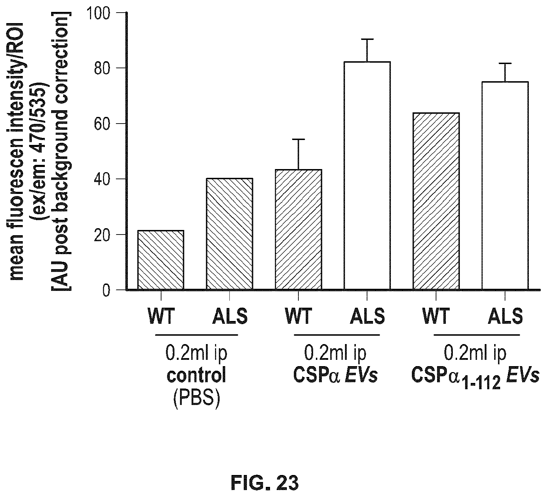

Fig. 23 is a graph of GFP fluorescence intensity detected *ex vivo* in the brains of approximately 6-month old ALS and WT mice, 3 hours after IP injection (1 ml) of phosphate-buffered saline (PBS, control); CSPα-EVs containing GFP-tagged 72Q huntingtin^{exon1}; or CSPα₁₋₁₁₂-EVs containing GFP-tagged 72Q huntingtin^{exon1}. GFP was imaged using a 470-535 nm emission filter. (AU, arbitrary photon units) (N = 1-5 per group; mean and SEM)

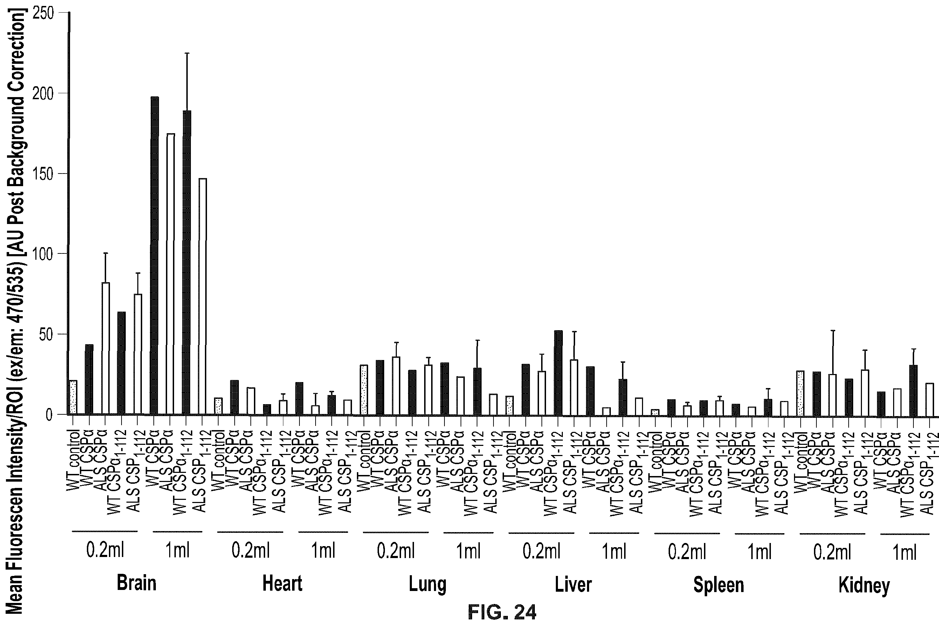

Fig. 24 is a graph of GFP fluorescence intensity detected *ex vivo* in the brain, heart, lung, liver, spleen and kidney of approximately 6-month old ALS and WT mice, 3 hours after IP injection (0.2 ml or 1 ml) of PBS (control); CSPα-EVs containing GFP-tagged 72Q huntingtin^{exon1}; or CSPα₁₋₁₁₂-EVs containing GFP-tagged 72Q huntingtin^{exon1}. GFP was imaged using a 470-535 nm emission filter. (AU, arbitrary photon units) (N = 1-5 per group; mean and SEM)

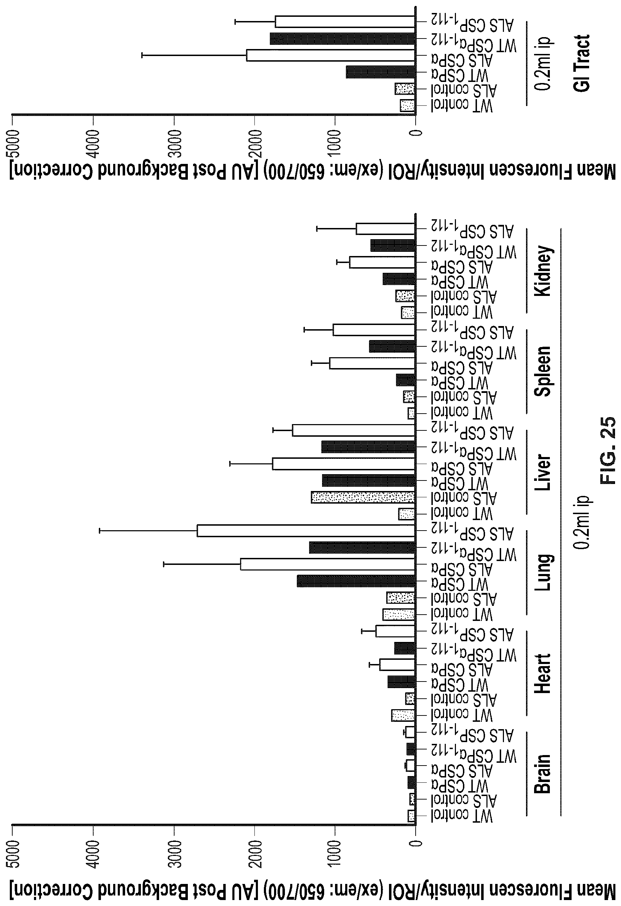

Fig. 25 is a set of graphs of Deep Red fluorescence intensity detected *ex vivo* in the brain, heart, lung, liver, spleen and kidney of approximately 6-month old ALS and WT mice, 3 hours after IP injection (0.2 ml or 1 ml) of PBS (control); CSPα-EVs containing GFP-tagged 72Q huntingtin^{exon1}; or CSPα₁₋₁₁₂-EVs containing GFP-tagged 72Q huntingtin^{exon1}. The gastrointenstinal (GI) tract was imaged separately using the same imaging parameters. Deep Red was imaged using a 650-700 nm emission filter. (AU, arbitrary photon units) (N = 1-5; mean and SD)

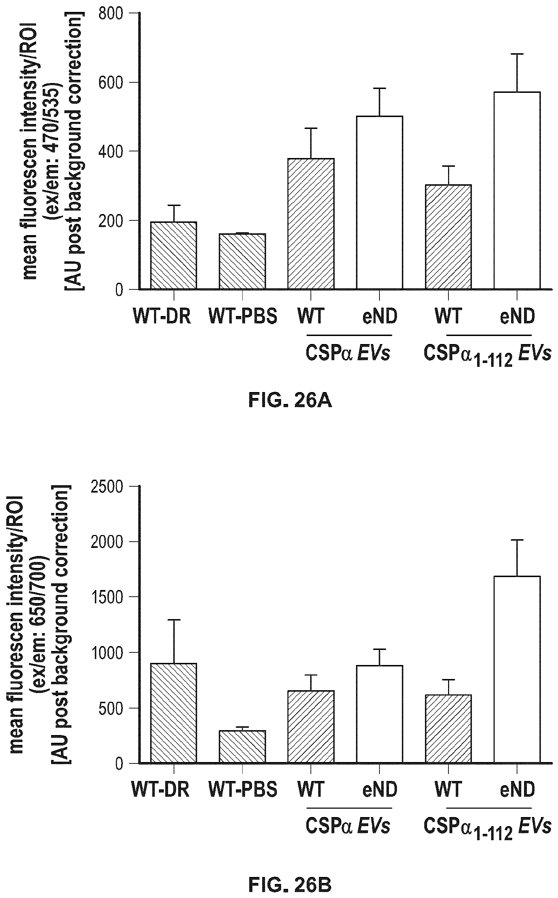

Fig. 26A is a graph of GFP fluorescence intensity detected *ex vivo* in the brains of 21-28-day old early neurodegeneration mouse models (eND, CSPα KO) and age-matched WT mice, 3 hours after IP injection (0.2 ml) of PBS (control); CSPα-EVs containing GFP-tagged 72Q huntingtin^{exon1}; or CSPα₁₋₁₁₂-EVs containing GFP-tagged 72Q huntingtin^{exon1}. GFP was imaged using a 470-535 nm emission filter. (AU, arbitrary photon units) (N = 3-12 per group; mean and SEM)
Fig. 26B is a graph of Deep Red fluorescence intensity detected in the brains of the eND and WT mice used in the *ex vivo* imaging experiment shown in Fig. 26A. Deep Red was imaged using a 650-700 nm emission filter. (AU, arbitrary photon units) (N = 3-12 per group; mean and SEM)

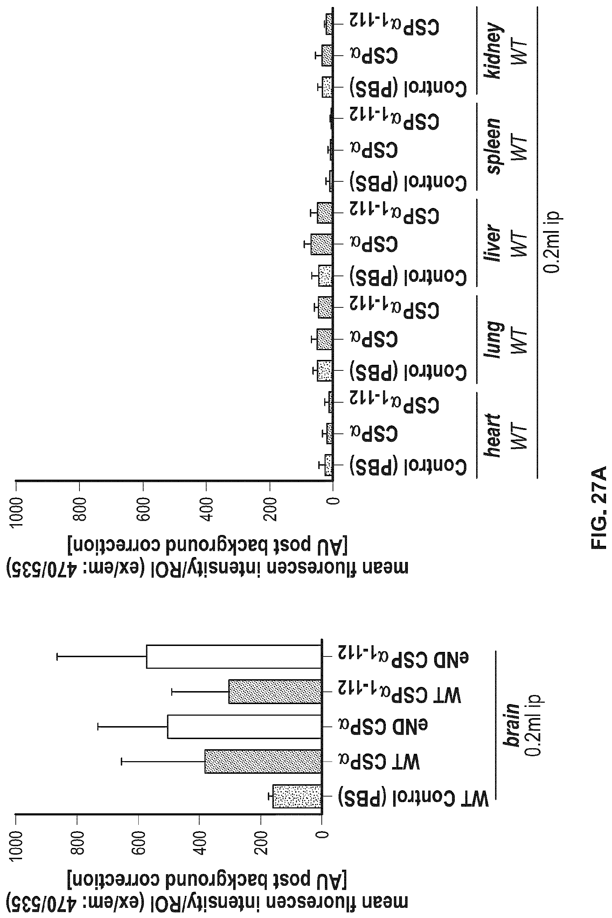

Fig. 27A is a set of graphs of GFP fluorescence intensity detected *ex vivo* in the brain, heart, lung, liver, spleen and kidney of 21-28-day old eND and WT mice, 3 hours after IP injection (0.2 ml) of PBS (control); CSPα-EVs containing GFP-tagged 72Q huntingtin^{exon1}; or CSPα₁₋₁₁₂-EVs containing GFP-tagged 72Q huntingtin^{exon1}. GFP was imaged using a 470-535 nm emission filter. (AU, arbitrary photon units) (N = 5-12 per group; mean and SD)

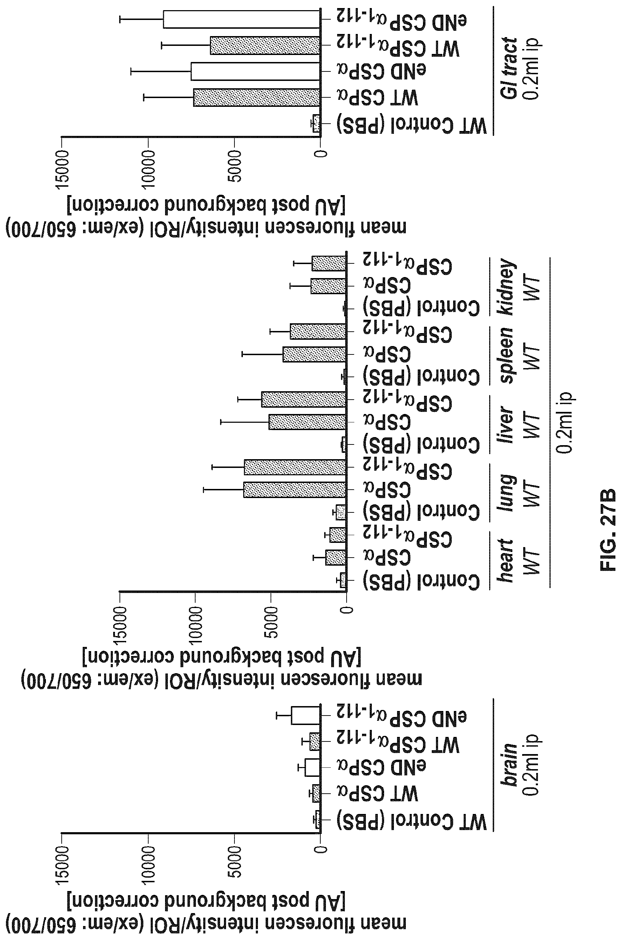

Fig. 27B is a set of graphs of Deep Red fluorescence intensity detected in the brain, heart, lung, liver, spleen, kidney and GI tract of the eND and WT mice used in the *ex vivo* imaging experiment shown in Fig. 26A. Deep Red was imaged using a 650-700 nm emission filter. (AU, arbitrary photon units) (N = 5-12 per group; mean and SD)

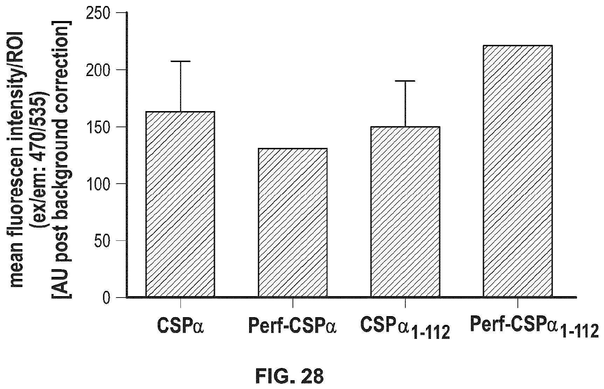

Fig. 28 is a graph of GFP fluorescence intensity detected *ex vivo* in the brains of 21-28-day old WT mice, 3 hours after IP injection (0.2 ml) of CSPα-EVs containing GFP-tagged 72Q huntingtin^{exon1} or CSPα₁₋₁₁₂-EVs containing GFP-tagged 72Q huntingtin^{exon1}, with or without intracardiac perfusion (perf) with 10 ml of PBS over a period of 5 minutes. GFP was imaged using a 470-535 nm emission filter. (AU, arbitrary photon units) (N = 2-4 per group; mean and SEM)

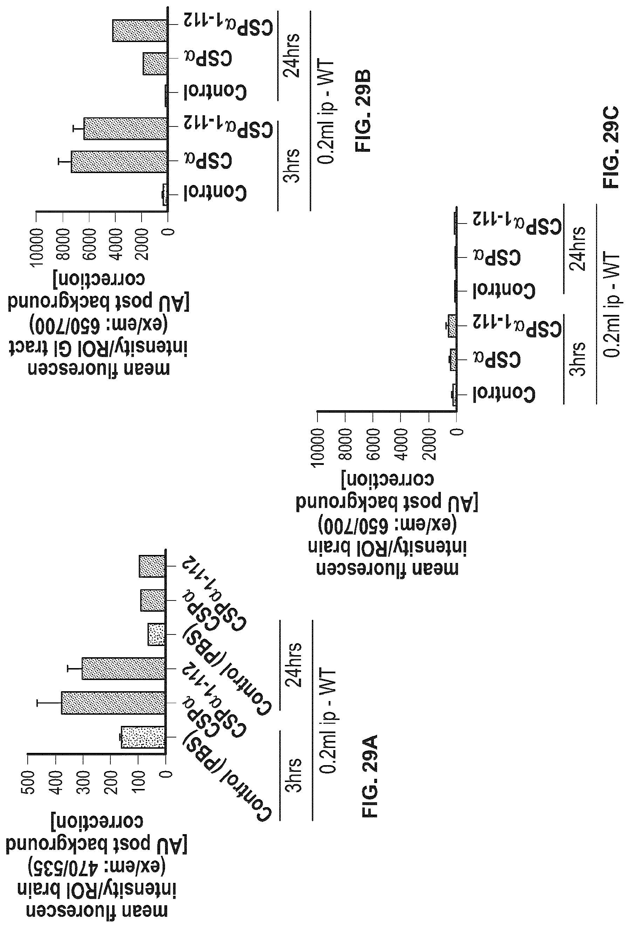

Fig. 29A is a graph of GFP fluorescence intensity detected *ex vivo* in the brains of 21-28-day old WT mice, 3 hours or 24 hours after IP injection (0.2 ml) of PBS (control); CSPα-EVs containing GFP-tagged 72Q huntingtin^{exon1}; or CSPα₁₋₁₁₂-EVs containing GFP-tagged 72Q huntingtin^{exon1}. GFP was imaged using a 470-535 nm emission filter. (AU, arbitrary photon units) (N = 2-12; mean and SEM)
Fig. 29B is a graph of Deep Red fluorescence intensity detected *ex vivo* in the GI tracts of the mice used in the experiment shown in Fig. 29A. Deep Red was imaged using a 650-700 nm emission filter. (AU, arbitrary photon units) (N = 2-12; mean and SEM)
Fig. 29C is a graph of Deep Red fluorescence intensity detected *ex vivo* in the brains of the mice used in the experiments shown in Figs. 29A and 29B. Deep Red was imaged using a 650-700 nm emission filter. (AU, arbitrary photon units) (N = 2-12; mean and SEM)

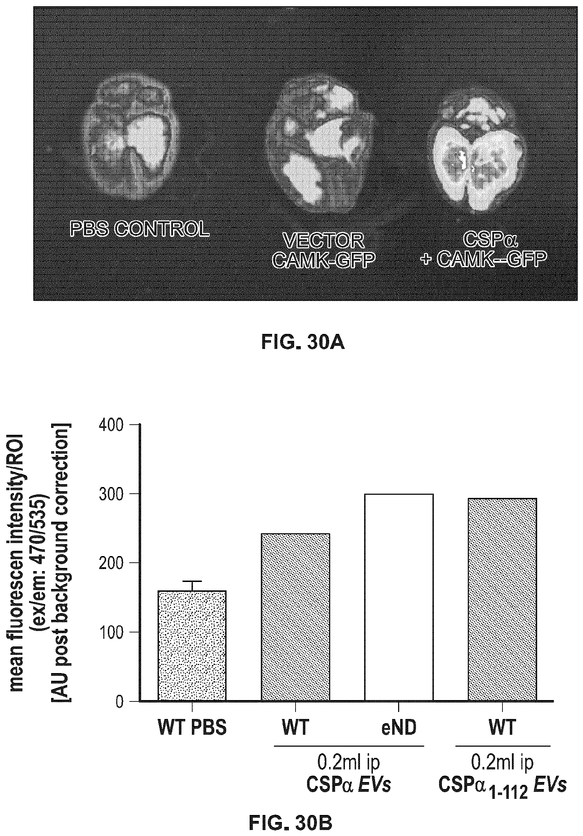

Fig. 30A is an *ex vivo* image overlay (black and white and fluorescent) of brains harvested from 21-28-day old eND (CSPα KO) mice 3 hours after IP injection (0.2 ml) of PBS, or EVs produced from cells expressing a vector control and GFP-tagged calcium/calmodulin-dependent protein kinase II alpha (CAMK) or from cells expressing CSPα and GFP-tagged CAMK.
Fig. 30B is a graph of GFP fluorescence intensity detected *ex vivo* in brains harvested from 21-28-day old WT or eND mice 3 hours after IP injection (0.2 ml) of PBS; CSPα-EVs containing GFP-tagged CAMK; or CSPα₁₋₁₁₂-EVs containing GFP-tagged CAMK. GFP was imaged using a 470-535 nm emission filter. (AU, arbitrary photon units) (N=2-5; mean and SD)

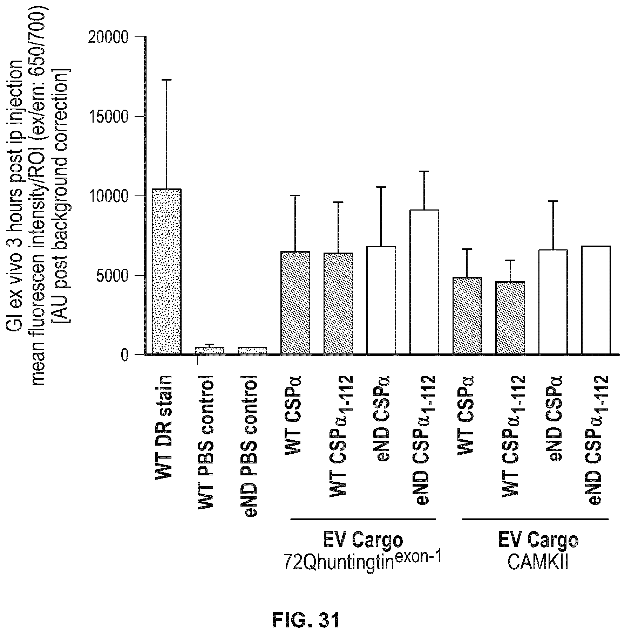

Fig. 31 is a graph of Deep Red fluorescence intensity detected *ex vivo* in GI tracts harvested from 21-28-day old WT or eND (CSPα KO) mice 3 hours after IP injection (0.2 ml) of PBS; CSPα-EVs containing GFP-tagged 72Q huntingtin^{exon1} or CAMK; or CSPα₁₋₁₁₂-EVs containing GFP-tagged 72Q huntingtin^{exon1} or CAMK. Deep Red was imaged using a 650-700 nm emission filter. (AU, arbitrary photon units) (N = 2-12; mean and SD)

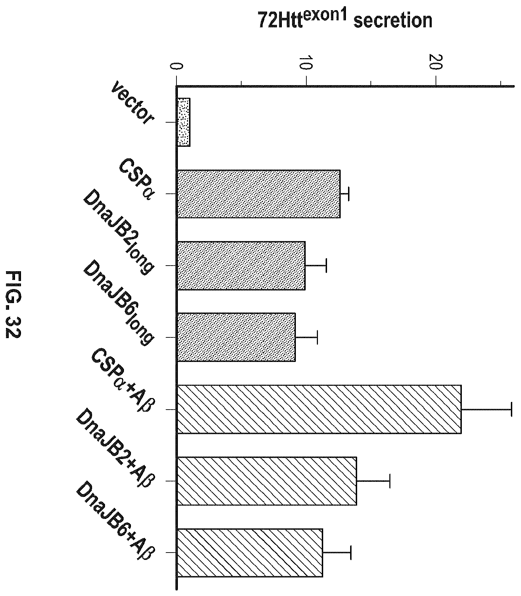

Fig. 32 is a graph of 72Q huntingtin^{exon1} secretion from CAD cells expressing GFP-72Q huntingtin^{exon1} and one of CSPα, DnaJB2_{long}, or DnaJB6_{long}. The cells were cultured in the presence or absence of 12.5 µg/ml β amyloid peptide (Aβ, AggreSure^{™} obtained from AnaSpec) for 48 hours. (N = 4; mean and SEM)

Other features and advantages of the present disclosure will become more apparent from the following detailed description and from the exemplary embodiments.

### DETAILED DESCRIPTION OF THE DISCLOSURE

Generally, the present disclosure provides a method of delivering an agent across the blood-brain barrier; a method of delivering an agent to the CNS; a method of treating a CNS disease or disorder; an agent delivery vehicle for same; and an extracellular vesicle for same.

### Abbreviations

The following abbreviations are used in the present disclosure:
- AD:: Alzheimer's disease
- ALS:: amyotrophic lateral sclerosis
- ANCL:: adult neuronal ceroid lipofuscinosis
- AU:: arbitrary photon units
- CAD cells:: catecholaminergic derived central nervous system cells
- CAMK:: calcium/calmodulin-dependent protein kinase II alpha
- CNS:: central nervous system
- CSPα:: cysteine string protein alpha (also known as DnaJC5)
- DNA:: deoxyribonucleic acid
- DnaJB2:: DnaJ Heat Shock Protein Family (Hsp40) Member B2
- DnaJB6:: DnaJ Heat Shock Protein Family (Hsp40) Member B6
- DnaJC5:: DnaJ Heat Shock Protein Family (Hsp40) Member C5 (also known as CSPα)
- DnaJC7:: DnaJ Heat Shock Protein Family (Hsp40) Member C7
- DnaJC10:: DnaJ Heat Shock Protein Family (Hsp40) Member C10
- EV(s):: extracellular vesicle(s)
- FACS:: fluorescence-activated cell sorting
- GFP:: green fluorescent protein
- GI tract:: gastrointestinal tract
- Hsc:: heat shock cognate protein
- Hsp:: heat shock protein
- Htt:: huntingtin
- IP:: intraperitoneal
- IV:: intravenous
- KO:: knock-out (genotype)
- NTA:: nanoparticle tracking analysis
- PBS:: phosphate-buffered saline
- RNA:: ribonucleic acid
- SD:: standard deviation
- SDS-PAGE:: sodium dodecyl sulfate polyacrylamide gel electrophoresis
- SEM:: standard error of the mean
- TDP-43:: TAR DNA-binding protein of 43 kDa
- TEM:: transmission electron microscopy
- WT:: wild-type (genotype)

### Definitions

Unless defined otherwise, all technical and scientific terms used herein generally have the same meaning as commonly understood by one of ordinary skill in the art to which this disclosure belongs.

As used herein, the singular forms "a", "an", and "the", are intended to include the plural forms as well, unless the context clearly indicates otherwise.

The phrase "and/or" should be understood to mean "either or both" of the elements so conjoined, i.e., elements that are conjunctively present in some cases and disjunctively present in other cases.

As used herein, the phrase "one or more", in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "one or more" refers, whether related or unrelated to those elements specifically identified.

When the term "about" is used in conjunction with a numerical range, it modifies that range by extending the boundaries above and below those numerical values. In general, the term "about" is used herein to modify a numerical value above and below the stated value by a variance of 20%, 10%, 5%, or 1%. In certain embodiments, the term "about" is used to modify a numerical value above and below the stated value by a variance of 10%. In certain embodiments, the term "about" is used to modify a numerical value above and below the stated value by a variance of 5%. In certain embodiments, the term "about" is used to modify a numerical value above and below the stated value by a variance of 1 %.

When a range of values is listed herein, it is intended to encompass each value and sub-range within that range. For example, "1-5 ng" is intended to encompass 1 ng, 2 ng, 3 ng, 4 ng, 5 ng, 1-2 ng, 1 -3 ng, 1-4 ng, 1 -5 ng, 2-3 ng, 2-4 ng, 2-5 ng, 3-4 ng, 3-5 ng, and 4-5 ng.

It will be further understood that the terms "comprises", "comprising", "includes," and/or "including", when used herein, specify the presence of stated features, integers, steps, operations, elements, and/or components, but do not preclude the presence or addition of one or more other features, integers, steps, operations, elements, components, and/or groups thereof.

The term "treatment," "treat", "treating" or "amelioration" as used herein is an approach for obtaining beneficial or desired clinical results. For purposes of this disclosure, beneficial or desired clinical results include, but are not limited to, one or more of the following: decreased extent of damage from a disease, condition, or disorder, decreased duration of a disease, condition, or disorder, and/or reduction in the number, extent, or duration of symptoms related to a disease, condition, or disorder. The term includes the administration of the compounds, agents, drugs or pharmaceutical compositions of the present disclosure to prevent or delay the onset of one or more symptoms, complications, or biochemical indicia of a disease or condition; lessening or improving one or more symptoms; shortening or reduction in duration of a symptom; or arresting or inhibiting further development of a disease, condition, or disorder. Treatment may be prophylactic (to prevent or delay the onset of a disease, condition, or disorder, or to prevent the manifestation of clinical or subclinical symptoms
thereof) or therapeutic suppression or alleviation of symptoms after the manifestation of the disease, condition, or disorder.

A "subject" is a vertebrate, preferably a mammal (e.g., a non-human mammal), and still more preferably a human. Vertebrates include, but are not limited to, humans; primates; farm animals (e.g., dairy cattle, beef cattle, pigs, sheep, chicken, turkey and fish); sport animals (e.g., horses and camels); and companion animals / pets (e.g., canines, felines and birds). The disclosure describes that the subject has or is at risk of having a CNS disease or disorder. For example, the subject may inherit a genetic predisposition for a CNS disease or disorder; the subject may contract or be at risk of contracting a CNS disease or disorder; or the subject may develop a CNS disease or disorder because of sporadic mutation, aging, environmental exposure(s), or because of other/unknown reasons.

The term "administering" as used herein refers to the placement of an agent, a drug, a compound, or a pharmaceutical composition as disclosed herein into and/or onto a subject by a method or route which results in at least partial delivery of the composition to a desired site. The EVs and the agent delivery vehicle disclosed herein can be administered by any appropriate route which results in an effective treatment in the subject. For example, the EVs and the agent delivery vehicle disclosed herein can be administered to a site other than the CNS. Possible routes of administration of the extracellular vesicles and the agent delivery vehicle disclosed herein include, but are not limited to, intravenous, intraperitoneal, intramuscular, subcutaneous, transdermal, oral, buccal, sublingual, intraocular, intranasal, or rectal routes of administration, or a combination thereof.

The term "effective amount" as used herein is an amount sufficient to affect any one or more beneficial or desired results. In more specific aspects, an effective amount may prevent, alleviate or ameliorate symptoms of a disease, condition, or disorder. For prophylactic use, beneficial or desired results may include eliminating or reducing the risk, lessening the severity, or delaying the onset of a CNS disease or disorder, including biochemical, histological and/or behavioral symptoms of the disease, condition, or disorder, its complications and intermediate pathological phenotypes presenting during development of the disease, condition, or disorder. For therapeutic use, beneficial or desired results may include clinical results such as reducing one or more symptoms of a CNS disease or disorder; decreasing the dose of other medications required to treat the disease, condition, or disorder; enhancing the effect of another medication; and/or delaying the progression of the disease condition, or disorder in a subject. An effective amount of an agent delivery vehicle may be an amount sufficient to deliver an agent across the blood-brain barrier, to deliver an agent to the CNS, and/or to treat, prevent, alleviate or ameliorate a CNS disease or disorder. An effective amount of EVs according to this disclosure may be an amount sufficient to deliver an agent across the blood-brain barrier, to deliver an agent to the CNS, and/or to treat, prevent, alleviate or ameliorate a CNS disease or disorder. An effective amount of EVs comprises one or more extracellular vesicles. An effective amount can be administered in one or more than one dose, round of administration, or course of treatment.

For purposes of this disclosure, an effective dosage of an agent, a drug, a compound, or a pharmaceutical composition is an amount sufficient to accomplish prophylactic or therapeutic treatment either directly or indirectly. As is understood in the clinical context, an effective dosage of an agent, a drug, a compound, or a pharmaceutical composition may or may not be achieved in conjunction with another agent, drug, compound, or pharmaceutical composition. Thus, an "effective dosage" may be considered in the context of administering one or more therapeutic agents, and a single agent may be considered to be given in an effective amount if, in conjunction with one or more other agents, a desirable result may be or is achieved. The amount may vary from one subject to another and may depend upon one or more factors, such as, for example, subject gender, age, body weight, subject's health history, and/or the underlying cause of the disease, condition, or disorder to be prevented, inhibited and/or treated.

The term "CNS disease or disorder" as used herein refers to a disease, disorder or condition affecting the central nervous system. The CNS disease or disorder may be inherited (i.e., genetic), contracted or otherwise acquired, sporadic, age-related, or idiopathic. The CNS disease or disorder may be a neurodegenerative, psychiatric, neurodevelopmental, or motor disease, disorder or condition. Examples of CNS diseases or disorders include, but are not limited to, Alzheimer's disease, Huntington's disease, Parkinson's disease, amyotrophic lateral sclerosis, dementia, multiple sclerosis, epilepsy, a demyelinating disorder; a myelopathy, a chaperonopathy, an autoimmune disorder, a prion disease, addiction, depression, an anxiety disorder, obsessive-compulsive disorder, attention deficit hyperactivity disorder, Tourette's syndrome, bipolar affective disorder, schizophrenia, Asperger syndrome, autism, CNS infection, CNS trauma, or stroke.

The term "extracellular vesicle" or "EV" as used herein refers to a phospholipid bilayer membrane-delimited particle that is released by a cell. *In vivo,* EVs mediate the cell-to-cell transfer of complex cargos including proteins, lipids and nucleic acids. EVs can be heterogeneous in size and composition. For example, small EVs measuring about 40 to 130
nm in diameter are called exosomes. Exosomes are formed by inward budding of the endosomal membrane to generate intraluminal vesicles within multivesicular bodies. EVs that are intermediate in size, measuring about 100 to 1 ,000 nm in diameter, are called microvesicles. Microvesicles are formed by outward budding of the plasma membrane. Large EVs measuring more than 1 pm are called exophers or large extracellular vesicles. The EVs may be exosomes, microvesicles, exophers, large extracellular vesicles, or combinations thereof or mixtures thereof. The term "EVs" as used herein may refer to a heterogeneous population comprising EVs of various sizes. The term "EVs" as used herein may refer to a size-sorted (or otherwise purified) substantially homogeneous population comprising EVs which: are of the same size, are similar in size, or are within a predefined range of sizes. Methods of making a substantially homogeneous population of EVs include, but are not limited to, fluorescence-activated cell sorting (FACS) or various size-specific filtration techniques.

EVs may be obtained by any known method of vesicle preparation and/or purification. EVs may be prepared from various cell types, including established cell lines and primary cells derived from any tissue. EVs may be produced from catecholaminergic derived CNS (CAD) cells. EVs may be produced from cells derived from a subject/patient in need of treatment for a CNS disease or disorder, and/or treatment with an agent that is to be delivered across the blood-brain barrier, in order to ensure immunocompatibility and minimize immune rejection or destruction of the EVs. The cells from which EVs are produced may be transfected, electroporated or otherwise made to express one or more DnaJ-domain containing protein chaperones, such as CSPα. The cells from which EVs are produced may be generically modified, engineered or edited. Methods of genetic modification, engineering or editing are known to those of ordinary skill in the art. Examples include, but are not limited to, random mutagenesis; targeted mutagenesis using recombinant DNA techniques, site-specific nucleases and the like; zinc-finger nucleases (ZFNs); transcription activator-like effector nucleases (TALENs); and clustered regularly interspaced short palindromic repeats (CRISPR/Cas9) technology.

EVs may carry different cargo. EVs comprise one or more DnaJ domain-containing protein chaperones (e.g., a CSPα, DnaJB2, DnaJB6, DnaJC7 and/or DnaJC10 polypeptide), and carry one or more agents useful for therapeutic treatment of a CNS disease or disorder. EVs comprise a CSPα polypeptide or a fragment thereof and carry one or more agents useful for therapeutic treatment of a CNS disease or disorder. The agent useful for treatment of a CNS disease or
disorder may be chemically synthesized or naturally derived. The agent useful for treatment of a CNS disease or disorder may be a peptide, a polypeptide, a nucleic acid, a small molecule drug, or a combination thereof. The agent useful for treatment of a CNS disease or disorder may be encapsulated or packaged within the EVs comprising one or more DnaJ domain-containing protein chaperones according to the methods described herein. For example, where the agent is a therapeutic polypeptide (e.g., an antibody or antigen-binding fragment thereof), the polypeptide may be expressed in cells by any known method (including endogenous and exogenous expression methods). The polypeptide may be encapsulated or packaged in various types of vesicles, which are released/secreted from the cells as EVs. The agent useful for treatment of a CNS disease or disorder may be externally applied to cells by adding the agent to a growth medium used for incubating of the cells, or to a buffer used for resuspending and/or storing the cells. This leads to uptake and encapsulation/packaging of the agent into EVs for export from the cells (i.e., release/secretion of EVs containing the 3agent). Generally, the agent useful for treatment of a CNS disease or disorder may be introduced to the cells or expressed in the cells by any method that leads to the encapsulation/packaging and export of the agent in one or more types of EVs (including exosomes, microvesicles, exophers, large extracellular vesicles, or combinations thereof).

EVs comprising one or more DnaJ domain-containing protein chaperones and carrying one or more agents useful for therapeutic treatment of a CNS disease or disorder may be produced, for example, using the methods described herein. The presently disclosed EVs may be produced from human cells or from cells derived from other mammals including, but not limited to, non-human primate, simian, bovine, ovine, porcine or murine cells. The presently disclosed EVs may be produced from human cells, including from cells derived from a human patient undergoing the treatment and drug delivery methods disclosed herein, so as to ensure histocompatibility and avoid immune rejection of the EVs. The EVs may be produced from cells derived from various organs and/or tissues. The EVs may be produced from various cell types, e.g., fibroblasts.

The term "DnaJ domain-containing protein chaperone", also known as "DnaJ protein chaperone", "J protein chaperone", or "J protein" refers to a member of a family of chaperones that comprise a DnaJ domain. The DnaJ domain is also known as the J domain and the heat shock protein 40 (Hsp40) domain. DnaJ domain-containing protein chaperones function in many cellular processes by regulating the ATPase activity of 70 kDa heat shock proteins, such as Hsc70 and Hsp70. Due to their interaction with 70 kDa heat shock proteins, DnaJ domain-containing protein chaperones are also referred to as co-chaperones. Various DnaJ domain-containing protein chaperones have diverse roles in proteostasis in vivo. Disruption or absence of a number of different DnaJ domain-containing protein chaperones is known to compromise neuronal function. Mass spectrometry analysis of EVs released from CAD cells revealed that these vesicles comprise at least the following DnaJ domain-containing protein chaperones: CSPα, DnaJB2, DnaJB6, DnaJC7 and DnaJC10. The disclosure, the DnaJ domain-containing protein chaperone is any one or more of CSPα, DnaJB2, DnaJB6, DnaJC7 and DnaJC10. In an embodiment, the DnaJ domain-containing protein chaperone is a CSPα polypeptide or a fragment thereof. In certain embodiments, the DnaJ domain-containing protein chaperone is a CSPα polypeptide, or a fragment thereof, in combination with one or more of DnaJB2, DnaJB6, DnaJC7 and DnaJC10 polypeptides, or fragments thereof. DnaJ domain-containing protein chaperones can be isolated from nature or can be produced by recombinant and/or synthetic means, as known to those of ordinary skill in the art. For example, a DnaJ domain-containing protein chaperone polypeptide may be cloned, expressed, and/or purified using molecular and biochemical techniques known in the art.

The term "fragment" refers to any part, portion, or segment of a full length protein that retains at least some of the functionality or activity of the full length protein. For example, a fragment of a DnaJ domain-containing protein chaperone retains the functionality required for the methods, the agent delivery vehicle, and the extracellular vesicle disclosed herein. Fragments of a DnaJ domain-containing protein chaperone will preferably have at least 80%, more preferably, at least 90%, and even more preferably, at least 95% amino acid sequence identity with at least one part of the native full length sequence of the DnaJ domain-containing protein chaperone. Fragments of a DnaJ domain-containing protein chaperone will generally be characterized by having the same type of activity as the naturally occurring DnaJ domain-containing protein chaperone, such as the ability, in the context of an EV comprising the fragment, to deliver agents across the blood-brain barrier and/or to the CNS. Various fragments of DnaJ domain-containing protein chaperones may be cloned, expressed, and/or purified using molecular and biochemical techniques known in the art. The fragment of CSPα may be CSPα₁₋₁₁₂ which is a truncated form comprising amino acid residues 1-112 of the full length polypeptide.

The term "cysteine string protein alpha" or "CSPα" also known as "cysteine string protein", "DnaJ Heat Shock Protein Family (Hsp40) Member C5" or "DnaJC5", as well as other names/designations, refers to the protein encoded by the DNAJC5 gene in humans, the
Dnajc5 gene in mice, or the corresponding (e.g., homologous or orthologous) gene in other animals. CSPα is a member of the DnaJ domain-containing protein chaperone family. In neurons, CSPα is a central player in the synapse-specific machinery that protects against local proteostasis imbalances at the synapse, which can lead to neurotransmission deficits, accumulation of misfolded, nonfunctional proteins, synaptic loss and neurodegeneration. Human mutations in CSPα, L115R and L116Δ, cause the neurodegenerative disorder adult neuronal ceroid lipofuscinosis (ANCL). Deletion of CSPα likewise leads to neurodegeneration in multiple experimental models. CSPα knock-out mice exhibit fulminant neurodegeneration and have a reduced lifespan with no mice surviving beyond about 3 months of age. Loss-of-function CSPα *Drosophila* mutants demonstrate uncoordinated movements, temperature sensitive paralysis and early lethality. Similarly, CSPα null C. *elegans* mutants display neurodegeneration and reduced lifespan. In humans, CSPα dysfunction has been implicated in several neurodegenerative disorders in addition to ANCL, including Alzheimer's disease, Parkinson's disease and Huntington's disease.

As used herein, "a CSPα polypeptide" refers to a mammalian CSPα, such as human, rat or mouse, as well as non-human primate, simian, bovine, ovine or porcine CSPα. The CSPα may be human CSPα (see, e.g., Accession Number NP_079495.1) and is a polypeptide comprising the amino acid sequence shown in SEQ ID NO: 1 , or is encoded by a nucleic acid molecule comprising the polynucleotide sequence shown in SEQ ID NO: 2. The CSPα polypeptide may be murine (see, e.g., Accession Number NP_058055.1) and is a polypeptide comprising the amino acid sequence shown in SEQ ID NO: 3, or is encoded by a nucleic acid molecule comprising the polynucleotide sequence shown in SEQ ID NO: 4. A CSPα polypeptide can be isolated from nature or can be produced by recombinant and/or synthetic means, as known to those of ordinary skill in the art. For example, a CSPα polypeptide may be cloned, expressed, and/or purified using molecular and biochemical techniques known in the art.

The term "CSPα polypeptide" also encompasses portions, variants, isoforms, and other homologs of such CSPα molecules. A variant of CSPα includes those sequences, wherein one or more nucleotides of the polynucleotide sequence or amino acids of the polypeptide sequence have been substituted, deleted, and/or inserted, that encode a functional CSPα polypeptide (i.e., a CSPα variant that retains the functionality required for the methods, the agent delivery vehicle, and the extracellular vesicle disclosed herein). Variants of CSPα will preferably have at least 80%, more preferably, at least 90%, and even more preferably, at least 95% amino acid sequence identity with the native sequence CSPα
polypeptides. Variant CSPα molecules will generally be characterized by having the same type of activity as naturally occurring CSPα, such as the ability, in the context of an EV comprising the variant CSPα, to deliver agents across the blood-brain barrier and/or to the CNS. CSPα variant polypeptides can be isolated from nature or can be produced by recombinant and/or synthetic means, as known to those of ordinary skill in the art. For example, CSPα variant polypeptides may be cloned, expressed, and/or purified using molecular and biochemical techniques known in the art.

As used herein, "a DnaJB2 polypeptide" refers to a mammalian DnaJB2, such as human, rat or mouse, as well as non-human primate, simian, bovine, ovine or porcine DnaJB2. The DnaJB2 may be human DnaJB2 (see, e.g., Accession Number NP_006727.2) and is a polypeptide comprising the amino acid sequence shown in SEQ ID NO: 5, or is encoded by a nucleic acid molecule comprising the polynucleotide sequence shown in SEQ ID NO: 6. The DnaJB2 polypeptide may be murine (see, e.g., Accession Number NP_001153355.1) and is a polypeptide comprising the amino acid sequence shown in SEQ ID NO: 7, or is encoded by a nucleic acid molecule comprising the polynucleotide sequence shown in SEQ ID NO: 8. The term "DnaJB2 polypeptide" also encompasses portions, variants, isoforms, and other homologs of such DnaJB2 molecules.

As used herein, "a DnaJB6 polypeptide" refers to a mammalian DnaJB6, such as human, rat or mouse, as well as non-human primate, simian, bovine, ovine or porcine DnaJB6. The DnaJB6 may be human DnaJB6 (see, e.g., Accession Number 075190.2) and is a polypeptide comprising the amino acid sequence shown in SEQ ID NO: 9, or is encoded by a nucleic acid molecule comprising the polynucleotide sequence shown in SEQ ID NO: 10. The DnaJB6 polypeptide may be murine (see, e.g., Accession Number NP_001033029.1) and is a polypeptide comprising the amino acid sequence shown in SEQ ID NO: 1 1 , or is encoded by a nucleic acid molecule comprising the polynucleotide sequence shown in SEQ ID NO: 12. The term"DnaJB6 polypeptide" also encompasses portions, variants, isoforms, and other homologs of such DnaJB6 molecules.

As used herein, "a DnaJC7 polypeptide" refers to a mammalian DnaJC7, such as human, rat or mouse, as well as non-human primate, simian, bovine, ovine or porcine DnaJC7. The DnaJC7 may be human DnaJC7 (see, e.g., Accession Number NP_003306.3) and is a polypeptide comprising the amino acid sequence shown in SEQ ID NO: 13, or is encoded by a nucleic acid molecule comprising the polynucleotide sequence shown in SEQ ID NO: 14. The DnaJC7 polypeptide may be murine (see, e.g., Accession Number NP_062769.2) and is a polypeptide comprising the amino acid sequence shown in SEQ ID NO: 15, or is encoded by a nucleic acid molecule comprising the polynucleotide sequence shown in SEQ ID NO: 16. The term "DnaJC7 polypeptide" also encompasses portions, variants, isoforms, and other homologs of such DnaJC7 molecules.

As used herein, "a DnaJC10 polypeptide" refers to a mammalian DnaJC10, such as human, rat or mouse, as well as non-human primate, simian, bovine, ovine or porcine DnaJC10. The DnaJC10 may be human DnaJC10 (see, e.g., Accession Number NP_061854.1) and is a polypeptide comprising the amino acid sequence shown in SEQ ID NO: 17, or is encoded by a nucleic acid molecule comprising the polynucleotide sequence shown in SEQ ID NO: 18. The DnaJC10 polypeptide may be murine (see, e.g., Accession Number NP_077143.2) and is a polypeptide comprising the amino acid sequence shown in SEQ ID NO: 19, or is encoded by a nucleic acid molecule comprising the polynucleotide sequence shown in SEQ ID NO: 20. The term "DnaJC10 polypeptide" also encompasses portions, variants, isoforms, and other homologs of such DnaJC10 molecules.

DnaJB2, DnaJB6, DnaJC7 and/or DnaJC10 polypeptides and variants thereof can be isolated from nature or can be produced by recombinant and/or synthetic means, as known to those of ordinary skill in the art. For example, a DnaJB2, a DnaJB6, a DnaJC7 and/or a DnaJC10 polypeptide or a variant thereof may be cloned, expressed, and/or purified using molecular and biochemical techniques known in the art.

The term "an agent useful for treatment of a CNS disease or disorder" refers to any agent (e.g., a molecule) or group, combination, or mixture of agents, which is being used, has been used or may be used for treating, preventing or ameliorating a disease, condition, or disorder of the CNS. The agent useful for treatment of a CNS disease or disorder may be encapsulated or packaged within the EVs comprising one or more DnaJ domain-containing protein chaperones. The one or more DnaJ domain-containing protein chaperones may directly or indirectly interact with, or bind to, the agent useful for treatment of a CNS disease or disorder. The agent useful for treatment of a CNS disease or disorder may be chemically synthesized or naturally derived. The agent useful for treatment of a CNS disease or disorder may be a peptide, a polypeptide, a nucleic acid, a small molecule drug, or a combination thereof.

Examples of peptides useful for treatment of a CNS disease or disorder include, but are not limited to, peptides that block channels used to treat neuropathic pain (e.g., ziconotide / Prialt^{™}) and peptides that alter nitric oxide production used to treat stroke (e.g., nerinetide). Where the agent useful for treatment of a CNS disease or disorder is a polypeptide, the polypeptide may be a structural protein, an enzyme, or an antibody or antigen-reactive fragment thereof. For example, the agent may be an antibody or an antibody fragment reactive with a neurotoxic protein, such as a prion, an amyloid peptide, α-synuclein, superoxide dismutase, TAR DNA-binding protein of 43 kDa (TDP-43), or tau. Where the agent useful for treatment of a CNS disease or disorder is a nucleic acid, the nucleic acid may be DNA, RNA, or a derivative thereof (e.g., peptide nucleic acid (PNA)), for example, a silencing antisense oligonucleotide (e.g., RG6042) for treating Huntington's disease.

Where the agent useful for treatment of a CNS disease or disorder is a small molecule drug, the small molecule may be an existing or experimental pharmaceutical and/or veterinary drug or prodrug. The agent may be a small molecule drug that inhibits, inactivates, or prevents the accumulation of a neurotoxic protein, such as a prion, an amyloid peptide, α-synuclein, superoxide dismutase, TDP-43, or tau. The agent may be an anesthetic, an anticonvulsant, an antiemetic, an antidepressant, an anxiolytic, a CNS stimulant or depressant, a muscle relaxant, a narcotic or non-narcotic analgesic, or a sedative. Examples of small molecules useful for treatment of a CNS disease or disorder include, but are not limited to, TRPV1 receptor antagonists (e.g., capsazepine) and nitric-oxide synthase inhibitors (e.g., Nitro-L-arginine methyl ester (L-NAME) for pain; microglia inhibitors (e.g., senicapoc) for Alzheimer's disease; various small molecules currently used to treat peripheral neuropathy that may also be useful for treating CNS diseases or disorders; and other agents that were not advanced to clinical trials because they did not cross the blood-brain barrier when used with prior delivery methods.

Resveratrol (which is naturally found in, e.g., red wine, but which can also be synthesized by chemical methods) is a multi-target polyphenol with anti-inflammatory, cardioprotective, anti-tumor and neuroprotective properities. Resveratrol is found in many chemical libraries used for drug screens. A functional link between resveratrol and the CSPα ortholog in C. elegans has been previously identified (Kashyap et al (2014) Hum. Mol. Genet. 23, 5916-5927).

The term "pharmaceutically acceptable carrier, diluent, or excipient" as used herein includes any material which, when combined with an active ingredient, allows the ingredient to retain biological activity and is non-reactive with the subject's immune system. Examples include, but are not limited to, any of the standard pharmaceutical carriers such as a phosphate buffered saline solution, water, emulsions such as oil/water emulsion, and various types of wetting agents. Diluents for aerosol or parenteral
administration may be phosphate buffered saline (PBS) or normal (0.9%) saline. Compositions comprising such carriers are formulated by well-known conventional methods (see, for example, Remington's Pharmaceutical Sciences, 18th edition, A. Gennaro, ed., Mack Publishing Co., Easton, PA, 1990; and Remington, The Science and Practice of Pharmacy 20th Ed. Mack Publishing, 2000).

### General Techniques

Unless otherwise defined herein, scientific and technical terms used in connection with the present disclosure shall have the meanings that are commonly understood by those of ordinary skill in the art. Generally, nomenclatures used in connection with, and techniques of, cell and tissue culture, molecular biology, immunology, microbiology, genetics and protein and nucleic acid chemistry and hybridization described herein are those well-known and commonly used in the art.

The practice of the present disclosure will employ, unless otherwise indicated, conventional techniques of molecular biology (including recombinant techniques), microbiology, cell biology, biochemistry and immunology, which are within the skill of the art. Such techniques are explained fully in the literature, such as, Molecular Cloning: A Laboratory Manual, second edition (Sambrook et al., 1989) Cold Spring Harbor Press; Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney, ed., 1987); Introduction to Cell and Tissue Culture (J.P. Mather and P.E. Roberts, 1998) Plenum Press; Cell and Tissue Culture: Laboratory Procedures (A. Doyle, J.B. Griffiths, and D.G. Newell, eds., 1993-1998) J. Wiley and Sons; Methods in Enzymology (Academic Press, Inc.); Handbook of Experimental Immunology (D.M. Weir and C.C. Blackwell, eds.); Gene Transfer Vectors for Mammalian Cells (J.M. Miller and M.P. Calos, eds., 1987); Current Protocols in Molecular Biology (F.M. Ausubel et al. , eds., 1987); PCR: The Polymerase Chain Reaction, (Mullis et al., eds., 1994); Current Protocols in Immunology (J.E. Coligan et al., eds., 1991); Sambrook and Russell, Molecular Cloning: A Laboratory Manual, 3rd. ed., Cold Spring Harbor Laboratory Press, Cold Spring Harbor, NY (2001); Ausubel et al., Current Protocols in Molecular Biology, John Wiley & Sons, NY (2002); Harlow and Lane Using Antibodies: A Laboratory Manual, Cold Spring Harbor Laboratory Press, Cold Spring Harbor, NY (1998); Coligan et al., Short Protocols in Protein Science, John Wiley & Sons, NY (2003); Short Protocols in Molecular Biology (Wiley and Sons, 1999); and Immunobiology (C.A. Janeway and P. Travers, 1997).

### Method and Vehicle for Delivering Agents Across the Blood-Brain Barrier and to the CNS

The present disclosure provides a method, extracellular vesicles and an agent delivery vehicle for delivering agents across the blood-brain barrier in a subject, for delivering agents to the CNS of a subject, and for treating a CNS disease or disorder in a subject. "Delivering" an agent across the blood-brain barrier and to the CNS, as used herein, refers to carrying, transporting, transferring or transmitting an agent from the bloodstream, through the blood-brain barrier and into the brain, spinal cord and/or cerebrospinal fluid of a subject. The delivering encompasses delivery of any amount or fraction of the administered agent across the blood-brain barrier and to the CNS. For example, about 0.001 %, 0.01 %, 0.1%, 1 %, 5%, 10%, 15%, 20%, 25%, 30%, 35%, 40%, 45%, 50%, 55%, 60%, 65%, 70%, 75%, 80%, 85%, 90%, 95%, or 100% of the administered agent may be delivered across the blood-brain barrier and to the CNS.

The methods, EVs and agent delivery vehicle disclosed herein may deliver agents only to the CNS, or deliver agents to the CNS and also to another tissue, organ or organ system. The methods, EVs and agent delivery vehicle may be completely selective or partially selective in targeting and delivering agents to the CNS relative to other tissues. Delivery of an agent to the CNS by the methods, EVs and agent delivery vehicle disclosed herein may be, for example 1.001 -fold, 1.01-fold, 1.1-fold, 1.5-fold, 2-fold, 5-fold, 10-fold, 100-fold, 1 ,000-fold, or 1 ,000,000-fold higher than delivery of the same agent to another (i.e., non-CNS) tissue or tissues in the same subject. The presently disclosed methods, EVs and agent delivery vehicle may selectively target and deliver agents to a specific part of the CNS, or to a group of parts of the CNS. For example, the presently disclosed methods, EVs and agent delivery vehicle may selectively target and deliver agents to a particular lobe or region of the brain.

The methods of delivering an agent across the blood-brain barrier and to the CNS comprise administering to the subject an effective amount of EVs comprising the agent and one or more DnaJ domain-containing protein chaperones, or fragments thereof. The EVs to be administered to a subject comprise CSPα, DnaJB2, DnaJB6, DnaJC7 and/or DnaJC10, or a fragment or fragments thereof. The EVs to be administered to a subject comprise a CSPα polypeptide or a fragment thereof. Methods for detecting the delivery of an agent across the blood-brain barrier and/or to the CNS are known to those of ordinary skill in the art, and include, for example, various imaging techniques. The delivery of an agent across the blood-brain barrier and/or to the CNS may be detected by magnetic resonance imaging (MRI) of EVs comprising the agent, one or more DnaJ domain-containing protein chaperones, and a contrast medium or dye.

The methods of delivering an agent across the blood-brain barrier and to the CNS disclosed herein may form part of a therapeutic method or regimen for treating a CNS disease or disorder in a subject. The subject who may benefit from the delivery method and vehicle disclosed herein may have a CNS disease or disorder only, or have a CNS disease or disorder and further have one or more other diseases, disorders or conditions affecting a different part of the body (e.g., a disease affecting a non-CNS tissue, organ or organ system). The non-CNS disease or disorder may modulate the susceptibility of a subject to the CNS disease or disorder, and vice versa. The EVs and the agent delivery vehicle of the present disclosure may be administered alone, in addition to, or in combination with other therapeutic agents or other delivery vehicles.

### EXAMPLES

The disclosure is further described in detail by reference to the following examples. These examples are provided for purposes of illustration only and are not intended to be limiting unless otherwise specified. Thus, the disclosure should in no way be construed as being limited to the following examples, but rather, should be construed to encompass any and all variations which become evident as a result of the teaching provided herein.

### EXAMPLE 1: Materials and Methods

### Preparation of Extracellular Vesicles (EVs)

Cell lines were transfected with the molecular chaperone CSPα and a GFP-tagged protein. CAD cells (catecholaminergic derived CNS cells) were the cell line utilized. Other cell lines, including patient-derived cell lines, may also be utilized. cDNAs encoding for myc-tagged CSPα or vector alone and GFP-72Q huntingtin^{exon1} were transiently transfected with Lipofectamine^{™} 3000 (Invitrogen) in Opti-MEM^{™} medium. Media was changed to serum-free media 6 hours post-transfection. Media was collected 48 hours after transfection, spun at 300Xg for 5 min to remove cell debris and evaluated without further processing by nanocyte tracking analysis, nanoscale flow cytometry analysis and dot blot analysis. For western blot analysis, EVs were isolated from the unfractionated media by exoquick precipitation solution (SBI) and solubilized in sample buffer. Following media collection, cell viability was determined using an XTT assay (New England Biolabs). When resveratrol was used, it was added to the serum-free media 6 hours post-transfection at the time of the media change.

For administration into mice, media was collected 48 hours after transfection and filtered sequentially through 40 µm, 10 µm and 1 µm nylon mesh filters, and the EV-containing filtrate was frozen at -20°C.

### Immunoblotting (Western Blotting)

Proteins were separated by SDS-PAGE and electrotransferred from polyacrylamide gels to nitrocellulose membrane (0.2 µm pore size). Membranes were blocked in tris-buffered saline (TBS) containing 0.1% Tween 20, 1% BSA and then incubated with primary antibody overnight at 4°C. The membranes were washed and incubated with horseradish peroxidase-coupled secondary antibody for about 2 hours at room temperature. Bound antibodies on the membranes were detected by incubation with Pierce chemiluminescent reagent and exposure to Cdigit, LiCor (Mandel). The chemiluminescent signals were quantified using image studio digits software (Mandel). For dot blots, 10 µl of unfractionated media was spotted on a nitrocellulose membrane, dried and the membrane was processed as described above.

### Plasmids

cDNAs encoding CSPα, CSPα mutants, and J proteins were expressed in the plasmid myc-pCMV. GFP-72Q huntingtin^{exon1} was expressed in the plasmid pcDNA3.1. All amplified regions of all plasmids were sequenced to ensure the absence of any undesired mutations.

### Transmission Electron Microscopy

Media was centrifuged at 2,000Xg for 10 min, and at 10,000Xg for 30 min to discard membrane and debris. The supernatant was then centrifuged at 100,000g for 2 hours and EVs collected. EVs were suspended in PBS, fixed sequentially on a 150 mesh grid in 4% paraformaldehyde followed by 2% glutaraldehyde and stained with 2% uranyl acetate for 10 minutes. EVs were imaged with a Tecnai F20 TEM and a Gaten CCD camera.

### Fluorescence Imaging

Images of live cells were taken with the EVOS FL Auto Imaging System. Images of donor cells were taken at the time of media collection. Images of recipient cells were taken between 1-48 hours. The intensity of the GFP-72Q huntingtin^{exon1} varied among cells and a single slider was used until the on-screen brightness of the lowest intensity aggregate was satisfactory and used this setting to capture images in high-quality mode. Where indicated, after the 300Xg for 5 minutes conditioned media spin to remove cell debris, 60 µl of Exo-Red (SBI) was incubated with 1 ml media containing EVs for 10 minutes at 37°C to label the RNA EV cargo. 150 µl of exo-quick was added, the labelled EVs were placed on ice for 30min and then centrifuged for 3 minutes at 20,000Xg. The labelled EV pellet was resuspended in 1 ml of DMEM-F12 media and applied to target cells.

### Nanoparticle Tracking Analysis

For NTA and nanoscale flow cytometry analysis, all samples were diluted 25 fold using 0.2x phosphate buffered saline that had been filtered twice through 0.2 µm filter. For NTA analysis, all samples were analyzed using the Nanosight LM10 (405 nm laser, 60 mW, software version 3.00064). Samples were analyzed in triplicate for 60 seconds per replicate with a count range of 20-100 particles per frame. A variety of NIST (Thermo Scientific 3000 Series) standards were analyzed, each the day prior to sample analysis. The system was cleaned between each sample and checked for any sample carryover using the PBS diluent. NTA measures EVs within a size range from about 30 nm to about 1,000 nm.

### Nanoscale Flow Cytometry

Samples were similarly prepared for nanoscale flow cytometry as for NTA. All samples were analyzed using the Apogee A50 flow cytometry platform. Light scatter was provided using the 405 nm laser (75 mW); GFP signal was generated using the 488 nm laser (50 mW, 535/35) and far red signal was generated using the 630 nm laser (75 mW, 680/35). All samples were analyzed for 60 seconds. Optimization was performed to insure single EVs were being analyzed and single events were triggered by light scatter only. The system was cleaned each day prior to sample analysis and a variety of silica and polystyrene standards (Apogee 1493 standards) processed for instrument set up and QC. The silica standards were used to assess the relative size range of EVs. Nanoscale flow cytometry measures EVs within a size range from about 100 nm to about 1,300 nm.

To demonstrate that the GFP-72Q huntingtin^{exon1} signal was associated with bona fide vesicles and not simply protein aggregates, samples were first mixed with CellMask^{™} Deep Red plasma membrane stain (Thermo Fisher Scientific (C10046), final concentration of 0.1X), incubated for 30 minutes at 37°C and then diluted in PBS. Palmitoylated-GFP positive EVs were obtained from the conditioned media of a PC3 prostate cancer cell line; the membrane of these EVs contains the FP proteins. Anti-CD-FITC antibody was used to generate protein aggregates. Antibody (10 µl) aliquots were incubated at 50°C for increasing periods of time to generate non-membrane, protein aggregates. These aggregates as well as the PC PALM-GFP controls were similarly stained with Deep Red cell mask. Aggregate concentration was analyzed by NTA and uptake of the membrane dye was measured using nanoscale flow cytometry. For these experiments, single particle detection was triggered using positive GFP/FITC fluorescence and the associated far red signal analyzed.

### Statistical Analysis

All data were graphed and statistically analyzed using GraphPad Prism version 6.01 for Windows, GraphPad Software, La Jolla California USA, www.graphpad.com. Statistics included One-Way and Two Way ANOVA with either Tukey's or Dunnett's post-test analysis if initial ANOVA was statistically significant (p<0.05; stars indicate significance *p<0.05, **p<0.01, ***p<0.001, ****p<0.0001). All values are presented as the mean +/- SEM where appropriate, otherwise the SD is presented as indicated.

### Whole Body Non-Invasive Imaging

At least 7 days prior to fluorescent screening/imaging the mice were fed a specific non-auto fluorescent diet (e.g., Harlan purified diet TD.94045 AIN-93G or TD.97184 AIN 93G) to eliminate any confounding fluorescence from lab chow.

To perform non-invasive whole body imaging efficiently animals have to be hairless. To do so mice were anesthetized with isoflurane (5% for induction; 2% for maintenance) and hair was completely removed related to the region of interest at least 24 hours prior to imaging. While under anesthesia hair was removed roughly via a clipper and afterwards removed completely using a skin friendly depilation crème. Afterwards mice were put back in their cage and placed on an animal heating pad to recover from anesthesia. Animals were monitored during recovery. At the time points of imaging animals were anesthetized with isoflurane (Isofluroane: 5% induction; 2% maintenance) and then transferred into the heated, anesthesia connected and ventilated whole body imaging chamber (BRUKER InVivoXtreme^{™} 4MP non-invasive whole body imaging device). Depending on the setup of the imaging protocol imaging lasts between 30 seconds to 5 minutes per screen. Multiple screens based on the site of exposure (ventral, lateral and dorsal), the angle of imaging (0 to 360 degrees) as well as the imaging modality (fluorescence or X-ray) were performed. After the imaging screen mice were returned to their cage and supervised during recovery. At the end of an experimental time line, mice were euthanized according to SOP E1. Organs were removed after euthanization to perform imaging *ex vivo.*

### Mouse Lines

5xFAD mice have mutant human amyloid precursor protein (APP(695)) with the Swedish (K670N, M671L), Florida (1716V), and London (V7171) mutations and mutant human presenilin1 (PS1) with, M146L and L286V. 5xFAD is considered an aggressive Alzheimer's disease model in that 5xFAD mice have impaired memory, rapidly accumulate Aβ42 and amyloid deposits start at about 3 months. 5xFAD mice and littermate WT controls were between 10-12 months old (i.e., 5xFAD mice were close to endpoint). Mice were treated intraperitoneally with 1 ml or intravenously (tail vein) with 0.2 ml of media containing EVs.

Cysteine string protein alpha knock-out (CSPα KO) mice, also referred to as early neurodegeneration (eND) mouse model, are normal at birth but undergo rapid fulminant neurodegeneration with no mice surviving beyond 3 months of age. CSPα KO and WT littermate controls were between 22-27 days old. 0.2 ml of media containing EVs was administered intraperitoneally.

SOD-1^{G93A} mice express a variant of the human superoxide dismutase 1 (*SOD1*) gene, which leads to paralysis due to a loss of motor neurons from the spinal cord. SOD-1^{G93A} mice have a reduced life expectancy and serve as a model for human amyotrophic lateral sclerosis (ALS).

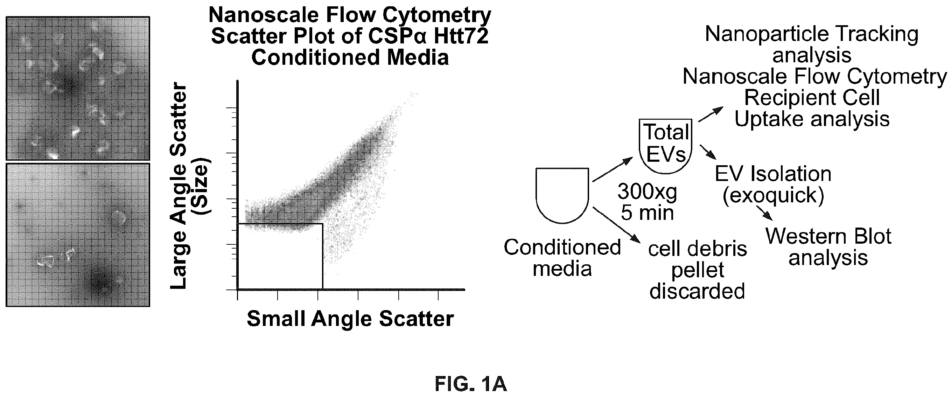

Prior to administration, media containing EVs was thawed on ice. Figs. 1-3 depict experiments involving direct administration of EVs. Figs. 4-6 depict experiments in which EVs were incubated with Deep Red plasma membrane stain (diluted 1:1000) for 10 minutes, free stain was removed through multiple rounds of centrifugation and resuspension in PBS prior to administration.

### EXAMPLE 2: Resveratrol reduces export of GFP-72Q huntingtin^{exon1} cargo by EVs comprising CSPα

First, EVs released from CAD neurons were characterized. The size distribution of CAD cell EVs was assessed by transmission electron microscopy (Fig. 1A, left panel). To characterize the heterogeneity of EVs by nanoscale flow cytometry and nanoparticle tracking analysis (NTA), media from CAD cells expressing CSPα and GFP-72Q huntingtin^{exon1} was collected, centrifuged at 300Xg for 5 minutes to remove cell debris and, without further processing, the EVs present in the media were evaluated (Fig. 1A, right panel). The heterogeneity of particles in this unfractionated media was evident by nanoscale flow cytometry (Fig. 1A, middle panel). The most frequent size distribution of EVs was found by NTA to be 144 +/- 2.7 nm, somewhat larger than the 129 +/- 2.2 nm EVs purified by differential centrifugation/exoquick techniques.

Next, it was determined which EVs contain misfolded-GFP cargo. Nanoscale flow cytometry analysis revealed that resveratrol increases total EV secretion from control, vector and CSPα-expressing cells (Fig. 1B). Secretion of EVs containing GFP-tagged 72Q huntingtin^{exon1} was dramatically increased when cells express CSPα (p < 0.0001) and this CSPα-EV export of EVs containing GFP-72Q huntingtin^{exon1} was significantly reduced by resveratrol (p < 0.0001). Comparison of the flow cytometry light scatter is shown in the right hand panel (Fig. 1B). The majority of EVs released from CSPα-expressing CAD cells in the absence of resveratrol were the smaller <180 nm, presumably exosome EVs (81.6%), while the 180-240 nm EVs and the >240 nm EVs were 17% and 1.4% respectively (Fig. 1C; Fig. 7). The bulk of EVs containing GFP-72Q huntingtin^{exon1} fall within the 180-240 nm range (71% (p < 0.0001)) and CSPα increased export of EVs containing GFP-72Q huntingtin^{exon1} within this subpopulation by >400% (p < 0.0001). In the presence of CSPα, EVs containing GFP cargo account for 0.06% (<180 nm), 1% (180-240 nm) and 1.6% (>240 nm) within their respective subpopulations. Resveratrol significantly reduced secretion of the 180-240 nm GFP-labelled EV pool in a concentration-dependent manner while increasing export of total EVs (Figs. 1B and C). In the presence of CSPα and 50 µM resveratrol, EVs containing GFP cargo account for 0.03% (<180 nm), 0.53% (180-240 nm) and 0.78% (>240 nm) of the respective subpopulations.

To investigate whether neurons secrete free floating GFP-tagged 72Q huntingtin^{exon1} entities, in addition to GFP-72Q huntingtin^{exon1} as EV cargo, EVs were labelled with Cell Mask Deep Red plasma membrane stain for 30 minutes at 37°C. It was found that 95% of GFP-72Q huntingtin^{exon1} particles co-stain with Deep Red stain, confirming that the GFP particles are membrane bound vesicles (Fig. 1D; Fig. 8). By comparison, control protein aggregates of anti-CD38 do not demonstrate Deep Red staining (0.6%), while 80% of EVs containing the GFP-tagged membrane protein PC3 Palm-GFP co-stain with Deep Red. The GFP-containing 180-240 nm membrane bound vesicles were detergent-sensitive. Altogether, these results suggest that the CSPα-EV export pathway involves EVs primarily within the 180-240 nm range and that resveratrol selectively inhibits the CSPα-EV export of GFP-tagged 72Q huntingtin^{exon1} while increasing overall EV export.

### EXAMPLE 3: Treatment of naive cells with EVs comprising CSPα

When media containing total EVs was applied to naive CAD neurons in cell culture, GFP-72Q huntingtin^{exon1} cargo was observed in recipient CAD cells as early as 1 hour after application of the media and remained at 48 hours after application of the media (Fig. 9). When the media contained less EVs with GFP-72Q huntingtin^{exon1} cargo, for example media produced from neurons treated with 50 µM resveratrol, GFP cargo was still detected in recipient cells (Fig. 1E). In addition to the recipient cells, some large EVs carrying GFP-72Q huntingtin^{exon1} cargo were also observed in the cell culture. The size of these large EVs was found to be similar to the size of the recipient CAD cells. This observation makes it difficult to quantify the frequency of delivery of GFP-72Q huntingtin^{exon1} cargo to naive cells in culture. Due to this difficulty in quantifying EV-mediated delivery of cargo to cells in culture, further experiments were carried out *in vivo* using a variety of mouse models to study EV-mediated delivery of cargo to the brain and other organs (see Examples 9-14 and 16-21 provided herein).

### EXAMPLE 4: Export of EVs carrying GFP-72Q huntingtin^{exon1} by CSPα is concentration dependent

EVs were subjected to nanoscale flow cytometry and western blot analysis. This parallel analysis shows that increases in cellular CSPα expression correlates with increases in 180-240 nm EV export of GFP-72Q huntingtin^{exon1} without changes in the mean size of released EVs (Fig. 2). Cell viability was evaluated after media collection, and was not influenced by CSPα expression (Fig. 2D). The observation that transgenic expression of α-synuclein abolishes the lethality and neurodegeneration seen in CSPα knock-out mice raised the possibility that α-synuclein might also stimulate EV-export. That is, more than one mechanism may exist to package and export misfolded proteins from neurons. α-synuclein is the main component of the cytoplasmic inclusions formed in Parkinson's disease patients, and mutations in human α-synuclein or triplication of the α-synuclein gene cause Parkinson's disease. The possibility that α-synuclein facilitated EV-release was tested by comparing EV export of GFP-72Q huntingtin^{exon1} from cells expressing α-synuclein or CSPα. As shown in Fig. 2E, western blot analysis revealed that increases in α-synuclein expression did not result in mutant huntingtin export. These results rule out α-synuclein-driven EV export as the mechanism underlying prevention of neurodegeneration in CSPα knock-out mice and is consistent with the hypothesis that α-synuclein does not replace CSPα, but instead functions downstream of CSPα.

### EXAMPLE 5: Influence of CSPα mutants on EV export

The effect of CSPα mutations on EV genesis and export was evaluated. L115R and L116Δ are human mutations that cause the lysosomal storage disease, ANCL, whereas the CSPα_{HPD-AAA} is a loss-of-function mutant that does not interact with Hsp70 ATPases. The domain structure of CSPα and location of the CSPα_{L115R}, CSPα_{Δ116}, and CSPα_{HPD-AAA} mutations is shown in Fig. 3A. It was previously shown that CSPα_{L115R}, CSPα_{Δ116}, and CSPα_{HPD-AAA}, like wild-type CSPα, are exported from cells, and while CSPα_{L115R} and CSPα_{Δ116} promote export of GFP-72Q huntingtin^{exon1}, CSPα_{HPD-AAA} does not. Nanoparticle tracking analysis (NTA) was used to evaluate the influence of mutant CSPα variants on EV export in the absence of GFP-72Q huntingtin^{exon1}. The NTA profile of CSPα_{HPD-AAA}, CSPα_{L115R} and CSPα_{Δ116} mutations relative to CSPα is shown for comparison (Fig. 3B). No difference in mean size (total) of EVs was observed as a result of CSPα mutations (Fig. 3C). Furthermore, no difference in relative sizes (<180 nm, 180-240 nm, >240 nm) of EVs was observed as a result of CSPα mutations. Next, conditioned media was applied to naive neuronal cells and EV delivery of GFP-72Q huntingtin^{exon1} was monitored. Recipient cells that received control media from vector, CSPα_{HPD-AAA} and α-synuclein transfected cells showed low levels of GFP signal at 24 hours. GFP-72Q huntingtin^{exon1} aggregates were clearly observed in recipient cells receiving CSPα-EVs, CSPα_{L115R}-EVs or CSPα_{Δ116}-EVs as early as 1 hour and remained visible 24 hours after media application (Fig. 10).

### EXAMPLE 6: Resveratrol reduces export of GFP-72 huntingtin^{exon1} cargo by CSPa

To address the question of how resveratrol reduces the export of GFP-72Q huntingtin^{exon1} by CSPα-EVs, neurons were treated with 50µM resveratrol 6 hours following transfection. Fig. 4A shows the NTA profile of EVs collected in the presence (dashed line) and absence (solid line) of 50µM resveratrol. Resveratrol reduced secretion of GFP-72Q huntingtin^{exon1} but not CSPα (Fig. 4B, top panel), consistent with earlier observations. Moreover, resveratrol reduced secretion of GFP-72Q huntingtin^{exon1} mediated by CSPα, CSPα_{L115R} and CSPα_{Δ116}. Cell viability following media removal is shown in Fig. 4B, bottom panel. Nanoscale flow cytometry analysis verified that resveratrol increased total EVs exported by CAD cells in all conditions, while decreasing GFP-72Q huntingtin^{exon1}-containing EVs. Resveratrol also significantly, increased the mean size of vesicles in all conditions (Fig. 4C). While not being bound by any theory, one possibility is that resveratrol may directly influence cargo-loading machinery of the CSPα EVs. It is also conceivable that resveratrol may indirectly influence cargo-loading by modulating other proteostatic mechanisms that target GFP-72Q huntingtin^{exon1} (e.g., proteasome, lysosome clearance). Taken together, these results indicate that resveratrol reduces cargo-loading of GFP-tagged 72Q huntingtin^{exon1} into EVs without reducing export of CSPα or the CSPα_{L115R} and CSPα_{Δ116}.

### EXAMPLE 7: DnaJB2 and DnaJB6 promote EV export of GFP-72Q huntingtin^{exon1}

CSPα (DnaJC5) is a type III J protein with a well-defined domain architecture that includes an N terminal J domain and a cysteine string region after which the protein is named. J proteins are a family of chaperones that contain a wide variety of domains but the defining signature domain is the approximately 70 amino acid J domain that contains a histidine-proline-aspartic acid (HPD) motif required for activation of Hsp70.

To test whether other J proteins may also export misfolded proteins, several J proteins with reported links to neurodegeneration were screened for EV-mediated export activity. DnaJB2 was identified by both western blot analysis (Fig. 5A, left panel) and nanoscale flow cytometry (Fig. 5A, right panel) as a J protein that exports misfolded huntingtin in EVs, similar to CSPα (Figs. 5A and 5B). Alternative splicing of the DnaJB2 gene produces two isoforms, a DnaJB2ₛₕₒᵣₜ and a 42 kDa DnaJB2_{long} isoform that undergoes C terminal geranylgeranylation. DnaJB2_{long} was studied further. The related J proteins, DnaJA1, DnaJB1, DnaJB11, DnaJC14, DnaJC13 and DnaJC19 did not export misfolded huntingtin. Extracellular milieu includes free floating co-chaperones in addition to EVs, and chaperones that adhere to EVs were expected to co-isolate with EVs. DnaJA1, DnaJB1, DnaJB2, DnaJB6, DnaJB11 and DnaJC14 were found to be secreted from CAD cells (Fig. 5C). In fact, DnaJB11 as well as DnaJA1 were robustly secreted by CAD neurons under all conditions evaluated (Fig. 5C).

### EXAMPLE 8: EV export of GFP-72Q huntingtin^{exon1} by DnaJB2_{long} and DnaJB6_{long}

A confounding observation from the parallel analysis was that DnaJB6ₛₕₒᵣₜ was observed to export GFP-72Q huntingtin^{exon1} by nanoscale flow cytometry but not by western blot analysis (Fig. 5A). This suggested that cargo in DnaJB6 EVs may undergo degradation during EV isolation. To investigate this possibility further, GFP-72Q huntingtin^{exon1} export was analyzed by spotting media on nitrocellulose prior to EV purification. Dot blot analysis of unfractionated media and western blot analysis of EVs (Fig. 6A) revealed that, at high expression levels, DnaJB6ₛₕₒᵣₜ does indeed mediate export of GFP-72Q huntingtin^{exon1}. Alternative splicing of the DnaJB6 gene produces two isoforms, a DnaJB6ₛₕₒᵣₜ and a 36 kDa DnaJB6_{long} isoform that contains a C terminal nuclear localization signal. DnaJB6ₛₕₒᵣₜ and DnaJB6_{long} were compared for GFP-72Q huntingtin^{exon1} export activity. Unexpectedly, EV export of GFP-72Q huntingtin^{exon1} by DnaJB6_{long} was more robust than by DnaJB6ₛₕₒᵣₜ using western blot analysis. Domain structures of CSPα, DnaJB2_{long} and DnaJB6_{long} are illustrated in Fig. 6B. Each of these co-chaperones contains unique domains in addition to the common J domain. Fig. 6C shows the percentage of GFP-positive EVs within the total EV pool determined by nanoscale flow cytometry (Fig. 6C, left panel) and quantification of GFP-72Q huntingtin^{exon1} by western blot analysis (Fig. 6C, right panel). The percentage of EVs containing GFP-72Q huntingtin^{exon1} is dramatically increased when cells express CSPα (p < 0.0001), DnaJB2_{long} (p < 0.0001) or DnaJB6_{long} (p < 0.0001). EVs containing GFP-72Q huntingtin^{exon1} are primarily within the 180-240 nm range from DnaJB2_{long}- and DnaJB6_{long}-expressing cells, similar to that found for CSPα-expressing cells. It was found that exported DnaJB2_{long}- and DnaJB6_{long}-EVs containing GFP-72Q huntingtin^{exon1} deliver cargo to recipient cells (Fig. 11). In contrast to CSPα and CSPα mutants, media containing DnaJB2_{long} and DnaJB6_{long} EVs did not deliver GFP-huntingtin^{exon1} to recipient cells at 1 hour, but were found to deliver GFP-huntingtin^{exon1} cargo 24 hours following media application. Next, the RNA cargo of total EVs was labelled with Exo-Red (SBI), and the labelled EVs were applied to target cells in order to compare GFP-72Q huntingtin^{exon1} with Exo-Red transfer (Fig. 11). Nearly all recipient cells were Exo-Red-positive, demonstrating the effective delivery of EVs. These results indicate that, while EVs comprising DnaJB2_{long} or DnaJB6_{long} transfer GFP-72Q huntingtin^{exon1} between cells, there are differences in recipient cell uptake or proteostasis between the EVs comprising DnaJB2_{long}, DnaJB6_{long}, and CSPα.

Collectively, the results show that three DNAJ domain-containing protein chaperones that are key to neuronal protein quality control, CSPα, DnaJB2_{long} and DnaJB6_{long}, shuttle misfolded GFP-72Q huntingtin^{exon1} from parent to recipient cells in EVs and that the molecule resveratrol modulates this pathway. These neurotoxin removal-based mechanisms represent less than 1% of the total EVs produced, and yet, these mechanisms efficiently transfer cargo to naive neuronal cells.

### EXAMPLE 9: EVs comprising CSPα deliver cargo to the brain following intraperitoneal administration

EVs were produced from cells expressing CSPα and GFP-tagged 72Q huntingtin^{exon1}, or from cells expressing a vector control and GFP-tagged 72Q huntingtin^{exon1}. The EVs produced in the presence of CSPα were heterogeneous, and included a sub-population of EVs containing GFP-tagged 72Q huntingtin^{exon1} cargo. The EVs were administered to 10-12-month old 5xFAD mice or WT littermate controls by intraperitoneal injection. Each mouse received 1 ml of media containing the EVs produced from cells expressing CSPα and GFP-tagged 72Q huntingtin^{exon1} or from cells expressing a vector control and GFP-tagged 72Q huntingtin^{exon1} (Fig. 12A).

Three hours after intraperitoneal administration, the mice were euthanized and the brain of each mouse was analyzed by fluorescence imaging *ex vivo.* The imaging showed that EVs comprising CSPα cross the blood-brain barrier and rapidly deliver cargo to the brain following intraperitoneal administration (Fig. 12B).

### EXAMPLE 10: EVs comprising CSPα selectively deliver cargo to the brain, but not to the heart, lung, liver, spleen or kidney

EVs were produced from cells expressing CSPα and GFP-tagged 72Q huntingtin^{exon1}. The EVs produced in the presence of CSPα were heterogeneous, and included a sub-population of EVs containing GFP-tagged 72Q huntingtin^{exon1} cargo. The EVs were administered to 10-12-month old 5xFAD mice or WT littermate controls by intraperitoneal injection. Each mouse received 1 ml of media containing the EVs produced from cells expressing CSPα and GFP-tagged 72Q huntingtin^{exon1} or from cells expressing a vector control and GFP-tagged 72Q huntingtin^{exon1} (Fig. 13A).

Three hours after intraperitoneal administration, the mice were euthanized and the brain, heart, lung, liver, spleen and kidney of each mouse was analyzed by fluorescence imaging *ex vivo.* The imaging showed that EVs comprising CSPα cross the blood-brain barrier and selectively deliver cargo to the brain, but not to the heart, lung, liver, spleen or kidney and rapidly deliver cargo to the brain (Fig. 13B).

### EXAMPLE 11: EVs comprising CSPα deliver cargo to the brain following intravenous administration

EVs were produced from cells expressing CSPα and GFP-tagged 72Q huntingtin^{exon1}, or from cells expressing a vector control and GFP-tagged 72Q huntingtin^{exon1}. The EVs produced in the presence of CSPα were heterogeneous, and included a sub-population of EVs containing GFP-tagged 72Q huntingtin^{exon1} cargo. The EVs were administered to 10-12-month old 5xFAD mice or WT littermate controls by intravenous injection. Each mouse received 200 µl of media containing the EVs produced from cells expressing CSPα and GFP-tagged 72Q huntingtin^{exon1} or from cells expressing a vector control and GFP-tagged 72Q huntingtin^{exon1} (Fig. 14A).

One hour after intravenous administration, the mice were euthanized and the brain of each mouse was analyzed by fluorescence imaging *ex vivo.* The imaging showed that EVs comprising CSPα cross the blood-brain barrier and rapidly deliver cargo to the brain following intravenous administration (Fig. 14B).

### EXAMPLE 12: EVs comprising CSPα deliver cargo to the brain, whereas the majority of EVs without CSPα deliver cargo to the liver, lung and gastrointestinal (GI) tract

EVs were produced from cells expressing CSPα and GFP-tagged 72Q huntingtin^{exon1}, or from cells expressing a vector control and GFP-tagged 72Q huntingtin^{exon1}. The total EV pool, including EVs carrying GFP-tagged 72Q huntingtin^{exon1} cargo, were labelled with Deep Red membrane stain. The EVs were administered to 10-12-month old 5xFAD mice or WT littermate controls by intraperitoneal injection. Each mouse received 1 ml of media containing the EVs produced from cells expressing CSPα and GFP-tagged 72Q huntingtin^{exon1} or from cells expressing a vector control and GFP-tagged 72Q huntingtin^{exon1} (Fig. 15A).

Localization of EVs was assessed by whole body X-ray and fluorescence imaging of mice *in vivo,* using the multi-model mouse rotation system. The imaging was performed at two time points: immediately post-injection (Fig. 15B, top panels), and 3 hours post-injection (Fig. 15B, bottom panels). The imaging showed that Deep Red membrane-stained EVs can be readily visualized *in vivo* (Fig. 15B, left panels), whereas GFP fluorescence did not penetrate the body cavity (Fig. 15B, right panels).

Three hours after intraperitoneal administration, the mice were euthanized and the brain, heart, lung, liver, spleen and kidney of each mouse was analyzed by fluorescence imaging *ex vivo.* Quantification of Deep Red membrane stained EVs showed that total EVs were delivered in high levels to the liver and the lung (Figs. 15C and 20B); whereas quantification of GFP fluorescence intensity showed that the sub-population of EVs carrying GFP-tagged 72Q huntingtin^{exon1} cargo were selectively delivered to the brain in both 5xFAD mice and their WT littermates (Figs. 15C and 20A). In parallel, quantification of Deep Red fluorescence intensity in the GI tract of 5xFAD and WT mice showed that a substantial proportion of total EVs are delivered to the GI tract (Fig. 21).

Together, these results show that while the majority of EVs are delivered to the lung, liver and GI tract, CSPα-EVs are preferentially delivered to the brain.

### EXAMPLE 13: EVs comprising CSPα deliver cargo to the brain following intraperitoneal or intraveneous administration

EVs were produced from cells expressing CSPα and GFP-tagged 72Q huntingtin^{exon1}, or from cells expressing a vector control and GFP-tagged 72Q huntingtin^{exon1}. The total EV pool, including EVs carrying GFP-tagged 72Q huntingtin^{exon1} cargo, were labelled with Deep Red membrane stain. The EVs were administered to 10-12-month old 5xFAD mice or WT littermate controls by intravenous (200 µl of media) or intraperitoneal (1 ml of media) injection (Fig. 16A).

Three hours after intravenous or intraperitoneal administration, the mice were euthanized and the brain of each mouse was analyzed by fluorescence imaging *ex vivo.* The imaging showed that Deep Red membrane stained EVs comprising CSPα cross the blood-brain barrier and rapidly deliver GFP-tagged 72Q huntingtin^{exon1} cargo to the brain following either of intravenous and intraperitoneal administration (Fig. 16B). Quantification of the GFP fluorescence signal intensity revealed an increased level of GFP-tagged 72Q huntingtin^{exon1} in the brains of 5xFAD mice compared to their WT littermates (Fig. 19).

### EXAMPLE 14: EVs comprising CSPα deliver cargo to the brain in juvenile wild-type (WT) and CSPα knock-out (CSPα KO) mice

EVs were produced from cells expressing CSPα and GFP-tagged 72Q huntingtin^{exon1}. The EVs produced in the presence of CSPα were heterogeneous, and included a sub-population of EVs containing GFP-tagged 72Q huntingtin^{exon1} cargo. The total EV pool, including EVs carrying GFP-tagged 72Q huntingtin^{exon1} cargo, were labelled with Deep Red membrane stain. The EVs were administered to 22-27-day old CSPα KO mice or WT littermate controls by intraperitoneal injection. Each mouse received 0.2 ml of media containing the Deep Red membrane stain EVs produced from cells expressing CSPα and GFP-tagged 72Q huntingtin^{exon1} (Fig. 17A).

Three hours after intraperitoneal administration, the mice were euthanized and the brain of each mouse was analyzed by fluorescence imaging *ex vivo.* The imaging showed that Deep Red membrane stained EVs comprising CSPα cross the blood-brain barrier and rapidly deliver GFP-tagged 72Q huntingtin^{exon1} cargo to the brain following intraperitoneal administration to juvenile WT and CSPα KO mice (Fig. 17B).

### EXAMPLE 15: Export of GFP-72Q huntingtin^{exon1} by exophers and/or large extracellular vesicles

The majority of GFP-72Q huntingtin^{exon1}-containing EVs exported from CAD cells expressing CSPα are about 180-240 nm in size (see Examples 2 and 4). Such EVs can be classified as microvesicles. In addition, microscopic analysis revealed that some EVs containing GFP-72Q huntingtin^{exon1} may be significantly larger than 1 µm, and can be classified as exophers and/or large extracellular vesicles (Fig. 18). The exophers and/or large extracellular vesicles comprising CSPα and GFP-72Q huntingtin^{exon1} were further characterized and found to be in the range of about 18-25 µm in size (Fig. 18D).

### EXAMPLE 16: EVs comprising full length CSPα or a truncated form of CSPα deliver cargo to the brain in mouse models of ALS

EVs were produced from cells expressing GFP-tagged 72Q huntingtin^{exon1} and the full length (198 amino acid) CSPα protein or a truncated form comprising amino acids 1-112 of CSPα (CSPα₁₋₁₁₂), which retains the DnaJ domain but lacks the cysteine string region of the full length protein. 1 ml of media containing the CSPα-EVs or CSPα₁₋₁₁₂-EVs was administered to approximately 6-month old mouse models of ALS (SOD-1^{G93A}) or age-matched WT mice by intraperitoneal injection.

Three hours after administration, the mice were euthanized and the brain of each mouse was analyzed by fluorescence imaging *ex vivo.* The imaging showed that EVs comprising either the full length or the truncated form of CSPα cross the blood-brain barrier and rapidly deliver GFP-tagged 72Q huntingtin^{exon1} cargo to the brain of ALS mice, at levels comparable to WT mice (Fig. 22).

To assess whether administration of a lower amount of EVs is sufficient for delivering cargo to the brain, ALS or WT mice were intraperitoneally injected with 0.2 ml of PBS (control), or 0.2 ml of media containing CSPα-EVs or CSPα₁₋₁₁₂-EVs. Quantification of GFP fluorescence intensity revealed that CSPα-EVs or CSPα₁₋₁₁₂-EVs efficiently deliver GFP-tagged 72Q huntingtin^{exon1} cargo to the brain, even when the amount of the administered EVs is reduced 5-fold (Fig. 23).

### EXAMPLE 17: EVs comprising full length CSPα or a truncated form of CSPα deliver cargo to the brain, whereas the majority of EVs without CSPα deliver cargo to the liver, lung and GI tract in mouse models of ALS

EVs were produced from cells expressing GFP-tagged 72Q huntingtin^{exon1} and CSPα, CSPα₁₋₁₁₂, or a control vector. The total EV pool, including EVs carrying GFP-tagged 72Q huntingtin^{exon1} cargo, were labelled with Deep Red membrane stain. The EVs were administered to approximately 6-month old ALS mice or WT age-matched controls by intraperitoneal injection. Each mouse received 0.2 ml or 1 ml of media containing the EVs.

Three hours after administration, the mice were euthanized and the brain, heart, lung, liver, spleen, kidney and GI tract of each mouse was analyzed by fluorescence imaging *ex vivo.* Quantification of GFP fluorescence intensity showed that CSPα-EVs and CSPα₁₋₁₁₂-EVs carrying GFP-tagged 72Q huntingtin^{exon1} cargo were selectively delivered to the brain (Fig. 24); whereas quantification of Deep Red fluorescence intensity showed that total EVs were delivered in high levels to the liver, lung and GI tract (Fig. 25) in both ALS and WT mice.

Together, these results show that while the majority of EVs are delivered to the lung, liver and GI tract, EVs comprising the full length or truncated form of CSPα are preferentially delivered to the brain, irrespective of whether a low amount (0.2 ml) or a high amount (1 ml) of EVs is administered.

### EXAMPLE 18: EVs comprising full length CSPα or a truncated form of CSPα deliver cargo selectively to the brain in juvenile WT and CSPα KO mice

EVs were produced from cells expressing GFP-tagged 72Q huntingtin^{exon1} and CSPα or CSPα₁₋₁₁₂. The total EV pool, including EVs carrying GFP-tagged 72Q huntingtin^{exon1} cargo, were labelled with Deep Red membrane stain. 0.2 ml of media containing the EVs was administered to 21-28-day old CSPα KO early neurodegeneration mouse models or WT littermate controls by intraperitoneal injection.

Three hours following administration, the mice were euthanized and the brain of each mouse was analyzed by fluorescence imaging *ex vivo.* Quantification of GFP and Deep Red fluorescence intensities revealed that both the CSPα-EVs and CSPα₁₋₁₁₂-EVs deliver GFP-tagged 72Q huntingtin^{exon1} cargo to the brain in juvenile WT and CSPα KO mice (Figs. 26A and 26B).

In parallel with the imaging of the brain, the heart, lung, liver, spleen, kidney and GI tract of each mouse were also analyzed by fluorescence imaging *ex vivo.* Quantification of GFP and Deep Red fluorescence intensities revealed that while the majority of EVs are delivered to the lung, liver, kidney and GI tract, EVs comprising the full length or truncated form of CSPα cross the blood-brain barrier and preferentially deliver their cargo to the brain (Figs. 27A and 27B).

### EXAMPLE 19: Delivery of cargo to the brain by CSPα-EVs and CSPα₁₋₁₁₂-EVs is not affected by washing out the blood

To examine the effects of blood washout on CSPα-mediated brain delivery, 0.2 ml of media containing CSPα-EVs or CSPα₁₋₁₁₂-EVs comprising GFP-tagged 72Q huntingtin^{exon1} was administered to WT mice by intraperitoneal injection.

Three hours following administration, a subset of the mice were subjected to intracardiac perfusion with 10 ml of PBS for 5 minutes. Subsequently, the mice were euthanized and the brain of each mouse was analyzed by fluorescence imaging *ex vivo.* Quantification of GFP fluorescence intensity revealed that washing out the blood by intracardiac perfusion does not reduce the level of GFP-tagged 72Q huntingtin^{exon1} delivered to the brain (Fig. 28).

### EXAMPLE 20: Cargo delivered to the brain by CSPα-EVs and CSPα₁₋₁₁₂-EVs is detectable but reduced 24 hours after administration

To evaluate the retention of GFP-tagged 72Q huntingtin^{exon1} in the brain following CSPα-mediated delivery, 0.2 ml of media containing CSPα-EVs or CSPα₁₋₁₁₂-EVs comprising GFP-tagged 72Q huntingtin^{exon1} was administered to WT mice by intraperitoneal injection. he mice were euthanized 3 hours or 24 hours after administration of the EVs, and the brain of each mouse was analyzed by fluorescence imaging *ex vivo.*

Quantification of GFP and Deep Red fluorescence intensities showed that although the level of GFP-tagged 72Q huntingtin^{exon1} in the brain was reduced at 24 hours compared to 3 hours after administration, some of the GFP-tagged 72Q huntingtin^{exon1} remains detectable in the brain after 24 hours (Fig. 29A-29C). These results indicate that CSPα-EV-mediated delivery of cargo to the brain is transient and scalable, which facilitates the development of various dosage forms for delivery of agents to the brain.

### EXAMPLE 21: CSPα-EVs and CSPα₁₋₁₁₂-EVs deliver calcium/calmodulin-dependent protein kinase II alpha (CAMK) cargo to the brain

To rule out the possibility that the fluorescence intensity measurements disclosed herein are biased as a result of the propensity of 72Q huntingtin^{exon1} protein to form aggregates, the delivery of a different protein to the brain was also measured. Unlike 72Q huntingtin^{exon1}, CAMK does not tend to form aggregates. EVs were produced from cells expressing GFP-tagged CAMK and CSPα or CSPα₁₋₁₁₂. 0.2 ml of PBS (control), or media containing CSPα-EVs or CSPα₁₋₁₁₂-EVs comprising GFP-tagged CAMK was administered to 21-28-day old WT or CSPα KO mice by intraperitoneal injection.

Three hours after administration, the mice were euthanized and the brain of each mouse was analyzed by fluorescence imaging *ex vivo* (Fig. 30A). Quantification of GFP fluorescence intensity revealed that both the CSPα-EVs and CSPα₁₋₁₁₂-EVs deliver GFP-tagged CAMK cargo to the brain (Fig. 30B). Additionally, quantification of Deep Red fluorescence intensity showed that the (non-targeted) delivery of total EVs to the GI tract is not affected by the type of cargo carried by the CSPα-EVs and CSPα₁₋₁₁₂-EVs (Fig 31).

Together, these results indicate that CSPα-EVs are not limited to delivering 72Q huntingtin^{exon1} protein to the brain. Rather, CSPα-EVs are capable of delivering various types of cargo across the blood-brain barrier.

### EXAMPLE 22: Effect of β amyloid peptide on the secretion of 72Q huntingtin^{exon1} by EVs comprising various DnaJ domain-containing protein chaperones

CAD cells were transiently transfected with GFP-tagged 72Q huntingtin^{exon1} and a vector control, CSPα, DnaJB2_{long}, or DnaJB6_{long}. 6 hours after transfection, the cells were treated with 12.5 µg/ml β amyloid peptide (Aβ) or left untreated. 48 hours after transfection, the media was collected, EVs were prepared, and EV secretion of GFP-tagged 72Q huntingtin^{exon1} was measured.

Quantification of GFP-tagged 72Q huntingtin^{exon1} secretion revealed that CSPα-EVs, DnaJB2_{long}-EVs and DnaJB6_{long}-EVs are all competent in exporting GFP-tagged 72Q huntingtin^{exon1} from CAD cells (Fig. 32). Additionally, incubation of the cells in the presence of β amyloid peptide increased the secretion of GFP-tagged 72Q huntingtin^{exon1} by all three types of DnaJ domain-containing protein chaperones.

### EXAMPLE 23: CSPα-EVs mediate the delivery of agents across the blood-brain barrier under a variety of different conditions

Taken together, the examples provided herein demonstrate a broad range of utility and industrial applicability for the presently disclosed method of delivering an agent across the blood-brain barrier; the presently disclosed method of delivering an agent to the CNS; the presently disclosed method of treating a CNS disease or disorder; the presently disclosed agent delivery vehicle; and the presently disclosed extracellular vesicle.

As shown in Table 1, the examples provided herein demonstrate that:
a) CSPα-EVs deliver proteins across the blood-brain barrier in WT mice and in 3 mouse models of different CNS disorders (5xFAD, SOD-1^{G93A} and CSPα KO);
b) CSPα-EVs deliver proteins across the blood-brain barrier in mice of 3 different ages;
c) CSPα-EVs administered either intraperitoneally or intravenously deliver proteins across the blood-brain barrier;
d) CSPα-EVs administered intraperitoneally in low amounts (0.2 ml) deliver proteins across the blood-brain barrier; and
e) CSPα-EVs deliver two representative proteins (72Q huntingtin^{exon1} and CAMK) across the blood-brain barrier.

**Table 1: CSPα-EVs efficiently deliver diverse cargo to the brain under disparate conditions**

| **Mouse model** | **Age** | **Administration route of CSPα-EVs (volume)** | **Delivery across the blood-brain barrier** | |
|---|---|---|---|---|
| | | | **72Q huntingtin^{exon1}** | **CAMK** |
| 5xFAD Alzheimer's disease model | ~11 months old | Intraperitoneal (1 ml) | Yes | Not tested |
| | | Intravenous (0.2 ml) | | |
| SOD-1^{G93A} amyotrophic lateral sclerosis (ALS) model | ~6 months old | Intraperitoneal (1 ml) | Yes | Not tested |
| | | Intraperitoneal (0.2 ml) | | |
| CSPα KO early neurodegeneration (eND) model | ~21-28 days old | Intraperitoneal (0.2 ml) | Yes | Yes |
| WT | ~21-28 days old | Intraperitoneal (0.2 ml) | Yes | Not tested |
| | | +/- intracardiac perfusion with 10 ml PBS before imaging | | |

As shown in Table 2, the examples provided herein demonstrate that:
a) full length CSPα-EVs and the truncated form CSPα₁₋₁₁₂-EVs deliver proteins across the blood-brain barrier; and
b) CSPα-EVs and CSPα₁₋₁₁₂-EVs deliver two types of proteins (72Q huntingtin^{exon1} and CAMK) across the blood-brain barrier.

**Table 2: Full length CSPα-EVs and truncated form CSPα₁₋₁₁₂-EVs efficiently deliver two types of proteins to the brain of two disparate mouse models**

| **Mouse model** | **Administration route of CSPα-EVs (volume)** | **Delivery across the blood-brain barrier** | | | |
|---|---|---|---|---|---|
| | | **72Q huntingtin^{exon1}** | | **CAMK** | |
| | | **CSPα** | **CSPα₁₋₁₁₂** | **CSPα** | **CSPα₁₋₁₁₂** |
| SOD-1^{G93A} (ALS model) | Intraperitoneal (1 ml) | Yes | Yes | Yes | Yes |
| | Intraperitoneal (0.2 ml) | | | | |
| CSPα KO (eND model) | Intraperitoneal (0.2 ml) | Yes | Yes | Yes | Yes |

### DOCUMENTS CITED

Alvarez-Erviti, L. et al. Lysosomal dysfunction increases exosome-mediated alpha-synuclein release and transmission. Neurobiol Dis 42, 360-367, doi: 10.1016/j.nbd.201 1.01.029 (201 1).
Aprile, F. A., Kallstig, E., Limorenko, G., Vendruscolo, M., Ron, D., and Hansen, C. (2017) Sci. Rep. 7, 9039
Ast, A. et al. mHTT Seeding Activity: A Marker of Disease Progression and Neurotoxicity in Models of Huntington's Disease. Mol Cell 71 , 675-688 e676, doi:10.1016/j.molcel.2018.07.032 (2018).
Benitez, B. A., Alvarado, D., Cai, Y., Mayo, K., Chakraverty, S., Norton, J., Morris, J. C., Sands, M. S., Goate, A., and Cruchaga, C. (201 1) PLoS. ONE. 6, e26741
Blumen, S. C., Astord, S., Robin, V., Vignaud, L., Toumi, N., Cieslik, A., Achiron, A., Carasso, R. L., Gurevich, M., Braverman, I., Blumen, N., Munich, A., Barkats, M., and Viollet, L. (2012) Ann. Neurol 71 , 509-519
Braun, J. E. and Scheller, R. H. (1995) Neuropharmacology 34, 1361 -1369
Braun, J. E., Wilbanks, S. M., and Scheller, R. H. (1996) J Biol Chem 271 , 25989-25993
Budnik, V., Ruiz-Canada, C. & Wendler, F. Extracellular vesicles round off communication in the nervous system. Nat Rev Neurosci 17, 160-172, doi:10.1038/nrn.2015.29 (2016).
Chandra, S., Gallardo, G., Fernandez-Chacon, R., Schluter, O. M., and Sudhof, T. C. (2005) Cell 123, 383-396
Chappie, J. P. and Cheetham, M. E. (2003) J Biol Chem 278, 19087-19094
Chapple, J. P., van der, S. J., Poopalasundaram, S., and Cheetham, M. E. (2004) Biochem. Soc. Trans. 32, 640-642
Cheetham, M. E., Brion, J. P., and Anderton, B. H. (1992) Biochem. J 284 (Pt 2), 469-476
Chen, H. J., Mitchell, J. C., Novoselov, S., Miller, J., Nishimura, A. L., Scotter, E. L., Vance, C. A., Cheetham, M. E., and Shaw, C. E. (2016) Brain 139, 1417-1432
Chiti, F. & Dobson, C. M. Protein Misfolding, Amyloid Formation, and Human Disease: A Summary of Progress Over the Last Decade. Annu Rev Biochem 86, 27-68, doi:10.1146/annurev-biochem-061516-045115 (2017).
Chuang, J. Z., Zhou, H., Zhu, M., Li, S. H., Li, X. J., and Sung, C. H. (2002) J Biol Chem 277, 19831-19838
Cicchetti, F. et al. Mutant huntingtin is present in neuronal grafts in Huntington disease patients. Ann Neurol 76, 31-42, doi:10.1002/ana.24174 (2014).
Conlan, R. S., Pisano, S., Oliveira, M. I., Ferrari, M. & Mendes Pinto, I. Exosomes as Reconfigurable Therapeutic Systems. Trends Mol Med 23, 636-650, doi:10.1016/j.molmed.2017.05.003 (2017).
Costanzo, M., Abounit, S., Marzo, L., Danckaert, A., Chamoun, Z., Roux, P., and Zurzolo, C. (2013) J. Cell Sci. 126, 3678-3685
Deng, J. et al. Neurons Export Extracellular Vesicles Enriched in Cysteine String Protein and Misfolded Protein Cargo. Sci Rep 7, 956, doi:10.1038/s41598-017-01115-6 (2017).
Di Pardo, A. et al. Ganglioside GM1 induces phosphorylation of mutant huntingtin and restores normal motor behavior in Huntington disease mice. Proc Natl Acad Sci U S A 109, 3528-3533, doi:10.1073/pnas.1114502109 (2012).
Donnelier, J., Braun, S. T., Dolzhanskaya, N., Ahrendt, E., Braun, A. P., Velinov, M., and Braun, J. E. (2015) PLoS. ONE. 10, e0125205
Durrenberger, P. F., Filiou, M. D., Moran, L. B., Michael, G. J., Novoselov, S., Cheetham, M. E., Clark, P., Pearce, R. K., and Graeber, M. B. (2009) J. Neurosci. Res. 87, 238-245
Emmanouilidou, E., Stefanis, L. & Vekrellis, K. Cell-produced alpha-synuclein oligomers are targeted to, and impair, the 26S proteasome. Neurobiol Aging 31, 953-968, doi:10.1016/j.neurobiolaging.2008.07.008 (2010).
Fayazi, Z., Ghosh, S., Marion, S., Bao, X., Shero, M., and Kazemi-Esfarjani, P. (2006) Neurobiol Dis. 24, 226-244
Fernandez-Chacon, R., Wolfel, M., Nishimune, H., Tabares, L., Schmitz, F., Castellano-Munoz, M., Rosenmund, C., Montesinos, M. L., Sanes, J. R., Schneggenburger, R., and Sudhof, T. C. (2004) Neuron 42, 237-251
Fevrier, B. et al. Cells release prions in association with exosomes. Proc Natl Acad Sci U S A 101, 9683-9688, doi:10.1073/pnas.0308413101 (2004).
Fontaine, S. N. et al. DnaJ/Hsc70 chaperone complexes control the extracellular release of neurodegenerative-associated proteins. EMBO J 35, 1537-1549, doi:10.15252/embj.201593489 (2016).
Gao, X. C., Zhou, C. J., Zhou, Z. R., Zhang, Y. H., Zheng, X. M., Song, A. X., and Hu, H. Y. (2011) PLoS. ONE. 6, e19763
Genereux, J. C., Qu, S., Zhou, M., Ryno, L. M., Wang, S., Shoulders, M. D., Kaufman, R. J., Lasmezas, C. I., Kelly, J. W., and Wiseman, R. L. (2015) EMBO J. 34, 4-19
Gess, B., Auer-Grumbach, M., Schirmacher, A., Strom, T., Zitzelsberger, M., Rudnik-Schoneborn, S., Rohr, D., Halfter, H., Young, P., and Senderek, J. (2014) Neurology 83, 1726-1732
Gillis, J., Schipper-Krom, S., Juenemann, K., Gruber, A., Coolen, S., van den Nieuwendijk, R., van, V. H., Overkleeft, H., Goedhart, J., Kampinga, H. H., and Reits, E. A. (2013) J. Biol. Chem. 288, 17225-17237
Gomes, C., Keller, S., Altevogt, P. & Costa, J. Evidence for secretion of Cu,Zn superoxide dismutase via exosomes from a cell model of amyotrophic lateral sclerosis. Neurosci Lett 428, 43-46, doi:10.1016/j.neulet.2007.09.024 (2007).
Gorenberg, E. L. & Chandra, S. S. The Role of Co-chaperones in Synaptic Proteostasis and Neurodegenerative Disease. Front Neurosci 11, 248, doi:10.3389/fnins.2017.00248 (2017).
Grad, L. I. et al. Intercellular propagated misfolding of wild-type Cu/Zn superoxide dismutase occurs via exosome-dependent and -independent mechanisms. Proc Natl Acad Sci U S A 111, 3620-3625, doi:10.1073/pnas.1312245111 (2014).
Grad, L. I., Pokrishevsky, E., Silverman, J. M. & Cashman, N. R. Exosome-dependent and independent mechanisms are involved in prion-like transmission of propagated Cu/Zn superoxide dismutase misfolding. Prion 8, 331-335, doi:10.4161/19336896.2014.983398 (2014).
Gusella, J. F. & MacDonald, M. E. Molecular genetics: unmasking polyglutamine triggers in neurodegenerative disease. Nat Rev Neurosci 1, 109-115, doi:10.1038/nrd1077 (2000).
Hageman, J., Rujano, M. A., van Waarde, M. A., Kakkar, V., Dirks, R. P., Govorukhina, N., Oosterveld-Hut, H. M., Lubsen, N. H., and Kampinga, H. H. (2010) Mol. Cell 37, 355-369
Hanson, P. I. & Cashikar, A. Multivesicular body morphogenesis. Annu Rev Cell Dev Biol 28, 337-362, doi:10.1146/annurev-cellbio-092910-154152 (2012).
Harms, M. B., Sommerville, R. B., Allred, P., Bell, S., Ma, D., Cooper, P., Lopate, G., Pestronk, A., Weihl, C. C., and Baloh, R. H. (2012) Ann. Neurol. 71, 407-416
Henderson, M. X., Wirak, G. S., Zhang, Y. Q., Dai, F., Ginsberg, S. D., Dolzhanskaya, N., Staropoli, J. F., Nijssen, P. C., Lam, T. T., Roth, A. F., Davis, N. G., Dawson, G., Velinov, M., and Chandra, S. S. (2016) Acta Neuropathol. 131, 621-637
Howarth, J. L., Kelly, S., Keasey, M. P., Glover, C. P., Lee, Y. B., Mitrophanous, K., Chapple, J. P., Gallo, J. M., Cheetham, M. E., and Uney, J. B. (2007) Mol. Ther. 15, 1100-1105
Howitt, J. and Hill, A. F. (2016) J. Biol. Chem. 291, 26589-26597
Jucker, M. & Walker, L. C. Propagation and spread of pathogenic protein assemblies in neurodegenerative diseases. Nat Neurosci 21, 1341-1349, doi:10.1038/s41593-018-0238-6 (2018).
Kakkar, V., Mansson, C., de Mattos, E. P., Bergink, S., van der Zwaag, M., van Waarde, M. A., Kloosterhuis, N. J., Melki, R., van Cruchten, R. T., Al-Karadaghi, S., Arosio, P., Dobson, C. M., Knowles, T. P., Bates, G. P., van Deursen, J. M., Linse, S., van de Sluis, B., Emanuelsson, C., and Kampinga, H. H. (2016) Mol. Cell
Kakkar, V., Prins, L. C., and Kampinga, H. H. (2012) Curr. Top. Med. Chem. 12, 2479-2490
Kalra, H., Drummen, G. P., and Mathivanan, S. (2016) Int. J. Mol. Sci. 17, 170
Kampinga, H. H. and Craig, E. A. (2010) Nat Rev Mol. Cell Biol 11, 579-592
Kashyap, S. S., Johnson, J. R., McCue, H. V., Chen, X., Edmonds, M. J., Ayala, M., Graham, M. E., Jenn, R. C., Barclay, J. W., Burgoyne, R. D., and Morgan, A. (2014) Hum. Mol. Genet. 23, 5916-5927
Koutras, C. and Braun, J. E. (2014) Front Cell Neurosci. 8, 191
Kyle, B. D., Ahrendt, E., Braun, A. P., and Braun, J. E. (2013) Sci. Rep. 3, 2447
Labbadia, J. & Morimoto, R. I. The biology of proteostasis in aging and disease. Annu Rev Biochem 84, 435-464, doi: 10. 1146/annurev-biochem-060614-033955 (2015).
Labbadia, J., Novoselov, S. S., Bett, J. S., Weiss, A., Paganetti, P., Bates, G. P., and Cheetham, M. E. (2012) Brain 135, 1180-1196
Lee, J. et al. Secretion of misfolded cytosolic proteins from mammalian cells is independent of chaperone-mediated autophagy. J Biol Chem 293, 14359-14370, doi:10.1074/jbc.RA118.003660 (2018).
Maas, S. L. N., Breakefield, X. O. & Weaver, A. M. Extracellular Vesicles: Unique Intercellular Delivery Vehicles. Trends Cell Biol 27, 172-188, doi: 1 0.1 016/j.tcb.2016.11.003 (2017).
Mansson, C., Arosio, P., Hussein, R., Kampinga, H. H., Hashem, R. M., Boelens, W. C., Dobson, C. M., Knowles, T. P., Linse, S., and Emanuelsson, C. (2014) J. Biol. Chem. 289, 31066-31076
Maroteaux, L., Campanelli, J. T., and Scheller, R. H. (1988) J Neurosci 8, 2804-2815
Melentijevic, I. et al. C. elegans neurons jettison protein aggregates and mitochondria under neurotoxic stress. Nature 542, 367-371, doi:10.1038/nature21362 (2017).
Miller, L. C., Swayne, L. A., Chen, L., Feng, Z. P., Wacker, J. L., Muchowski, P. J., Zamponi, G. W., and Braun, J. E. A. (2003) J Biol Chem 278, 53072-53081
Mitra, A., Fillmore, R. A., Metge, B. J., Rajesh, M., Xi, Y., King, J., Ju, J., Pannell, L., Shevde, L. A., and Samant, R. S. (2008) Breast Cancer Res. 10, R22
Morimoto, R. I. (2008) Genes Dev. 22, 1427-1438
Muchowski, P. J. & Wacker, J. L. Modulation of neurodegeneration by molecular chaperones. Nat Rev Neurosci 6, 11-22, doi:10.1038/nrn1587 (2005).
Nonaka, T. et al. Prion-like properties of pathological TDP-43 aggregates from diseased brains. Cell Rep 4, 124-134, doi:10.1016/j.celrep.2013.06.007 (2013).
Noskova, L., Stranecky, V., Hartmannova, H., Pristoupilova, A., Baresova, V., Ivanek, R., Hulkova, H., Jahnova, H., van der, Z. J., Staropoli, J. F., Sims, K. B., Tyynela, J., Van Broeckhoven, C., Nijssen, P. C., Mole, S. E., Elleder, M., and Kmoch, S. (2011) Am. J Hum. Genet 89, 241-252
Novoselov, S. S., Mustill, W. J., Gray, A. L., Dick, J. R., Kanuga, N., Kalmar, B., Greensmith, L., and Cheetham, M. E. (2013) PLoS. ONE. 8, e73944
Ohyama, T., Verstreken, P., Ly, C. V., Rosenmund, T., Rajan, A., Tien, A. C., Haueter, C., Schulze, K. L., and Bellen, H. J. (2007) J Cell Biol 179, 1481-1496
Palmio, J., Jonson, P. H., Evila, A., Auranen, M., Straub, V., Bushby, K., Sarkozy, A., Kiuru-Enari, S., Sandell, S., Pihko, H., Hackman, P., and Udd, B. (2015) Neuromuscul. Disord. 25, 835-842
Polanco, J. C., Scicluna, B. J., Hill, A. F., and Gotz, J. (2016) J. Biol. Chem. 291, 12445-12466
Rajendran, L. et al. Alzheimer's disease beta-amyloid peptides are released in association with exosomes. Proc Natl Acad Sci U S A 103, 11172-11177, doi:10.1073/pnas.0603838103 (2006).
Rose, J. M., Novoselov, S. S., Robinson, P. A., and Cheetham, M. E. (2011) Hum. Mol. Genet. 20, 16-27
Sanchez, E., Darvish, H., Mesias, R., Taghavi, S., Firouzabadi, S. G., Walker, R. H., Tafakhori, A., and Paisan-Ruiz, C. (2016) Hum. Mutat. 37, 1180-1189
Sarparanta, J., Jonson, P. H., Golzio, C., Sandell, S., Luque, H., Screen, M., McDonald, K., Stajich, J. M., Mahjneh, I., Vihola, A., Raheem, O., Penttila, S., Lehtinen, S., Huovinen, S., Palmio, J., Tasca, G., Ricci, E., Hackman, P., Hauser, M., Katsanis, N., and Udd, B. (2012) Nat. Genet. 44, 450-452
Sato, T., Hayashi, Y. K., Oya, Y., Kondo, T., Sugie, K., Kaneda, D., Houzen, H., Yabe, I., Sasaki, H., Noguchi, S., Nonaka, I., Osawa, M., and Nishino, I. (2013) Neuromuscul. Disord. 23, 269-276
Sharples, R. A. et al. Inhibition of gamma-secretase causes increased secretion of amyloid precursor protein C-terminal fragments in association with exosomes. FASEB J 22, 1469-1478, doi:10.1096/fj.07-9357com (2008).
Shen, B., Wu, N., Yang, J. M., and Gould, S. J. (2011) J. Biol. Chem. 286, 14383-14395
Stern, R. A. et al. Preliminary Study of Plasma Exosomal Tau as a Potential Biomarker for Chronic Traumatic Encephalopathy. J Alzheimers Dis 51, 1099-1109, doi:10.3233/JAD-151028 (2016).
Suarez-Cedeno, G., Winder, T., and Milone, M. (2014) Muscle Nerve 49, 607-610
Takeuchi, T., Suzuki, M., Fujikake, N., Popiel, H. A., Kikuchi, H., Futaki, S., Wada, K., and Nagai, Y. (2015) Proc. Natl. Acad. Sci. U. S. A 112, E2497-E2506
The Lancet Neurology Editorial Board. Joining Forces to Fight Neurodegenerative Diseases. Lancet Neurol 12, 119, doi:10.1016/S1474-4422(13)70004-8 (2013).
Tiwari, S. S., d'Orange, M., Troakes, C., Shurovi, B. N., Engmann, O., Noble, W., Hortobagyi, T., and Giese, K. P. (2015) Mol. Brain 8, 6
Velinov, M., Dolzhanskaya, N., Gonzalez, M., Powell, E., Konidari, I., Hulme, W., Staropoli, J. F., Xin, W., Wen, G. Y., Barone, R., Coppel, S. H., Sims, K., Brown, W. T., and Zuchner, S. (2012) PLoS. ONE. 7, e29729
Vella, L. J., Greenwood, D. L., Cappai, R., Scheerlinck, J. P. & Hill, A. F. Enrichment of prion protein in exosomes derived from ovine cerebral spinal fluid. Vet Immunol Immunopathol 124, 385-393, doi:10.1016/j.vetimm.2008.04.002 (2008).
Vella, L. J., Hill, A. F. & Cheng, L. Focus on Extracellular Vesicles: Exosomes and Their Role in Protein Trafficking and Biomarker Potential in Alzheimer's and Parkinson's Disease. Int J Mol Sci 17, 173, doi:10.3390/ijms17020173 (2016).
Watson, E. D., Geary-Joo, C., Hughes, M., and Cross, J. C. (2007) Development 134, 1809-1817
Westhoff, B., Chapple, J. P., van der, S. J., Hohfeld, J., and Cheetham, M. E. (2005) Curr. Biol 15, 1058-1064
Xu, Y. et al. DNAJC5 facilitates USP19-dependent unconventional secretion of misfolded cytosolic proteins. Cell Discov 4, 11, doi:10.1038/s41421-018-0012-7 (2018).
Zarouchlioti, C., Parfitt, D. A., Li, W., Gittings, L. M., and Cheetham, M. E. (2018) Philos. Trans. R. Soc. Lond B Biol. Sci. 373
Zhang, Y. Q., Henderson, M. X., Colangelo, C. M., Ginsberg, S. D., Bruce, C., Wu, T., and Chandra, S. S. (2012) Neuron 74, 136-150
Zinsmaier, K. E., Eberle, K. K., Buchner, E., Walter, N., and Benzer, S. (1994) Science 263, 977-980

## Claims

1. A composition for use in delivering an agent across the blood-brain barrier in a subject or to the central nervous system (CNS) of a subject, the composition comprising extracellular vesicles which comprise the agent and a DnaJ domain-containing protein chaperone or a fragment thereof.

2. A composition for use in the treatment of a CNS disease or disorder in a subject, the composition comprising extracellular vesicles which comprise:
a DnaJ domain-containing protein chaperone or a fragment thereof, and
an agent useful for treatment of the CNS disease or disorder.

3. The composition for use of claim 2, wherein the CNS disease or disorder is a neurodegenerative, psychiatric, neurodevelopmental, or motor disease or disorder, and optionally,
wherein the CNS disease or disorder is Alzheimer's disease, Huntington's disease, Parkinson's disease, amyotrophic lateral sclerosis, dementia, multiple sclerosis, epilepsy, a demyelinating disorder; a myelopathy, a chaperonopathy, an autoimmune disorder, a prion disease, addiction, depression, an anxiety disorder, obsessive-compulsive disorder, attention deficit hyperactivity disorder, Tourette's syndrome, bipolar affective disorder, schizophrenia, Asperger syndrome, autism, CNS infection, CNS trauma, or stroke.

4. The composition for use of any one of claims 1 to 3, wherein the DnaJ domain-containing protein chaperone comprises one or more of a CSPα polypeptide, a DnaJB2 polypeptide, a DnaJB6 polypeptide, a DnaJC7 polypeptide, and a DnaJC10 polypeptide, or a fragment or fragments thereof, and optionally,
wherein the DnaJ domain-containing protein chaperone is a CSPα polypeptide or a fragment thereof or a DnaJC7 polypeptide or a fragment thereof.

5. The composition for use of any one of claims 1 to 4, wherein the extracellular vesicles comprise exosomes, microvesicles, exophers, large extracellular vesicles, or combinations thereof.

6. The composition for use of any one of claims 1 to 5, for administration to a site other than the CNS, and optionally,
for administration intravenously, intraperitoneally, intramuscularly, subcutaneously, transdermally, orally, buccally, intraocularly, nasally, or rectally, or a combination thereof.

7. An agent delivery vehicle comprising an effective amount of extracellular vesicles capable of crossing the blood-brain barrier, wherein the extracellular vesicles comprise:
a DnaJ domain-containing protein chaperone or a fragment thereof, and
an agent useful for treatment of a CNS disease or disorder.

8. The agent delivery vehicle of claim 7, wherein the DnaJ domain-containing protein chaperone comprises one or more of a CSPα polypeptide, a DnaJB2 polypeptide, a DnaJB6 polypeptide, a DnaJC7 polypeptide, and a DnaJC10 polypeptide, or a fragment or fragments thereof, and optionally,
wherein the DnaJ domain-containing protein chaperone is a CSPα polypeptide or a fragment thereof or a DnaJC7 polypeptide or a fragment thereof.

9. The agent delivery vehicle of any one of claims 7 to 8, wherein the extracellular vesicles comprise exosomes, microvesicles, exophers, large extracellular vesicles, or combinations thereof.

10. The agent delivery vehicle of any one of claims 7 to 9, wherein the CNS disease or disorder is a neurodegenerative, psychiatric, neurodevelopmental, or motor disease or disorder, and optionally,
wherein the CNS disease or disorder is Alzheimer's disease, Huntington's disease, Parkinson's disease, amyotrophic lateral sclerosis, dementia, multiple sclerosis, epilepsy, a demyelinating disorder; a myelopathy, a chaperonopathy, an autoimmune disorder, a prion disease, addiction, depression, an anxiety disorder, obsessive-compulsive disorder, attention deficit hyperactivity disorder, Tourette's syndrome, bipolar affective disorder, schizophrenia, Asperger syndrome, autism, CNS infection, CNS trauma, or stroke.

11. An extracellular vesicle comprising a DnaJ domain-containing protein chaperone or a fragment thereof and an agent useful for treatment of a CNS disease or disorder.

12. The extracellular vesicle of claim 11, wherein the DnaJ domain-containing protein chaperone comprises one or more of a CSPα polypeptide, a DnaJB2 polypeptide, a DnaJB6 polypeptide, a DnaJC7 polypeptide, and a DnaJC10 polypeptide, or a fragment or fragments thereof, optionally,
wherein the DnaJ domain-containing protein chaperone is a CSPα polypeptide or a fragment thereof or a DnaJC7 polypeptide or a fragment thereof.

13. The extracellular vesicle of any one of claims 11 to 12, wherein the extracellular vesicle is an exosome, a microvesicle, an exopher, or a large extracellular vesicle.

14. The extracellular vesicle of any one of claims 11 to 13, wherein the CNS disease or disorder is a neurodegenerative, psychiatric, neurodevelopmental, or motor disease or disorder, and optionally,
wherein the CNS disease or disorder is Alzheimer's disease, Huntington's disease, Parkinson's disease, amyotrophic lateral sclerosis, dementia, multiple sclerosis, epilepsy, a demyelinating disorder; a myelopathy, a chaperonopathy, an autoimmune disorder, a prion disease, addiction, depression, an anxiety disorder, obsessive-compulsive disorder, attention deficit hyperactivity disorder, Tourette's syndrome, bipolar affective disorder, schizophrenia, Asperger syndrome, autism, CNS infection, CNS trauma, or stroke.

15. A composition comprising the extracellular vesicle of any one of claims 11 to 14 and a pharmaceutically acceptable carrier, diluent, or excipient.

## Patentansprüche

1. Zusammensetzung zur Verwendung beim Überführen eines Mittels über die Blut-Hirn-Schranke eines Individuums oder Zuführen eines Mittels an das zentrale Nervensystem (ZNS) eines Individuums, wobei die Zusammensetzung extrazelluläre Vesikel umfasst, die das Mittel und ein DnaJ-Domäne enthaltendes Proteinchaperon oder ein Fragment davon umfassen.

2. Zusammensetzung zur Verwendung bei der Behandlung einer Erkrankung oder Störung des ZNS bei einem Individuum, wobei die Zusammensetzung extrazelluläre Vesikel umfasst, die Folgendes umfassen:
ein DnaJ-Domäne enthaltendes Proteinchaperon oder ein Fragment davon und
ein Mittel, das bei der Behandlung der Erkrankung oder Störung des ZNS nützlich ist.

3. Zusammensetzung zur Verwendung nach Anspruch 2, wobei es sich bei der Erkrankung oder Störung des ZNS um eine neurodegenerative, psychiatrische, Neuroentwicklungs- oder Motoneuronkrankheit oder - störung handelt und gegebenenfalls
wobei es sich bei der Erkrankung oder Störung des ZNS um Morbus Alzheimer, Chorea Huntington, Morbus Parkinson, amyotrophe Lateralsklerose, Demenz, multiple Sklerose, Epilepsie, eine demyelinisierende Störung, eine Myelopathie, eine Chaperonopathie, eine Autoimmunstörung, eine Prion-Krankheit, Sucht, Depression, eine Angststörung, Zwangsstörung (OCD= obsessive compulsive disorder), Aufmerksamkeitsdefizit-Hyperaktivitätsstörung, Tourette-Syndrom, bipolare affektive Störung, Schizophrenie, Asperger-Syndrom, Autismus, CZNS-Infektion, ZNS-Trauma oder Schlaganfall handelt.

4. Zusammensetzung zur Verwendung nach einem der Ansprüche 1 bis 3, wobei das DnaJ-Domäne enthaltende Proteinchaperon ein CSPα-Polypeptid und/oder ein DnaJB2-Polypeptid und/oder ein DnaJB6-Polypeptid und/oder ein DnaJC7-Polypeptid und/oder ein DnaJC10-Polypeptid oder ein Fragment oder Fragmente davon umfasst und gegebenenfalls
wobei es sich bei dem DnaJ-Domäne enthaltenden Proteinchaperon um ein CSPa-Polypeptid oder ein Fragment davon oder ein DnaJC7-Polypeptid oder ein Fragment davon handelt.

5. Zusammensetzung zur Verwendung nach einem der Ansprüche 1 bis 4, wobei die extrazellulären Vesikel Exosomen, Mikrovesikel, Exophere, große extrazelluläre Vesikel oder Kombinationen davon umfassen.

6. Zusammensetzung zur Verwendung nach einem der Ansprüche 1 bis 5, zur Verabreichung an anderer Stelle als dem ZNS und gegebenenfalls
zur intravenösen intraperitonealen, intramuskulären, subkutanen, transdermalen, oralen, bukkalen, intraokulären, nasalen oder rektalen oder daraus kombinierten Verabreichung.

7. Mittelzuführungsvehikel, umfassend eine wirksame Menge von extrazellulären Vesikeln, die in der Lage sind, die Blut-Hirn-Schranke zu durchqueren, wobei die extrazellulären Vesikel Folgendes umfassen:
ein DnaJ-Domäne enthaltendes Proteinchaperon oder ein Fragment davon und
ein Mittel, das bei der Behandlung einer Erkrankung oder Störung des ZNS nützlich ist.

8. Mittelzuführungsvehikel nach Anspruch 7, wobei das DnaJ-Domäne enthaltende Proteinchaperon ein CSPα-Polypeptid und/oder ein DnaJB2-Polypeptid und/oder ein DnaJB6-Polypeptid und/oder ein DnaJC7-Polypeptid und/oder ein DnaJC10-Polypeptid oder ein Fragment oder Fragmente davon umfasst und gegebenenfalls wobei es sich bei dem DnaJ-Domäne enthaltenden Proteinchaperon um ein CSPa-Polypeptid oder ein Fragment davon oder ein DnaJC7-Polypeptid oder ein Fragment davon handelt.

9. Mittelzuführungsvehikel nach Anspruch 7 oder 8, wobei die extrazellulären Vesikel Exosomen, Mikrovesikel, Exophere, große extrazelluläre Vesikel oder Kombinationen davon umfassen.

10. Mittelzuführungsvehikel nach einem der Ansprüche 7 bis 9, wobei es sich bei der Erkrankung oder Störung des ZNS um eine neurodegenerative, psychiatrische, Neuroentwicklungs- oder Motoneuronkrankheit oder - störung handelt und gegebenenfalls
wobei es sich bei der Erkrankung oder Störung des ZNS um Morbus Alzheimer, Chorea Huntington, Morbus Parkinson, amyotrophe Lateralsklerose, Demenz, multiple Sklerose, Epilepsie, eine demyelinisierende Störung, eine Myelopathie, eine Chaperonopathie, eine Autoimmunstörung, eine Prion-Krankheit, Sucht, Depression, eine Angststörung, Zwangsstörung (OCD= obsessive compulsive disorder), Aufmerksamkeitsdefizit-Hyperaktivitätsstörung, Tourette-Syndrom, bipolare affektive Störung, Schizophrenie, Asperger-Syndrom, Autismus, ZNS-Infektion, ZNS-Trauma oder Schlaganfall handelt.

11. Extrazelluläres Vesikel, umfassend ein DnaJ-Domäne enthaltendes Proteinchaperon oder ein Fragment davon und ein Mittel, das bei der Behandlung einer Erkrankung oder Störung des ZNS nützlich ist.

12. Extrazelluläres Vesikel nach Anspruch 11, wobei das DnaJ-Domäne enthaltende Proteinchaperon ein CSPα-Polypeptid und/oder ein DnaJB2-Polypeptid und/oder ein DnaJB6-Polypeptid und/oder ein DnaJC7-Polypeptid und/oder ein DnaJC10-Polypeptid oder ein Fragment oder Fragmente davon umfasst, gegebenenfalls
wobei es sich bei dem DnaJ-Domäne enthaltenden Proteinchaperon um ein CSPa-Polypeptid oder ein Fragment davon oder ein DnaJC7-Polypeptid oder ein Fragment davon handelt.

13. Extrazelluläres Vesikel nach Anspruch 11 oder 12, wobei es sich bei dem extrazellulären Vesikel um ein Exosom, ein Mikrovesikel, ein Exopher oder ein großes extrazelluläres Vesikel handelt.

14. Extrazelluläres Vesikel nach einem der Ansprüche 11 bis 13, wobei es sich bei der Erkrankung oder Störung des ZNS um eine neurodegenerative, psychiatrische, Neuroentwicklungs- oder Motoneuronkrankheit oder - störung handelt und gegebenenfalls
wobei es sich bei der Erkrankung oder Störung des ZNS um Morbus Alzheimer, Chorea Huntington, Morbus Parkinson, amyotrophe Lateralsklerose, Demenz, multiple Sklerose, Epilepsie, eine demyelinisierende Störung, eine Myelopathie, eine Chaperonopathie, eine Autoimmunstörung, eine Prion-Krankheit, Sucht, Depression, eine Angststörung, Zwangsstörung (OCD= obsessive compulsive disorder), Aufmerksamkeitsdefizit-Hyperaktivitätsstörung, Tourette-Syndrom, bipolare affektive Störung, Schizophrenie, Asperger-Syndrom, Autismus, ZNS-Infektion, ZNS-Trauma oder Schlaganfall handelt.

15. Zusammensetzung, umfassend das extrazelluläre Vesikel nach einem der Ansprüche 11 bis 14 und einen pharmazeutisch unbedenklichen Träger, ein pharmazeutisch unbedenkliches Verdünnungsmittel oder einen pharmazeutisch unbedenklichen Exzipienten.

## Revendications

1. Composition pour utilisation dans la délivrance d'un agent à travers la barrière hémato-encéphalique d'un sujet ou au système nerveux central (SNC) d'un sujet, la composition comprenant des vésicules extracellulaires qui comprennent l'agent et une protéine chaperone contenant un domaine DnaJ ou un fragment de celle-ci.

2. Composition pour utilisation dans le traitement d'une maladie ou d'un trouble du SNC chez un sujet, la composition comprenant des vésicules extracellulaires qui comprennent :
une protéine chaperone contenant un domaine DnaJ ou un fragment de celle-ci, et
un agent utile pour le traitement de la maladie ou du trouble du SNC.

3. Composition pour utilisation selon la revendication 2, dans laquelle la maladie ou le trouble du SNC est une maladie ou un trouble neurodégénératif, psychiatrique, neurodéveloppemental ou moteur, et éventuellement,
dans laquelle la maladie ou le trouble du SNC est la maladie d'Alzheimer, la maladie de Huntington, la maladie de Parkinson, la sclérose latérale amyotrophique, la démence, la sclérose en plaques, l'épilepsie, un trouble démyélinisant, une myélopathie, une chaperonopathie, une maladie auto-immune, une maladie à prions, une dépendance, une dépression, un trouble anxieux, un trouble obsessionnel-compulsif, un trouble déficitaire de l'attention avec hyperactivité, le syndrome de Gilles de la Tourette, un trouble affectif bipolaire, la schizophrénie, le syndrome d'Asperger, l'autisme, une infection du SNC, un traumatisme du SNC ou un accident vasculaire cérébral.

4. Composition pour utilisation selon l'une quelconque des revendications 1 à 3, dans laquelle la protéine chaperone contenant un domaine DnaJ comprend un ou plusieurs parmi un polypeptide CSPα, un polypeptide DnaJB2, un polypeptide DnaJB6, un polypeptide DnaJC7, et un polypeptide DnaJC10, ou un fragment ou des fragments de ceux-ci, et éventuellement,
dans laquelle la protéine chaperone contenant un domaine DnaJ est un polypeptide CSPα ou un fragment de celui-ci ou un polypeptide DnaJC7 ou un fragment de celui-ci.

5. Composition pour utilisation selon l'une quelconque des revendications 1 à 4, dans laquelle les vésicules extracellulaires comprennent des exosomes, des microvésicules, des exophères, de grandes vésicules extracellulaires, ou des combinaisons de ceux-ci.

6. Composition pour utilisation selon l'une quelconque des revendications 1 à 5, pour administration à un site autre que le SNC, et éventuellement,
pour administration par voie intraveineuse, intrapéritonéale, intramusculaire, sous-cutanée, transdermique, orale, buccale, intraoculaire, nasale ou rectale, ou une combinaison de celles-ci.

7. Véhicule de délivrance d'agent comprenant une quantité efficace de vésicules extracellulaires capables de traverser la barrière hémato-encéphalique, dans lequel les vésicules extracellulaires comprennent :
une protéine chaperone contenant un domaine DnaJ ou un fragment de celle-ci, et
un agent utile pour le traitement d'une maladie ou d'un trouble du SNC.

8. Véhicule de délivrance d'agent selon la revendication 7, dans lequel la protéine chaperone contenant un domaine DnaJ comprend un ou plusieurs parmi un polypeptide CSPα, un polypeptide DnaJB2, un polypeptide DnaJB6, un polypeptide DnaJC7, et un polypeptide DnaJC10, ou un fragment ou des fragments de ceux-ci, et éventuellement,
dans lequel la protéine chaperone contenant un domaine DnaJ est un polypeptide CSPα ou un fragment de celui-ci ou un polypeptide DnaJC7 ou un fragment de celui-ci.

9. Véhicule de délivrance d'agent selon l'une quelconque des revendications 7 à 8, dans lequel les vésicules extracellulaires comprennent des exosomes, des microvésicules, des exophères, de grandes vésicules extracellulaires, ou des combinaisons de ceux-ci.

10. Véhicule de délivrance d'agent selon l'une quelconque des revendications 7 à 9, dans lequel la maladie ou le trouble du SNC est une maladie ou un trouble neurodégénératif, psychiatrique, neurodéveloppemental ou moteur, et éventuellement,
dans lequel la maladie ou le trouble du SNC est la maladie d'Alzheimer, la maladie de Huntington, la maladie de Parkinson, la sclérose latérale amyotrophique, la démence, la sclérose en plaques, l'épilepsie, un trouble démyélinisant, une myélopathie, une chaperonopathie, une maladie auto-immune, une maladie à prions, une dépendance, une dépression, un trouble anxieux, un trouble obsessionnel-compulsif, un trouble déficitaire de l'attention avec hyperactivité, le syndrome de Gilles de la Tourette, un trouble affectif bipolaire, la schizophrénie, le syndrome d'Asperger, l'autisme, une infection du SNC, un traumatisme du SNC ou un accident vasculaire cérébral.

11. Vésicule extracellulaire comprenant une protéine chaperone contenant un domaine DnaJ ou un fragment de celle-ci et un agent utile pour le traitement d'une maladie ou d'un trouble du SNC.

12. Vésicule extracellulaire selon la revendication 11, dans laquelle la protéine chaperone contenant un domaine DnaJ comprend un ou plusieurs parmi un polypeptide CSPα, un polypeptide DnaJB2, un polypeptide DnaJB6, un polypeptide DnaJC7, et un polypeptide DnaJC10, ou un fragment ou des fragments de ceux-ci, éventuellement,
dans laquelle la protéine chaperone contenant un domaine DnaJ est un polypeptide CSPα ou un fragment de celui-ci ou un polypeptide DnaJC7 ou un fragment de celui-ci.

13. Vésicule extracellulaire selon l'une quelconque des revendications 11 à 12, dans laquelle la vésicule extracellulaire est un exosome, une microvésicule, un exophère ou une grande vésicule extracellulaire.

14. Vésicule extracellulaire selon l'une quelconque des revendications 11 à 13, dans laquelle la maladie ou le trouble du SNC est une maladie ou un trouble neurodégénératif, psychiatrique, neurodéveloppemental ou moteur, et éventuellement,
dans laquelle la maladie ou le trouble du SNC est la maladie d'Alzheimer, la maladie de Huntington, la maladie de Parkinson, la sclérose latérale amyotrophique, la démence, la sclérose en plaques, l'épilepsie, un trouble démyélinisant, une myélopathie, une chaperonopathie, une maladie auto-immune, une maladie à prions, une dépendance, une dépression, un trouble anxieux, un trouble obsessionnel-compulsif, un trouble déficitaire de l'attention avec hyperactivité, le syndrome de Gilles de la Tourette, un trouble affectif bipolaire, la schizophrénie, le syndrome d'Asperger, l'autisme, une infection du SNC, un traumatisme du SNC ou un accident vasculaire cérébral.

15. Composition comprenant la vésicule extracellulaire selon l'une quelconque des revendications 11 à 14 et un support, un diluant ou un excipient pharmaceutiquement acceptable.
